(19) **Europäisches Patentamt**
**European Patent Office**
**Office européen des brevets**

(11) **EP 1 904 645 B1**

(12) **EUROPEAN PATENT SPECIFICATION**

(45) Date of publication and mention
of the grant of the patent:
**04.08.2010 Bulletin 2010/31**

(51) Int Cl.:
***C12Q 1/68*** (2006.01)

(21) Application number: **06754554.1**

(22) Date of filing: **24.06.2006**

(86) International application number:
**PCT/EP2006/006114**

(87) International publication number:
**WO 2007/006408 (18.01.2007 Gazette 2007/03)**

(54) **METHOD FOR PREDICTING AND MONITORING DIRECT RESPONSE TO CANCER THERAPY**

VERFAHREN ZUR VORHERSAGE UND ÜBERWACHUNG EINER UNMITTELBAREN REAKTION
AUF EINE KREBSTHERAPIE

METHODE DE PREVISION ET DE SURVEILLANCE D'UNE REPONSE DIRECTE A UNE THERAPIE
ANTI-CANCEREUSE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **08.07.2005 EP 05014891**

(43) Date of publication of application:
**02.04.2008 Bulletin 2008/14**

(73) Proprietor: **Siemens Healthcare Diagnostics
GmbH
65760 Eschborn (DE)**

(72) Inventors:
• **MUNNES, Marc
40699 Erkrath (DE)**
• **VON MINCKWITZ, Gunther
63263 Neu-Isenburg (DE)**
• **RODY, Achim
60596 Frankfurt (DE)**
• **KARN, Thomas
61440 Oberursel (DE)**

(74) Representative: **Maier, Daniel Oliver et al
Siemens AG
CT IP
Postfach 22 16 34
80506 München (DE)**

(56) References cited:
**EP-A- 1 365 034          WO-A-2005/021788
US-A1- 2004 101 855**

• **EGAWA CHIYOMI ET AL: "Increased expression
of BRCA1 mRNA predicts favorable response to
anthracycline-containing chemotherapy in
breast cancers." BREAST CANCER RESEARCH
AND TREATMENT, vol. 78, no. 1, March 2003
(2003-03), pages 45-50, XP002426612 ISSN:
0167-6806**
• **COLLEONI M ET AL: "Preoperative systemic
treatment: Prediction of responsiveness."
BREAST, vol. 12, no. 6, December 2003 (2003-12),
pages 538-542, XP002426613 ISSN: 0960-9776**
• **RING A E ET AL: "PREDICTORS OF RESPONSE
TO SYSTEMIC THERAPY IN BREAST CANCER"
FORUM, GENOVA, IT, vol. 12, no. 1, 2002, pages
19-32, XP009012703 ISSN: 1121-8142**
• **MODLICH O ET AL: "Predictors of primary breast
cancers responsiveness to preoperative
epirubicin/cyclophosphamide-based
chemotherapy: Translation of microarray data
into clinically usefull predictive signatures"
JOURNAL OF TRANSLATIONAL MEDICINE 09
AUG 2005 UNITED KINGDOM, vol. 3, 9 August
2005 (2005-08-09), page 18p, XP021009880 ISSN:
1479-5876**
• **DATABASE WPI Derwent Publications Ltd.,
London, GB; AN 2005-061418 XP002426632 & JP
2005 000056 A (SUMITOMO SEIYAKU KK) 6
January 2005 (2005-01-06)**

**(Cont. next page)**

- ZHU Y ET AL: "Amplification and overexpression of peroxisome proliferator-activated receptor binding protein (PBP/PPARBP) gene in breast cancer." PROCEEDINGS OF THE NATIONAL ACADEMY OF SCIENCES OF THE UNITED STATES OF AMERICA 14 SEP 1999, vol. 96, no. 19, 14 September 1999 (1999-09-14), pages 10848-10853, XP002426631 ISSN: 0027-8424

**Description**

The present invention relates to methods for the prediction of therapeutic success in cancer therapy (e.g. tumor's response to therapy),as well as to the diagnosis, prognosis, treatment of neoplastic diseases. Cancer cells display a specific pattern of gene expression related to their morphological type, state of progression, acquirement of genomic alterations, point mutations in critical genes such as gatekeepers and tumor suppressors or due to the dependency of external signals such as growth factors, hormones or other secondary messengers. In a preferred embodiment of the invention it relates to methods for prediction of therapeutic success in preoperative chemo therapy as performed with the combination of the therapeutic agents Taxol, Anthracycline and Cyclophosphamide. The methods of the invention are based on determination of expression levels of PPARBP and optionally 85 other human genes which are differentially expressed prior to the onset of an anti-cancer chemotherapy. The invention discloses genes which show an altered expression in a particular neoplastic tissue compared to other neoplastic lesions unresponsive to a given chemotherapy. The genes disclosed in this invention have been identified in breast cancers and are linked to the predictable outcome in the use of a certain therapy regimen The methods is equally well useful in the investigation of other types of primary systemic chemotherapy.

## BACKGROUND OF THE INVENTION AND PRIOR ART

Cancer is the second leading cause of death in the United States after cardiovascular disease. One in three Americans will develop cancer in his or her lifetime, and one of every four Americans will die of cancer. More specifically breast cancer claims the lives of approximately 40,000 women and is diagnosed in approximately 200,000 women annually in the United States alone. Tumors in general are classified based on different parameters, such as tumor size, invasion status, involvement of lymph notes, metastasis, histopathology, imunohistochemical markers, and molecular markers (1 ; 2). With the recent advances in gene chip technology, researchers are increasingly focusing on the categorization of tumors based on the distinct expression of marker genes (3 ; 4).

It is a well established fact, that adjuvant systemic treatment after surgery reduces the risk of disease relapse and death in patients with primary operable breast cancer. As an alternative therapeutic concept neoadjuvant or primary systemic therapy (PST) can be offered to those patients with either larger inoperable breast cancers or to patients interested in breast conserving surgery (80). The PST in general does not offer a survival advantage over standard adjuvant treatment, but may identify patients with a pathologically confirmed complete response (CR). This clinical response to PST is associated with improved survival (81) and reflects a great benefit to app.20% of the PST treated patients. Studies elaborating PST have demonstrated that early gene expression changes are significantly associated with clinical response (83). The identification of those patients who won't benefit from a once administered PST (given combination of chemotherapeutics) after the first round of treatment (e.g. few hours post i.v. application of the drug) will give the opportunity to change regimen for patients' health improvement.

In general, all patients of a given cohort do receive the same treatment, even though many will fail in treatment success. Bio-markers reflecting the tumor response can function as sensitive short-term surrogates of long-term outcome. The use of such bio-markers will make chemotherapy more effective for the individual patient and will allow changing regimen early in case of the non responding tumors.

Although much effort has been made to develop an optimal clinical treatment course for an individual patient with breast cancer, only little progress could be achieved predicting the individual's response to a certain therapy. Such predictions are usually based on standard clinical parameters such as tumor stage and grade, estrogen (ER) and progesterone (PgR) receptors' status, growth rate, over-expression of the HER2/neu and p53 oncogenes. However, evidences about association of ER and/or PgR gene expression with outcome prediction for adjuvant endocrine chemotherapy are still controversial. Studies have shown that levels of ER and PgR gene expression of breast cancer patients are of prognostic importance independently from subsequent adjuvant chemotherapy. From the theoretical point of view, it is not unexpected that the therapeutic response in patients with breast cancer depends on the ER/PgR status. It causes problems finding such factors using conventional biological techniques because all these analyses survey one gene at a time.

Researchers are increasingly focusing on the categorization of tumors based on the distinct expression of marker genes and the DNA microarray technology has been very useful for quantitative measurements of expression levels of thousands of genes simultaneously in one sample. So far this technology has been applied for the classification of cancer tissues e.g., breast tumors (68 - 72), prediction of metastasis and patient's outcome (73 - 75), and tumor response to chemotherapy (76 - 79).

But nevertheless adjuvant chemotherapy remains a mainstay in therapeutic regimens offered to patients with breast cancer, particularly those who have cancer that has metastasized from its site of origin (5). There are several chemo-therapeutic agents that have demonstrated activity in the treatment of breast cancer and research is continuously in an attempt to determine optimal drugs and regimens. However, different patients tend to respond differently to the same therapeutic regimen. Currently, the individuals response to certain therapy can only be assessed statistically, based on data of former clinical studies. There are still a great number of patients who will not benefit from a systemic chemotherapy. Especially, breast cancers are very heterogeneous in their aggressiveness and treatment response. They contain different genetic mutations and variations affecting growths characteristic and sensitivity to several drugs. Identification of each tumor's molecular fingerprint, then, could help to segregate patients who have particularly aggressive tumors or who need to be treated with specific beneficial therapies. As research involving genetics and associated responses to treatment matures, standard practice will undoubtedly become more individualized, enabling physicians to provide specific treatment regimens matched with a tumor's genetic profiles to ensure optimal outcomes.

## SUMMARY OF THE INVENTION

The present invention discloses the identification of 86 human genes being differentially expressed in neoplastic tissue resulting in an altered clinical behavior of a neoplastic lesion. The differential expression of these 86 genes is not limited to a specific neoplastic lesion in a certain tissue of the human body.

The neoplastic lesion, of which these 86 genes are altered in their expression is a cancer of the human breast. This cancer is not limited to females and may also be diagnosed and analyzed in males.

The invention relates to a method, for predicting outcome of PST of breast cancer. "Breast cancer" as used herein includes carcinomas, (e.g., carcinoma in situ, invasive carcinoma, and metastatic carcinoma) and pre-malignant conditions, neomorphic changes independent of their histological origin (e.g. ductal, lobular, medullary, mixed origin). The method of the present invention comprise comparing the level of mRNA expression of PPARBP and optionally other (e.g. 2, 5, 10, or 50 or more) of genes (hereinafter "marker genes", listed in Table 1, SEQUENCE:1 to 86, the respective polypeptide sequences coded by them., see also Table 1) in a patient sample, and the average level of expression of the marker gene(s) in a sample from a control subject (e.g., a human subject without breast cancer).

The method of the invention of compares the level of mRNA expression in an unclassified patient sample, and the average level of expression of the marker gene(s) in a sample cohort comprising patient responding in different intensity to an administered breast cancer therapy. In preferred embodiments of this invention the specific expression of the marker genes can be utilized for discrimination of responders and non-responders to an anthracycline based (e.g. polychemotherapies with epirubicin or doxorubicin) combined with a taxol (e.g. docetaxel or paclitaxel) in addition to a cyclophosphamide chemo-therapeutic intervention. Such therapy schemes are frequently abbreviated by alliterations such as TEC, TAC, ETC or ECT.

The present invention is based on the unexpected finding, that 86 human genes are differentially expressed in neoplastic tissue of patients responding well to a PST chemotherapy with the drug combinations mentioned above as compared to patients not responding well to such PST chemotherapy. Response to an PST chemotherapy may be reflected by the prolonged recurrence free survival time after intervention for the primary tumor, but may also reflect the over all survival time. Hence, elevated or decreased levels of expression in one or several of the 86 genes at the time of tumor surgery or prior to any intervention (e.g. punch biopsy sample) was found to provide valuable information on whether or not a patient's tumor is likely to respond to the chemotherapy applied.

In further preferred embodiments, the control level of mRNA expression is the average level of expression of the marker gene(s) in samples from several (e.g., 2, 3, 4, 5, 8, 10, 12, 15, 20, 30 or 50) control subjects. These control subjects may also be affected by breast cancer and be classified by their clinical and not necessarily by their individual expression profile.

As elaborated below, a significant change in the level of expression of one or more of the marker genes (set of marker genes) in the patient sample relative to the control level provides significant information regarding the patient's breast cancer status and responsiveness to chemotherapy. In the invention, said given mode compositions, methods, and kits of chemotherapy is an anthracycline based (e.g. polychemotherapies with epirubicin or doxorubicin) combined with a taxol (e.g. docetaxel or paclitaxel) in addition to a cyclophosphamide chemotherapy.

The present invention furthermore relates to methods of investigating the response of a patient to anti-cancer chemotherapy by determination of the differential expression of PPARBP and optionally other of the said 86 human genes, at the time of tumor excision and before the onset of anti-cancer chemotherapy in a patient. Said investigation of the response can be performed immediately after first biopsy, at a stage in which other methods can not provide the required information on the patient's response to chemotherapy.

Hence the current invention provides means to decide - shortly after tumor biopsy - whether or not a certain mode of chemotherapy is likely to be beneficial to the patient's health and/or whether to maintain or change the applied mode of chemotherapy treatment.

As elaborated below, a significant change in the level of expression of one or more of the marker genes (set of marker genes) in the patient sample relative to the control level provides significant information regarding the patient's breast cancer status and responsiveness to chemotherapy., preferably TEC, TAC or ECT chemotherapy. In the method of the present invention PPARBP may also be used in combination with well known breast cancer marker genes (e.g.

ESR1, PGR, ERBB2, CEA, mammaglobin, TOP2A, or CA 15-3) or cell proliferation associated genes (e.g CCNE1, CCNE2, CCNB2, BUB1, K167).

The marker gene(s) and marker gene sets are selected such that the positive predictive value of the compositions, methods, and kits of the invention is at least about 10%, preferably about 25%, more preferably about 50% and most preferably about 90%. Also preferred for use in the method of the invention are marker gene(s) and sets that are differentially expressed, as compared to normal breast cells, by at least the minimal median differential expression factor presented in Table 3, in at least about 20%, more preferably about 50% and most preferably about 75% of any of the following conditions: stage 0 breast cancer patients, stage I breast cancer patients, stage II breast cancer patients, stage III breast cancer patients, stage IV breast cancer patients, grade I breast cancer patients, grade II breast cancer patients, grade III breast cancer patients, malignant breast cancer patients, patients with primary carcinomas of the breast, and all other types of cancers, malignancies and transformations associated with the breast.

The detection of marker gene expression is not limited to the detection within a primary, secondary or metastatic lesion of breast cancer patients, and may also be detected in lymph nodes affected by breast cancer cells or minimal residual disease cells either locally deposited (e.g. bone marrow, liver, kidney) or freely floating throughout the patients body.

In one embodiment of the method of the present invention, the sample to be analyzed is tissue material from neoplastic lesion taken by aspiration or punctuation, excision or by any other surgical method leading to biopsy or resected cellular material. In one embodiment of the method of the present invention, the sample comprises cells obtained from the patient. The cells may be found in a breast cell "smear" collected, for example, by a nipple aspiration, ductal lavarge, fine needle biopsy or from provoked or spontaneous nipple discharge. In another embodiment, the sample is a body fluid. Such fluids include, for example, blood fluids, lymph, ascitic fluids, gynecological fluids, or urine but not limited to these fluids.

In accordance with the method of the present invention the determination of gene expression is not limited to any specific method or to the detection of mRNA. The presence and/or level of expression of the marker gene in a sample can be assessed, for example, by measuring and/or quantifying of:

1) a protein encoded by the marker gene in Table 1 (SEQUENCE:1 to 86) or a polypeptide resulting from processing or degradation of the protein (e.g. using a reagent, such as an antibody, an antibody derivative, or an antibody fragment, which binds specifically with the protein or polypeptide)

2) a metabolite which is produced directly (i.e., catalyzed) or indirectly by a protein encoded by the marker gene in Table I (SEQUENCE: 1 to 86) or by a polypeptide comprising a polypeptide encoded from SEQUENCE: 1 to 86 thereby.

3) a RNA transcript (e.g., mRNA, hnRNA) encoded by the marker gene in Table 1, or a fragment of the RNA transcript (e.g. by contacting a mixture of RNA transcripts obtained from the sample or cDNA prepared from the transcripts with a substrate having nucleic acid comprising a sequence of one or more of the marker genes listed within Table 1 fixed thereto at selected positions). The mRNA expression of these genes can be detected e.g. with DNA-microarrays as provided by Affymetrix Inc. or other manufacturers. U.S. (US Pat. No. 5,556,752.). In a further embodiment the expression of these genes can be detected with bead based direct fluorescent readout techniques such as provided by Luminex Inc. PCT No. (WO 97/14028.).

The method of the present invention is particularly useful for identifying patients who will not respond to a certain chemotherapy and therefor develop recurrent disease.

It will be appreciated that the method of the present invention may also include known cancer marker genes including known breast cancer marker genes.

## DETAILED DESCRIPTION OF THE INVENTION

### Definitions

The term "neoplastic lesion" or " neoplastic disease" or "neoplasia" refers to a cancerous tissue this includes carcinomas, (e.g., carcinoma in situ, invasive carcinoma, metastatic carcinoma) and pre-malignant conditions, neomorphic changes independent of their histological origin (e.g. ductal, lobular, medullary, mixed origin).

The term "cancer" is not limited to any stage, grade, histomorphological feature, invasiveness, aggressiveness or malignancy of an affected tissue or cell aggregation. In particular stage 0 breast cancer, stage I breast cancer, stage II breast cancer, stage III breast cancer, stage IV breast cancer, grade I breast cancer, grade II breast cancer, grade III breast cancer, malignant breast cancer, primary carcinomas of the breast, and all other types of cancers, malignancies and transformations associated with the breast are included.

The terms "neoplastic lesion" or " neoplastic disease" or "neoplasia" or "cancer" are not limited to any tissue or cell type they also include primary, secondary or metastatic lesion of cancer patients, and also comprises lymph nodes affected by cancer ells or minimal residual disease cells either locally deposited (e.g. bone marrow, liver, kidney) or freely floating throughout the patients body.

Furthermore, the term "characterizing the state of a neoplastic disease" is related to, but not limited to, measurements and assessment of one or more of the following conditions: Type of tumor, histomorphological appearance, dependence on external signal (e.g. hormones, growth factors), invasiveness, motility, state by TNM (2) or similar, aggressiveness, malignancy, metastatic potential, and responsiveness to a given therapy.

The term "biological sample", as used herein, refers to a sample obtained from an organism or from components (e.g., cells) of an organism. The sample may be of any biological tissue or fluid. Frequently the sample will be a "clinical sample" which is a sample derived from a patient. Such samples include, but are not limited to, sputum, blood, blood cells (e.g., white cells), tissue or fine needle biopsy samples, cell-containing body fluids, free floating nucleic acids, urine, peritoneal fluid, and pleural fluid, or cells therefrom. Biological samples may also include sections of tissues such as frozen or fixed sections taken for histological purposes. A biological sample to be analyzed is tissue material from neoplastic lesion taken by aspiration or punctuation, excision or by any other surgical method leading to biopsy or resected cellular material. Such biological sample may comprises cells obtained from a patient. The cells may be found in a breast cell "smear" collected, for example, by a nipple aspiration, ductal lavarge, fine needle biopsy or from provoked or spontaneous nipple discharge. In another embodiment, the sample is a body fluid. Such fluids include, for example, blood fluids, lymph, ascitic fluids, gynecological fluids, or urine but not limited to these fluids.

The term "therapy modality", "therapy mode", "regimen" or "chemo regimen" as well as "therapy regime" refers to a timely sequential or simultaneous administration of anti tumor, and/or immune stimulating, and/or blood cell proliferative agents, and/or radiation therapy, and/or hyperthermia, and/or hypothermia for cancer therapy. The administration of these can be performed in an adjuvant and/or neoadjuvant mode. The composition of such "protocol" may vary in dose of the single agent, timeframe of application and frequency of administration within a defined therapy window. Currently various combinations of various drugs and/or physical methods, and various schedules are under investigation.

The term "classifying the state" or "determine the state" of a cancer or neoplastic lesion refers to the analysis of certain genes or their expression either on DNA, RNA or protein level. While the term "state" does refer to a certain condition or property of a tumor encoded by the genomic background of such. The state of a tumor at a given point in time (preferably at diagnosis) does reflect the ability of such tumor to invade, proliferate, progress, respond to certain drugs, regress or diminish.

The phrase "tumor response", "therapeutic success", or "response to therapy" refers, in the adjuvant chemotherapeutic setting to the observation of a defined tumor free or recurrence free survival time (e.g. 2 years, 4 years, 5 years, 10 years). This time period of disease free survival may vary among the different tumor entities but is sufficiently longer than the average time period in which most of the recurrences appear. In a neoadjuvant therapy modality response may be monitored by measurement of tumor shrinkage due to apoptosis and necrosis of the tumor mass.

The terms "to respond" or "not to respond" are to be understood in a qualitative and/or in a quantitative fashion. "To respond" and "not to respond" is to be assessed with regard to a suitable reference responses, such as, e.g., responses shown by "responders" and "not-responders" to a certain mode of treatment or modality of treatment. Preferably tumor response is assessed in a neoadjuvant (PST) *in vivo* monitoring situation where the size of a tumor post systemic intervention can be compared to the initial size and dimensions as measured by CT, PET, mammogram, ultrasound or palpation. Size could also be measured by caliper assessment or incisional biopsy.

The term "recurrence" or "recurrent disease" does include distant metastasis that can appear even many years after the initial diagnosis and therapy of a tumor, or to local events such as infiltration of tumor cell into regional lymph nodes, or occurrence of tumor cells at the same site and organ of origin within an appropriate time.

"Prediction of recurrence" or "prediction of success" does refer to the methods and compositions described in this invention. Wherein a tumor specimen is analyzed for it's gene expression and furthermore classified based on correlation of the expression pattern to known ones from reference samples. This classification may either result in the statement that such given tumor will develop recurrence and therefore is considered as a "non responding" tumor to the given therapy, or may result in a classification as a tumor with a prorogued disease free post therapy time.

"Differential expression", or "expression" as used herein, refers to both quantitative as well as qualitative differences in the genes' expression patterns observed in at least two different individuals or samples taken from individuals. Differential expression may depend on differential development, different genetic background of tumor cells and/or reaction to the tissue environment of the tumor. Differentially expressed genes may represent "marker genes," and/or "target genes".

The expression pattern of a differentially expressed gene disclosed herein may be utilized as part of a prognostic or diagnostic breast cancer evaluation. The term "pattern of expression" refers, e.g., to a determined level of gene expression compared either to a reference gene (e.g. housekeeper) or to a computed average expression value (e.g.

in DNA-chip analyses). A pattern is not limited to the comparison of two genes but even more related to multiple comparisons of genes to a reference genes or samples. A certain "pattern of expression" may also result and be determined by comparison and measurement of several genes disclosed hereafter and displays the relative abundance of these transcripts to each other.

Alternatively, a differentially expressed gene disclosed herein may be used in methods for identifying reagents and compounds and uses of these reagents and compounds for the treatment of breast cancer as well as methods of treatment. The differential regulation of the gene is not limited to a specific cancer cell type or clone, but rather displays the interplay of cancer cells, muscle cells, stromal cells, connective tissue cells, other epithelial cells, endothelial cells and blood vessels as well as cells of the immune system (e.g. lymphocytes, macrophages, killer cells).

A "reference pattern of expression levels", within the meaning of the invention shall be understood as being any pattern of expression levels that can be used for the comparison to another pattern of expression levels. In a preferred embodiment of the invention, a reference pattern of expression levels is, e.g., an average pattern of expression levels observed in a group of healthy or diseased individuals, serving as a reference group.

The term "marker" or "biomarker" refers a biological molecule, e.g., a nucleic acid, peptide, hormone, etc., whose presence or concentration can be detected and correlated with a known condition, such as a disease state.

The term "marker gene," as used herein, refers to a differentially expressed gene which expression pattern may be utilized as part of predictive, prognostic or diagnostic process in malignant neoplasia or breast cancer evaluation, or which, alternatively, may be used in methods for identifying compounds useful for the treatment or prevention of malignant neoplasia and breast cancer in particular. A marker gene may also have the characteristics of a target gene.

"Target gene", as used herein, refers to a differentially expressed gene involved in breast cancer in a manner by which modulation of the level of target gene expression or of target gene product activity may act to ameliorate symptoms of malignant neoplasia and breast cancer in particular. A target gene may also have the characteristics of a marker gene.

"BREAST CANCER GENES" or "BREAST CANCER GENE" as used herein refers to the polynucleotides of SEQUENCE: 1 to 86 (listed in Table 1), as well as derivatives, fragments, analogs and homologues thereof, the polypeptides encoded thereby, (SEQUENCE: 1 to 86, see Table 1) as well as derivatives, fragments, analogs and homologues thereof and the corresponding genomic transcription units which can be derived or identified with standard techniques well known in the art using the information disclosed in Table 1. The Gene symbol, Gene Description, Unigene_name, Reference Sequence ID, OMIM-identifier, and the locus link ID numbers of the polynucleotide sequences SEQUENCE: 1 to 86 and the polypeptides encoded by them are given in Table 1 and do refer to the commonly and publicly available identifier and abbreviations for a given gene sequence.

"Primer pairs and probes", within the meaning of the invention, shall have the ordinary meaning of this term which is well known to the person skilled in the art of molecular biology. In a preferred embodiment of the invention "primer pairs and probes", shall be understood as being polynucleotide molecules having a sequence identical, complementary, homologous, or homologous to the complement of regions of a target polynucleotide which is to be detected or quantified.

"Individually labeled probes", within the meaning of the invention, shall be understood as being molecular probes comprising a polynucleotide or oligonucleotide and a label, helpful in the detection or quantification of the probe. Preferred labels are fluorescent labels, luminescent labels, radioactive labels and dyes.

"Arrayed probes", within the meaning of the invention, shall be understood as being a collection of immobilized probes, preferably in an orderly arrangement. In a preferred embodiment of the invention, the individual "arrayed probes" can be identified by their respective position on the solid support, e.g., on a "chip".

By "array" or "matrix" is meant an arrangement of addressable locations or "addresses" on a device. The locations can be arranged in two dimensional arrays, three dimensional arrays, or other matrix formats. The number of locations can range from several to at least hundreds of thousands. Most importantly, each location represents a totally independent reaction site. Arrays include but are not limited to nucleic acid arrays, protein arrays and antibody arrays. A "nucleic acid array" refers to an array containing nucleic acid probes, such as oligonucleotides, polynucleotides or larger portions of genes. The nucleic acid on the array is preferably single stranded. Arrays wherein the probes are oligonucleotides are referred to as "oligonucleotide arrays" or "oligonucleotide chips." A "microarray," herein also refers to a "biochip" or "biological chip", an array of regions having a density of discrete regions of at least about 100/cm$^2$, and preferably at least about 1000/cm$^2$. The regions in a microarray have typical dimensions, e.g., diameters, in the range of between about 10-250 $\mu$m, and are separated from other regions in the array by about the same distance. A "protein array" refers to an array containing polypeptide probes or protein probes which can be in native form or denatured. An "antibody array" refers to an array containing antibodies which include but are not limited to monoclonal antibodies (e.g. from a mouse), chimeric antibodies, humanized antibodies or phage antibodies and single chain antibodies as well as fragments from antibodies.

It is apparent to the person skilled in the art that, in order to determine the expression of a gene, parts and fragments of said gene can be used instead.

The invention also relates to methods for determining the probability of successful application of a given mode of treatment in a subject having breast cancer, wherein sequences being homologues to the sequences of Table 1 are used. Preferred homologues have an 80, 90, 95, or 99% sequence identity towards the original sequence. Preferably the homologues still have the same biological activity and/or function as have the original molecules.

It is obvious to the person skilled in the art that a reference to a nucleotide sequence is meant to comprise the reference to the associated protein sequence which is coded by said nucleotide sequence.

"% identity" of a first sequence towards a second sequence, within the meaning of the invention, means the % identity which is calculated as follows: First the optimal global alignment between the two sequences is determined with the CLUSTALW algorithm [Thomson JD, Higgins DG, Gibson TJ. 1994.] ClustalW Version 1.8, applying the following command line syntax: ./clustalw - infile=./infile.txt -output= -outorder=aligned -pwmatrix=gonnet -pwdnamatrix=clustalw -pwgapopen=10.0 -pwgapext=0.1 -matrix=gonnet -gapopen=10.0 -gapext=0.05 -gapdist=8 -hgapresidues=GPSNDQERK -maxdiv=40. Implementations of the CLUSTAL W algorithm are readily available at numerous sites on the internet, including, e.g., http://www.ebi.ac.uk. Thereafter, the number of matches in the alignment is determined by counting the number of identical nucleotides (or amino acid residues) in aligned positions. Finally, the total number of matches is divided by the number of nucleotides (or amino acid residues) of the longer of the two sequences, and multiplied by 100 to yield the % identity of the first sequence towards the second sequence.

The term "derivative" refers to the chemical modification of a polypeptide sequence, or a polynucleotide sequence. Chemical modifications of a polynucleotide sequence can include, for example, replacement of hydrogen by an alkyl, acyl, or amino group. A derivative polynucleotide encodes a polypeptide which retains at least one biological or immunological function of the natural molecule. A derivative polypeptide is one modified by glycosylation, pegylation, or any similar process that retains at least one biological or immunological function of the polypeptide from which it was derived. The term "derivative" furthermore refers to phosphorylated forms of a polypeptide sequence or protein.

The term "nucleotide analog" refers to oligomers or polymers being at least in one feature different from naturally occurring nucleotides, oligonucleotides or polynucleotides, but exhibiting functional features of the respective naturally occurring nucleotides (e.g. base paring, hybridization, coding information) and that can be used for said compositions. The nucleotide analogs can consist of non-naturally occurring bases or polymer backbones, examples of which are LNAs, PNAs and Morpholinos. The nucleotide analog has at least one molecule different from its naturally occurring counterpart or equivalent.

"Biological activity" or "bioactivity" or "activity" or "biological function", which are used interchangeably, herein mean an effector or antigenic function that is directly or indirectly performed by a polypeptide (whether in its native or denatured conformation), or by any fragment thereof *in vivo* or *in vitro.* Biological activities include but are not limited to binding to polypeptides, binding to other proteins or molecules, enzymatic activity, signal transduction, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The terms "modulated" or "modulation" or "regulated" or "regulation" and "differentially regulate" as used herein refer to both upregulation (i.e., activation or stimulation (e.g., by agonizing or potentiating) and down regulation [i.e., inhibition or suppression (e.g., by antagonizing, decreasing or inhibiting)].

"Transcriptional regulatory unit" refers to DNA sequences, such as initiation signals, enhancers, and promoters, which induce or control transcription of protein coding sequences with which they are operably linked. In preferred embodiments, transcription of one of the genes is under the control of a promoter sequence (or other transcriptional regulatory sequence) which controls the expression of the recombinant gene in a cell-type in which expression is intended. It will also be understood that the recombinant gene can be under the control of transcriptional regulatory sequences which are the same or which are different from those sequences which control transcription of the naturally occurring forms of the polypeptide.

The term "agonist", as used herein, is meant to refer to an agent that mimics or upregulates (e.g., potentiates or supplements) the bioactivity of a protein. An agonist can be a wild-type protein or derivative thereof having at least one bioactivity of the wild-type protein. An agonist can also be a compound that upregulates expression of a gene or which increases at least one bioactivity of a protein. An agonist can also be a compound which increases the interaction of a polypeptide with another molecule, e.g., a target peptide or nucleic acid.

The term "antagonist" as used herein is meant to refer to an agent that downregulates (e.g., suppresses or inhibits) at least one bioactivity of a protein. An antagonist can be a compound which inhibits or decreases the interaction between a protein and another molecule, e.g., a target peptide, a ligand or an enzyme substrate. An antagonist can also be a compound that downregulates expression of a gene or which reduces the amount of expressed protein present.

"Small molecule" as used herein, is meant to refer to a composition, which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

The term "chromosomal region" as used herein refers to a consecutive DNA stretch on a chromosome which can be defined by cytogenetic or other genetic markers such as e.g. restriction length polymorphisms (RFLPs), single nucleotide polymorphisms (SNPs), expressed sequence tags (ESTs), sequence tagged sites (STSs), microsatellites, variable number of tandem repeats (VNTRs) and genes. Typically a chromosomal region consists of up to 2 Megabases (MB), up to 4 MB, up to 6 MB, up to 8 MB, up to 10 MB, up to 20 MB or even more MB.

The term "kit" as used herein refers to any manufacture (e.g. a diagnostic or research product) comprising at least one reagent, e.g. a probe, for specifically detecting the expression of at least one marker gene disclosed in the invention, in particular of those genes listed in Table 1, whereas the manufacture is being sold, distributed, and/or promoted as a unit for performing the methods of the present invention. Also reagents (e.g. immunoassays) to detect the presence, the stability, activity, complexity of the respective marker gene products comprising polypeptides selected from SEQUENCE: 1 to 86 encoded by the genes listed in Table 1 regard as components of the kit. In addition, any combination of nucleic acid and protein detection as disclosed in the invention are regard as a kit.

It is apparent to the person skilled in the art that, in order to determine the expression of a gene, parts and fragments of said gene can be used for the specific determination.

The invention also relates to methods for investigating response to anti-cancer chemotherapy as described above, wherein, however, in step (i) and (ii), patterns of expression levels are determined and compared. A "pattern of expression levels" of a single gene is to be understood as the expression level of said gene as determined by suitable methods.

It is obvious to the person skilled in the art that a reference to a nucleotide sequence is meant to comprise the reference to the associated protein sequence which is coded by said nucleotide sequence.

*Experimental procedures and settings*

The present invention relates to predicting the successful application of a given mode of PST to a cancer patient, as those individual's tumor will respond to a given mode of treatment by reduction of the tumor mass by at least 50 %. In a preferred embodiment of the invention, said mode of treatment is a combination of anticancer drugs. Namely it is an anthracycline based (e.g. epirubicin or doxorubicin) combined with a taxol (e.g. docetaxel or paclitaxel) in addition to a cyclophosphamide chemotherapy.

These compounds have been established as important chemotherapeutic agents in the armamentarium of drugs to treat breast cancer since the 1970s and are still in use. Expression profiles of 50 pre-treatment biopsy samples have been obtained by the use of oligonucleotide microarrays (Affymetrix).

Analyzing the data for 50 tumor tissue samples and several "normal" not affected breast specimens by statistical methods as described in EXAMPLES 1 to 7 we identified 86 significantly differentially expressed genes listed in Table 1.

*Biological relevance of the genes of Table 1*

Some of the genes listed in Table 1 represent biological, cellular processes and are characterized by similar regulation of genes. By the way of illustration but limited to the following examples a few characteristic genes from Table1 are described in later by greater detail:

PPARBP

The thyroid hormone receptors (TRs) are hormone-dependent transcription factors that regulate expression of a variety of specific target genes. They must specifically interact with a number of proteins as they progress from their initial translation and nuclear translocation to heterodimerization with retinoid X receptors (RXRs), functional interactions with other transcription factors and the basic transcriptional apparatus, arid eventually, degradation.

PPARBP, or TRAP220, was isolated in 1998 from an immunopurified TR-alpha-TRAP complex. TRAP220 is nearly identical to RB18A. RB18A shares functional properties with p53, including DNA binding, p53 binding, and self-oligomerization. Furthermore, RB18A is able to activate the sequence-specific binding of p53 to DNA, which was induced through an unstable interaction between both proteins. TRAP220 also interacted with other nuclear receptors, including vitamin D receptor, RARA, RXRA, PPARA, PPARG, and estrogen receptor-alpha (ESR1), in a ligand-dependent manner. TRAP220, a nuclear receptor coactivator, interacts with ESR1 in the absence of estrogen. This interaction was enhanced in the presence of estrogen, but was reduced in the presence of the antiestrogen tamoxifen. TRAP220 gene amplification can be found in approximately 24% of breast tumors and approximately 30% of breast cancer cell lines.

SIX1

The vertebrate SIX genes are homologs of the Drosophila 'sine oculis' (so) gene, which is expressed primarily in the developing visual system of the fly. Members of the SIX gene family encode proteins that are characterized by a divergent DNA-binding homeodomain and an upstream SIX domain, which may be involved both in determining DNA-binding specificity and in mediating protein-protein interactions. Genes in the SIX family have been shown to play roles in vertebrate and insect development or have been implicated in maintenance of the differentiated state of tissues. Researchers cloned the human SIX1 homeobox gene from late S phase mammary carcinoma cells and demonstrated that overexpression of SIX1 leads to an abrogation of the DNA damage-induced G2 cell cycle checkpoint. In addition, they found that overexpression of SIX1 occurs in a large percentage of mammary carcinomas and correlates strongly with metastatic breast disease. It appeared that studies of several cancer cell lines suggested that SIX1 may be overexpressed in multiple types of tumors. Thus, the studies linked SIX1 to the cell cycle as well as to tumor progression and provided further evidence that 'master regulators' involved in development may contribute to tumorigenicity.

CDC20

A protein, designated p55CDC or CDC20, that is homologous to the S. cerevisiae cell division cycle 20 protein, in cycling mammalian cells has been identified. This transcript is detectable in all exponentially growing cell lines but disappears when cells are chemically induced to differentiate. The p55CDC protein is essential for cell division. Immunoprecipitation of p55CDC yielded protein complexes with kinase activity that fluctuated during the cell cycle. Since p55CDC did not have the conserved protein kinase domains, this activity must be due to one or more of the associated proteins in the immune complex. The highest levels of protein kinase activity were seen with alpha-casein and myelin basic protein as substrates and demonstrated a pattern of activity distinct from that described for the known cyclin-dependent cell division kinases. The p55CDC protein was also phosphorylated in dividing cells. The 499- amino acid sequence of p55CDC contains 7 repeats homologous to the beta subunit of G proteins. The highest degree of homology in these repeats was found with the S. cerevisiae Cdc20 and Cdc4 proteins, which have been proposed to be involved in the formation of a functional bipolar mitotic spindle in yeast cells. The G beta repeat has been postulated to mediate protein-protein interactions and, in p55CDC, may modulate its association with a unique cell cycle protein kinase.

CDC20 is a component of the mammalian cell cycle mechanism. Activation of the anaphase-promoting complex (APC) is required for anaphase initiation and for exit from mitosis. APC is activated during mitosis and G1 by 2 regulatory factors, CDC20 and HCDH1. These proteins directly bind to APC and activate its cyclin ubiquitination activity.

MAD1 and CDC20 contain MAD2-binding motifs that share a common consensus, and a class of MAD2-binding peptides (MBPs) with a similar consensus was identified. Binding of one of these ligands, MBP1, triggered an extensive rearrangement of the tertiary structure of MAD2. MAD2 also underwent a similar striking structural change upon binding to a MAD1 or CDC20 binding motif peptide. These data suggested that, upon checkpoint activation, MAD1 recruits MAD2 to unattached kinetochores and may promote binding of MAD2 to CDC20.

CCNB2

Two B-type cyclins, B1 and B2, have been identified in mammals. Proliferating cells express both cyclins, which bind to and activate p34 (CDC2). Cyclin B1 proved to be an essential gene; no homozygous B1-null pups were born. In contrast, nullizygous B2 mice developed normally and did not display any obvious abnormalities. Both male and female cyclin B2-null mice were fertile, which was unexpected in view of the high levels and distinct patterns of expression of cyclin B2 during spermatogenesis. The expression of cyclin B1 overlaps the expression of cyclin B2 in the mature testis, but not vice versa. Cyclin B1 can be found both on intracellular membranes and free in the cytoplasm, in contrast to cyclin B2, which is membrane-associated. These observations suggested that cyclin B1 may compensate for the loss of cyclin B2 in the mutant mice, and implies that cyclin B1 is capable of targeting the p34(CDC2) kinase to the essential substrates of cyclin B2.

GAS1

The overexpression of the human GAS1 gene is able to block cell proliferation in lung and bladder carcinoma cell lines, but not in an osteosarcoma cell line or in an adenovirus-type-5 transformed cell line. GAS1 overexpression experiment futher suggesting that the retinoblastoma and/or p53 gene products have an active role in mediating the growth-suppressing effect of GAS1. GAS1 was the first gene to be mapped to both human chromosome 9 and mouse chromosome 13. The location of GAS1 at a site of deletion in myeloid malignancies, together with the demonstration that GAS1 suppresses DNA synthesis, suggested that it is a tumor suppressor gene. GAS1 gene to 9q21.3-q22.1 in a region considered to be a fragile site. Observations suggesting involvement of this area in bladder carcinoma were cited

DCN

Decorin and biglycan are related but distinct small proteoglycans found in many connective tissues. Patients with osteogenesis imperfecta showed a gly415-to-ser mutation of the COL1A1 gene a partner of DCN. When expressed ectopically, decorin is capable of suppressing the growth of various tumor cell lines. Some researchers demonstrated that it induced a marked growth suppression in A431 squamous carcinoma cells, when either exogenously added or endogenously produced by a transgene. Decorin caused rapid phosphorylation of the EGF receptor and a concurrent activation of mitogen-activated protein (MAP) kinase signal pathway. Thus, EGF and decorin converge functionally to regulate the cell cycle through activation of a common pathway that ultimately leads to growth suppression.

*Polynucleotides*

A "BREAST CANCER GENE" polynucleotide can be single- or double-stranded and comprises a coding sequence or the complement of a coding sequence for a "BREAST CANCER GENE" polypeptide. Degenerate nucleotide sequences encoding human "BREAST CANCER GENE" polypeptides, as well as homologous nucleotide sequences which are at least about 50, 55, 60, 65, 70, preferably about 75, 90, 96, or 98% identical to the nucleotide sequences of SEQUENCE: 1 to also are "BREAST CANCER GENE" polynucleotides. Percent sequence identity between the sequences of two polynucleotides is determined using computer programs such as ALIGN which employ the FASTA algorithm, using an affine gap search with a gap open penalty of -12 and a gap extension penalty of -2. Complementary DNA (cDNA) molecules, species homologues, and variants of "BREAST CANCER GENE" polynucleotides which encode biologically active "BREAST CANCER GENE" polypeptides also are "BREAST CANCER GENE" polynucleotides.

*Preparation of Polynucleotides*

A naturally occurring "BREAST CANCER GENE" polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated "BREAST CANCER GENE" polynucleotides. For example, restriction enzymes and probes can be used to isolate polynucleotide fragments which comprises "BREAST CANCER GENE" nucleotide sequences. Isolated polynucleotides are in preparations which are free or at least 70, 80, or 90% free of other molecules.

"BREAST CANCER GENE" cDNA molecules can be made with standard molecular biology techniques, using "BREAST CANCER GENE" mRNA as a template. Any RNA isolation technique which does not select against the isolation of mRNA may be utilized for the purification of such RNA samples. Additionally, large numbers of tissue samples may readily be processed using techniques well known to those of skill in the art, such as, for example, the single-step RNA isolation process of Chomczynski, P. (1989, U.S. Pat. No. 4,843,155).

"BREAST CANCER GENE" cDNA molecules can thereafter be replicated using molecular biology techniques known in the art and disclosed in manuals such as Sambrook et al., 1989, (6) . An amplification technique, such as PCR, can be used to obtain additional copies of polynucleotides, using either human genomic DNA or cDNA as a template.

Alternatively, synthetic chemistry techniques can be used to synthesizes "BREAST CANCER GENE" polynucleotides. The degeneracy of the genetic code allows alternate nucleotide sequences to be synthesized which will encode a "BREAST CANCER GENE" polypeptide or a biologically active variant thereof.

*Identification of differential expression*

Transcripts within the collected RNA samples which represent RNA produced by differentially expressed genes may be identified by utilizing a variety of methods which are ell known to those of skill in the art. For example, differential screening (7), subtractive hybridization (8) and, preferably, differential display (Liang, P., and Pardee, A. B., 1993, U.S. Pat. No. 5,262,311), may be utilized to identify polynucleotide sequences derived from genes that are differentially expressed.

Differential screening involves the duplicate screening of a cDNA library in which one copy of the library is screened with a total cell cDNA probe corresponding to the mRNA population of one cell type while a duplicate copy of the cDNA library is screened with a total cDNA probe corresponding to the mRNA population of a second cell type. For example, one cDNA probe may correspond to a total cell cDNA probe of a cell type derived from a control subject, while the second cDNA probe may correspond to a total cell cDNA probe of the same cell type derived from an experimental subject. Those clones which hybridize to one probe but not to the other potentially represent clones derived from genes differentially expressed in the cell type of interest in control versus experimental subjects.

Subtractive hybridization techniques generally involve the isolation of mRNA taken from two different sources, e.g., control and experimental tissue, the hybridization of the mRNA or single-stranded cDNA reverse-transcribed from the isolated mRNA, and the removal of all hybridized, and therefore double-stranded, sequences. The remaining non-hybridized, single-stranded cDNAs, potentially represent clones derived from genes that are differentially expressed in the two mRNA sources. Such single-stranded cDNAs is then used as the starting material for the construction of a library comprising clones derived from differentially expressed genes.

The differential display technique describes a procedure, utilizing the well known polymerase chain reaction (PCR; the experimental embodiment set forth in Mullis, K. B., 1987, U.S. Pat. No. 4,683,202) which allows for the identification of sequences derived from genes which are differentially expressed. First, isolated RNA is reverse-transcribed into single-stranded cDNA, utilizing standard techniques which are well known to those of skill in the art. Primers for the reverse transcriptase reaction may include, but are not limited to, oligo dT-containing primers, preferably of the reverse primer type of oligonucleotide described below. Next, this technique uses pairs of PCR primers, as described below, which allow for the amplification of clones representing a random subset of the RNA transcripts present within any given cell. Utilizing different pairs of primers allows each of the mRNA transcripts present in a cell to be amplified. Among such amplified transcripts may be identified those which have been produced from differentially expressed genes.

The reverse oligonucleotide primer of the primer pairs may contain an oligo dT stretch of nucleotides, preferably eleven nucleotides long, at its 5' end, which hybridizes to the poly(A) tail of mRNA or to the complement of a cDNA reverse transcribed from an mRNA poly(A) tail. Second, in order to increase the specificity of the reverse primer, the primer may contain one or more, preferably two, additional nucleotides at its 3' end. Because, statistically, only a subset of the mRNA derived sequences present in the sample of interest will hybridize to such primers, the additional nucleotides allow the primers to amplify only a subset of the mRNA derived sequences present in the sample of interest. This is preferred in that it allows more accurate and complete visualization and characterization of each of the bands representing amplified sequences.

The forward primer may contain a nucleotide sequence expected, statistically, to have the ability to hybridize to cDNA sequences derived from the tissues of interest. The nucleotide sequence may be an arbitrary one, and the length of the forward oligonucleotide primer may range from about 9 to about 13 nucleotides, with about 10 nucleotides being preferred. Arbitrary primer sequences cause the lengths of the amplified partial cDNAs produced to be variable, thus allowing different clones to be separated by using standard denaturing sequencing gel electrophoresis. PCR reaction conditions should be chosen which optimize amplified product yield and specificity, and, additionally, produce amplified products of lengths which may be resolved utilizing standard gel electrophoresis techniques. Such reaction conditions are well known to those of skill in the art, and important reaction parameters include, for example, length and nucleotide sequence of oligonucleotide primers as discussed above, and annealing and elongation step temperatures and reaction times. The pattern of clones resulting from the reverse transcription and amplification of the mRNA of two different cell types is displayed via sequencing gel electrophoresis and compared. Differences in the two banding patterns indicate potentially differentially expressed genes.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' nontranscribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate software (e.g. GENOTYPER and Sequence NAVIGATOR, Perkin Elmer; ABI), and the entire process from loading of samples to computer analysis and electronic data display can be computer controlled. Capillary electrophoresis is especially preferable for the sequencing of small pieces of DNA which might be present in limited amounts in a particular sample.

Once potentially differentially expressed gene sequences have been identified via bulk techniques such as, for example, those described above, the differential expression of such putatively differentially expressed genes should be corroborated. Corroboration may be accomplished via, for example, such well known techniques as Northern analysis and/or RT-PCR. Upon corroboration, the differentially expressed genes may be further characterized, and may be identified as target and/or marker genes, as discussed, below.

Also, amplified sequences of differentially expressed genes obtained through, for example, differential display may be used to isolate full length clones of the corresponding gene. The full length coding portion of the gene may readily be isolated, without undue experimentation, by molecular biological techniques well known in the art. For example, the isolated differentially expressed amplified fragment may be labeled and used to screen a cDNA library. Alternatively, the labeled fragment may be used to screen a genomic library.

An analysis of the tissue distribution of the mRNA produced by the identified genes may be conducted, utilizing standard techniques well known to those of skill in the art. Such techniques may include, for example, Northern analyses and RT-PCR Such analyses provide information as to whether the identified genes are expressed in tissues expected to contribute to breast cancer. Such analyses may also provide quantitative information regarding steady state mRNA regulation, yielding data concerning which of the identified genes exhibits a high level of regulation in, preferably, tissues which may be expected to contribute to breast cancer.

Such analyses may also be performed on an isolated cell population of a particular cell type derived from a given tissue. Additionally, standard in situ hybridization techniques may be utilized to provide information regarding which cells within a given tissue express the identified gene. Such analyses may provide information regarding the biological function of an identified gene relative to breast cancer in instances wherein only a subset of the cells within the tissue is thought to be relevant to breast cancer.

*Extending Polynucleotides*

In one embodiment of such a procedure for the identification and cloning of full length gene sequences, RNA may be isolated, following standard procedures, from an appropriate tissue or cellular source. A reverse transcription reaction may then be performed on the RNA using an oligonucleotide primer complimentary to the mRNA that corresponds to the amplified fragment, for the priming of first strand synthesis. Because the primer is anti-parallel to the mRNA, extension will proceed toward the 5' end of the mRNA. The resulting RNA hybrid may then be "tailed" with guanines using a standard terminal transferase reaction, the hybrid may be digested with RNase H, and second strand synthesis may then be primed with a poly-C primer. Using the two primers; the 5' portion of the gene is amplified using PCR. Sequences obtained may then be isolated and recombined with previously isolated sequences to generate a full-length cDNA of the differentially expressed genes.

Various PCR-based methods can be used to extend the polynucleotide sequences disclosed herein to detect upstream sequences such as promoters and regulatory elements. For example, restriction site PCR uses universal primers to retrieve unknown sequence adjacent to a known locus (9). Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region (10). Primers can be designed using commercially available software, such as OLIGO 4.06 Primer Analysis software (National Biosciences Inc., Plymouth, Minn.), to be e.g. 2230 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA (11). In this method, multiple restriction enzyme digestions and ligations also can be used to place an engineered double-stranded sequence into an unknown fragment of the DNA molecule before performing PCR.

Additionally, PCR, nested primers, and PROMOTERFINDER libraries (CLONTECH, Palo Alto, Calif.) can be used to walk genomic DNA (CLONTECH, Palo Alto, Calif.). This process avoids the need to screen libraries and is useful in finding intron/exon junctions.

The sequences of the identified genes may be used, utilizing standard techniques, to place the genes onto genetic maps, e.g., mouse and human genetic maps (12). Such mapping information may yield information regarding the genes' importance to human disease by, for example, identifying genes which map near genetic regions to which known genetic breast cancer tendencies map.

*Identification of Polynucleotide Variants and Homologues or splice Variants*

Variants and homologues of the "BREAST CANCER GENE" polynucleotides described above also are "BREAST CANCER GENE" polynucleotides. Typically, homologous "BREAST CANCER GENE" polynucleotide sequences can be identified by hybridization of candidate polynucleotides to known "BREAST CANCER GENE" polynucleotides under stringent conditions, as is known in the art. For example, using the following wash conditions: 2X SSC (0.3 M NaCl, 0.03 M sodium citrate, pH 7.0), 0.1% SDS, room temperature twice, 30 minutes each; then 2X SSC, 0.1% SDS, 50 EC once, 30 minutes; then 2X SSC, room temperature twice, 10 minutes each homologous sequences can be identified which contain at most about 25-30% basepair mismatches. More preferably, homologous polynucleotide strands contain 15-25% basepair mismatches, even more preferably 5-15% basepair mismatches.

Species homologues of the "BREAST CANCER GENE" polynucleotides disclosed herein also can be identified by making suitable probes or primers and screening cDNA expression libraries from other species, such as mice, monkeys, or yeast. Human variants of "BREAST CANCER GENE" polynucleotides can be identified, for example, by screening human cDNA expression libraries. It is well known that the $T_m$ of a double-stranded DNA decreases by 1°C-1.5°C with every 1% decrease in homology (13). Variants of human "BREAST CANCER GENE" polynucleotides or "BREAST CANCER GENE" polynucleotides of other species can therefore be identified by hybridizing a putative homologous "BREAST CANCER GENE" polynucleotide with a polynucleotide having a nucleotide sequence of one of the genes of the SEQUENCE: 1 to 86 or the complement thereof to form a test hybrid. The melting temperature of the test hybrid is compared with the melting temperature of a hybrid comprising polynucleotides having perfectly complementary nucleotide sequences, and the number or percent of basepair mismatches within the test hybrid is calculated.

Nucleotide sequences which hybridize to "BREAST CANCER GENE" polynucleotides or their complements following stringent hybridization and/or wash conditions also are "BREAST CANCER GENE" polynucleotides. Stringent wash conditions are well known and understood in the art. Typically, for stringent hybridization conditions a combination of temperature and salt concentration should be chosen that is approximately 12 to 20°C below the calculated $T_m$ of the hybrid under study. The $T_m$ of a hybrid between a "BREAST CANCER GENE" polynucleotide having a nucleotide sequence of one of the sequences of the SEQUENCE: 1 to 86 or the complement thereof and a polynucleotide sequence which is at least about 50, preferably about 75, 90, 96, or 98% identical to one of those nucleotide sequences can be calculated, for example, using the equation below (14):

$$T_m = 81.5°C - 16.6(\log_{10}[Na^+]) + 0.41(\%G + C) - 0.63(\%formamide) - 600/l,$$

where 1= the length of the hybrid in basepairs.

Stringent wash conditions include, for example, 4X SSC at 65°C, or 50% formamide, 4X SSC at 28°C, or 0.5X SSC, 0.1% SDS at 65°C. Highly stringent wash conditions include, for example, 0.2X SSC at 65°C.

The biological function of the identified genes may be more directly assessed by utilizing relevant in vivo and in vitro systems. In vivo systems may include, but are not limited to, animal systems which naturally exhibit breast cancer predisposition, or ones which have been engineered to exhibit such symptoms, including but not limited to oncogene overexpression (e.g. HER2/neu, ras, raf, or EGFR) malignant neoplasia mouse.

Splice variants derived from the same genomic region, encoded by the same pre mRNA can be identified by hybridization conditions described above for homology search. The specific characteristics of variant proteins encoded by splice variants of the same pre transcript may differ and can also be assayed as disclosed. A "BREAST CANCER GENE" polynucleotide having a nucleotide sequence of one of the sequences of the SEQUENCE: 1 to 86 or the complement thereof may therefor differ in parts of the entire sequence. The prediction of splicing events and the identification of the utilized acceptor and donor sites within the pre mRNA can be computed (e.g. Software Package GRAIL or GenomeSCAN) and verified by PCR method by those with skill in the art.

*Antisense oligonucleotides*

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 6 nucleotides in length, but can be at least 7, 8, 10, 12, 15, 20, 25, 30, 35, 40, 45, or 50 or more nucleotides long. Longer sequences also can be used. Antisense oligonucleotide molecules can be provided in a DNA construct and introduced into a cell as described above to alter the level of "BREAST CANCER GENE" gene products in the cell.

Antisense oligonucleotides can be deoxyribonucleotides, ribonucleotides, peptide nucleic acids (PNAs; described in U.S. Pat. No. 5,714,331), locked nucleic acids (LNAs; described in WO 99/12826), or a combination of them. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters (15).

Modifications of "BREAST CANCER GENE" expression can be obtained by designing antisense oligonucleotides which will form duplexes to the control, 5', or regulatory regions of the "BREAST CANCER GENE". Oligonucleotides derived from the transcription initiation site, e.g., between positions 10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature (16). An antisense oligonucleotide also can be designed to block translation of mRNA by preventing the transcript from binding to ribosomes.

Precise complementarity is not required for successful complex formation between an antisense oligonucleotide and the complementary sequence of a "BREAST CANCER GENE" polynucleotide. Antisense oligonucleotides which comprise, for example, 2, 3, 4, or 5 or more stretches of contiguous nucleotides which are precisely complementary to a "BREAST CANCER GENE" polynucleotide, each separated by a stretch of contiguous nucleotides which are not complementary to adjacent "BREAST CANCER GENE" nucleotides, can provide sufficient targeting specificity for "BREAST CANCER GENE" mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular "BREAST CANCER GENE" polynucleotide sequence.

Antisense oligonucleotides can be modified without affecting their ability to hybridize to a "BREAST CANCER GENE" polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5' substituted oligonucleotide in which the 3' hydroxyl group or the 5' phosphate group are substituted, also can be employed in a modified antisense oligonucleotide. These modified oligonucleotides can be prepared by methods well known in the art (17).

*Ribozymes*

Ribozymes are RNA molecules with catalytic activity (18). Ribozymes can be used to inhibit gene function by cleaving an RNA sequence, as is known in the art (e.g., Haseloff et al., U.S. Patent 5,641,673). The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences.

The transcribed sequence of a "BREAST CANCER GENE" can be used to generate ribozymes which will specifically bind to mRNA transcribed from a "BREAST CANCER GENE" genomic locus. Methods of designing and constructing ribozymes which can cleave other RNA molecules in trans in a highly sequence specific manner have been developed and described in the art (19). For example, the cleavage activity of ribozymes can be targeted to specific RNAs by engineering a discrete "hybridization" region into the ribozyme. The hybridization region contains a sequence complementary to the target RNA and thus specifically hybridizes with the target [see, for example, Gerlach et al., EP 0 321201].

Specific ribozyme cleavage sites within a "BREAST CANCER GENE" RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate "BREAST CANCER GENE" RNA targets also can be evaluated by testing accessibility to hybridization with complementary oligonucleotides using ribonuclease protection assays. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease "BREAST CANCER GENE" expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells.

As taught in Haseloff et al., U.S Pat. No. 5,641,673, ribozymes can be engineered so that ribozyme expression will occur in response to factors which induce expression of a target gene. Ribozymes also can be engineered to provide an additional level of regulation, so that destruction of mRNA occurs only when both a ribozyme and a target gene are induced in the cells.

*Polypeptides*

"BREAST CANCER GENE" polypeptides comprise an a polypeptide selected from SEQUENCE: 1 to 86 or encoded by any of the polynucleotide sequences of the SEQUENCE: 1 to 86 or derivatives, fragments, analogues and homologues thereof. A BREAST CANCER GENE" polypeptide therefore can be a portion, a full-length, or a fusion protein comprising all or a portion of a "BREAST CANCER GENE" polypeptide.

*Protein Purification*

"BREAST CANCER GENE" polypeptides can be purified from any cell which expresses the responding protein, including host cells which have been transfected with "BREAST CANCER GENE" expression constructs.. A purified "BREAST CANCER GENE" polypeptide is separated from other compounds which are normally associate with the "BREAST CANCER GENE" polypeptide in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis. A preparation of purified "BREAST CANCER GENE" polypeptides is at least 80% pure; preferably, the preparations are 90%, 95%, or 99% pure. Purity of the preparations can be assessed by any means known in the art, such as SDS-polyacrylamide gel electrophoresis.

*Obtaining Polypeptides*

"BREAST CANCER GENE" polypeptides can be obtained, for example, by purification from human cells, by expression of "BREAST CANCER GENE" polynucleotides, or by direct chemical synthesis.

*Biologically Active Variants*

"BREAST CANCER GENE" polypeptide variants which are biologically active, i.e., retain an "BREAST CANCER GENE" activity, can be also regarded as "BREAST CANCER GENE" polypeptides. Preferably, naturally or non-naturally occurring "BREAST CANCER GENE" polypeptide variants have amino acid sequences which are at least about 60, 65, or 70, preferably about 75, 80, 85, 90, 92, 94, 96, or 98% identical to any of the amino acid sequences of the polypeptides of SEQUENCE: 1 to 86 encoded by the genes in Table 1 or the polypeptides encoded by any of the polynucleotides encoded by SEQUENCE: 1 to 86 or a fragment thereof. Percent identity between a putative "BREAST CANCER GENE" polypeptide variant and of the polypeptides of SEQUENCE: 1 to 86 polypeptides encoded by any of the polynucleotides of SEQUENCE: 1 to 86 or a fragment thereof is determined by conventional methods. [See, for example, (20) and (21)]. Briefly, two amino acid sequences are aligned to optimize the alignment scores using a gap opening penalty of 10, a gap extension penalty of 1, and the "BLOSUM62" scoring matrix of Henikoff & Henikoff, 1992 (21).

Those skilled in the art appreciate that there are many established algorithms available to align two amino acid sequences. The "FASTA" similarity search algorithm of Pearson & Lipman is a suitable protein alignment method for examining the level of identity shared by an amino acid sequence disclosed herein and the amino acid sequence of a putative variant (22). Briefly, FASTA first characterizes sequence similarity by identifying regions shared by the query sequence (e.g., SEQUENCE: 1 to 86) and a test sequence that have either the highest density of identities (if the ktup variable is 1) or pairs of identities (if ktup=2), without considering conservative amino acid substitutions, insertions, or deletions. The ten regions with the highest density of identities are then rescored by comparing the similarity of all paired amino acids using an amino acid substitution matrix, and the ends of the regions are "trimmed" to include only those residues that contribute to the highest score. If there are several regions with scores greater than the "cutoff" value (calculated by a predetermined formula based upon the length of the sequence the ktup value), then the trimmed initial regions are examined to determine whether the regions can be joined to form an approximate alignment with gaps. Finally, the highest scoring regions of the two amino acid sequences are aligned using a modification of the Needleman-Wunsch-Sellers algorithm (23 ; 24), which allows for amino acid insertions and deletions. Preferred parameters for FASTA analysis are: ktup=1, gap opening penalty=10, gap extension penalty=1, and substitution matrix=BLOSUM62. These parameters can be introduced into a FASTA program by modifying the scoring matrix file ("SMATRIX"), as explained in Appendix 2 of Pearson, (22).

FASTA can also be used to determine the sequence identity of nucleic acid molecules using a ratio as disclosed above. For nucleotide sequence comparisons, the ktup value can range between one to six, preferably from three to six, most preferably three, with other parameters set as default.

Variations in percent identity can be due, for example, to amino acid substitutions, insertions, or deletions. Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of a "BREAST CANCER GENE" polypeptide can be found using computer programs well known in the art, such as DNASTAR software. Whether an amino acid change results in a biologically active "BREAST CANCER GENE" polypeptide can readily be determined by assaying for "BREAST CANCER GENE" activity, as described for example, in the specific Examples, below. Larger insertions or deletions can also be caused by alternative splicing. Protein domains can be inserted or deleted without altering the main activity of the protein.

*Fusion Proteins*

Fusion proteins are useful for generating antibodies against "BREAST CANCER GENE" polypeptide amino acid sequences and for use in various assay systems. For example, fusion proteins can be used to identify proteins which interact with portions of a "BREAST CANCER GENE" polypeptide. Protein affinity chromatography or library-based assays for protein-protein interactions, such as the yeast two-hybrid or phage display systems, can be used for this purpose. Such methods are well known in the art and also can be used as drug screens.

A "BREAST CANCER GENE" polypeptide fusion protein comprises two polypeptide segments fused together by means of a peptide bond. The first polypeptide segment comprises at least 25, 50, 75, 100, 150, 200, 300, 400, 500, 600, 700 or 750 contiguous amino acids of an amino acid sequence encoded by any polynucleotide sequences of the SEQUENCE: 1 to 86 or of a biologically active variant, such as those described above. The first polypeptide segment also can comprise full-length "BREAST CANCER GENE".

The second polypeptide segment can be a full-length protein or a protein fragment. Proteins commonly used in fusion protein construction include β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S- tag, Lex a DNA binding domain (DBD) fusions, GAL4 DNA binding domain fusions, and herpes simplex virus (HSV) BP16 protein fusions. A fusion protein also can be engineered to contain a cleavage site located between the "BREAST CANCER GENE" polypeptide-encoding sequence and the heterologous protein sequence, so that the "BREAST CANCER GENE" polypeptide can be cleaved and purified away from the heterologous moiety.

A fusion protein can be synthesized chemically, as is known in the art. Preferably, a fusion protein is produced by covalently linking two polypeptide segments or by standard procedures in the art of molecular biology. Recombinant DNA methods can be used to prepare fusion proteins, for example, by making a DNA construct which comprises coding sequences selected from any of the polynucleotide sequences of the SEQUENCE: 1 to 86 in proper reading frame with nucleotides encoding the second polypeptide segment and expressing the DNA construct in a host cell, as is known in the art. Many kits for constructing fusion proteins are available from companies such as Promega Corporation (Madison, WI), Stratagene (La Jolla, CA), CLONTECH (Mountain View, CA), Santa Cruz Biotechnology (Santa Cruz, CA), MBL International Corporation (MIC; Watertown, MA), and Quantum Biotechnologies (Montreal, Canada; 1-888-DNA-KITS).

*Identification of Species Homologues*

Species homologues of human a "BREAST CANCER GENE" polypeptide can be obtained using "BREAST CANCER GENE" polynucleotides (described below) to make suitable probes or primers for screening cDNA expression libraries from other species, such as mice, monkeys, or yeast, identifying cDNAs which encode homologues of a "BREAST CANCER GENE" polypeptide, and expressing the cDNAs as is known in the art.

*Expression of Polynucleotides*

To express a "BREAST CANCER GENE" polynucleotide, the polynucleotide can be inserted into an expression vector which contains the necessary elements for the transcription and translation of the inserted coding sequence. Methods which are well known to those skilled in the art can be used to construct expression vectors containing sequences encoding "BREAST CANCER GENE" polypeptides and appropriate transcriptional and translational control elements. These methods include in vitro recombinant DNA techniques, synthetic techniques, and in vivo genetic recombination. Such techniques are described, for example (6).

A variety of expression vector/host systems can be utilized to contain and express sequences encoding a "BREAST CANCER GENE" polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (e.g., baculovirus), plant cell systems transformed with virus expression vectors (e.g., cauliflower mosaic virus, CaMV; tobacco mosaic virus, TMV) or with bacterial expression vectors (e.g., Ti or pBR322 plasmids), or animal cell systems.

The control elements or regulatory sequences are those regions of the vector enhancers, promoters, 5' and 3' untranslated regions which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (e.g., heat shock, RUBISCO, and storage protein genes) or from plant viruses (e.g., viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding a "BREAST CANCER GENE" polypeptide, vectors based on SV40 or EBV can be used with an appropriate selectable marker.

*Bacterial and Yeast Expression Systems*

In bacterial systems, a number of expression vectors can be selected depending upon the use intended for the "BREAST CANCER GENE" polypeptide. For example, when a large quantity of the "BREAST CANCER GENE" polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the "BREAST CANCER GENE" polypeptide can be ligated into the vector in frame with sequences for the amino terminal Met and the subsequent 7 residues of β-galactosidase so that a hybrid protein is produced. pIN vectors or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione agarose beads followed by elution in the presence of free glutathione. Proteins made in such systems can be designed to include heparin, thrombin, or factor Xa protease cleavage sites so that the cloned polypeptide of interest can be released from the GST moiety at will.
In the yeast Saccharomyces cerevisiae, a number of vectors containing constitutive or inducible promoters such as alpha factor, alcohol oxidase, and PGH can be used.

*Plant and Insect Expression Systems*

If plant expression vectors are used, the expression of sequences encoding "BREAST CANCER GENE" polypeptides can be driven by any of a number of promoters. For example, viral promoters such as the 35S and 19S promoters of CaMV can be used alone or in combination with the omega leader sequence from TMV. Alternatively, plant promoters such as the small subunit of RUBISCO or heat shock promoters can be used. These constructs can be introduced into plant cells by direct DNA transformation or by pathogen-mediated transfection. Such techniques are described in a number of generally available reviews.
An insect system also can be used to express a "BREAST CANCER GENE" polypeptide. For example, in one such system Autographa californica nuclear polyhedrosis virus (AcNPV) is used as a vector to express foreign genes in Spodoptera frugiperda cells or in Trichoplusia larvae. Sequences encoding "BREAST CANCER GENE" polypeptides can be cloned into a nonessential region of the virus, such as the polyhedrin gene, and placed under control of the polyhedrin promoter. Successful insertion of "BREAST CANCER GENE" polypeptides will render the polyhedrin gene inactive and produce recombinant virus lacking coat protein. The recombinant viruses can then be used to infect S. frugiperda cells or Trichoplusia larvae in which "BREAST CANCER GENE" polypeptides can be expressed.

*Mammalian Expression Systems*

A number of viral-based expression systems can be used to express "BREAST CANCER GENE" polypeptides in mammalian host cells. For example, if an adenovirus is used as an expression vector, sequences encoding "BREAST CANCER GENE" polypeptides can be ligated into an adenovirus transcription/translation complex comprising the late promoter and tripartite leader sequence. Insertion in a nonessential E1 or E3 region of the viral genome can be used to obtain a viable virus which is capable of expressing a "BREAST CANCER GENE" polypeptide in infected host cells (25). If desired, transcription enhancers, such as the Rous sarcoma virus (RSV) enhancer, can be used to increase expression in mammalian host cells.
Human artificial chromosomes (HACs) also can be used to deliver larger fragments of DNA than can be contained and expressed in a plasmid. HACs of 6M to 10M are constructed and delivered to cells via conventional delivery methods (e.g., liposomes, polycationic amino polymers, or vesicles).
Specific initiation signals also can be used to achieve more efficient translation of sequences encoding "BREAST CANCER GENE" polypeptides. Such signals include the ATG initiation codon and adjacent sequences. In cases where sequences encoding a "BREAST CANCER GENE" polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used.

*Host Cells*

A host cell strain can be chosen for its ability to modulate the expression of the inserted sequences or to process the expressed "BREAST CANCER GENE" polypeptide in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for Post-translational activities (e.g., CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express "BREAST CANCER GENE" polypeptides can be transformed using expression vectors which can contain viral origins of replication and/or endogenous expression elements and a selectable marker gene on the same or on a separate vector. Following the introduction of the vector, cells can be allowed to grow for 12 days in an enriched medium before they are switched to a selective medium. The purpose of the selectable marker is to confer resistance to selection, and its presence allows growth and recovery of cells which successfully express the introduced "BREAST CANCER GENE" sequences. Resistant clones of stably transformed cells can be proliferated using tissue culture techniques appropriate to the cell type (26).

Any number of selection systems can be used to recover transformed cell lines. These include, but are not limited to, the herpes simplex virus thymidine kinase and adenine phosphoribosyltransferase genes which can be employed in tk of aprt cells, respectively. Also, antimetabolite, antibiotic, or herbicide resistance can be used as the basis for selection. For example, dhfr confers resistance to methotrexate, npt confers resistance to the aminoglycosides, neomycin and G418, and als and pat confer resistance to chlorsulfuron and phosphinotricin acetyltransferase, respectively. Additional selectable genes have been described. For example, trpB allows cells to utilize indole in place of tryptophan, or hisD, which allows cells to utilize histinol in place of histidine. Visible markers such as anthocyanins, β-glucuronidase and its substrate GUS, and luciferase and its substrate luciferin, can be used to identify transformants and to quantify the amount of transient or stable protein expression attributable to a specific vector system.

*Detecting Expression and gene product*

Although the presence of marker gene expression suggests that the "BREAST CANCER GENE" polynucleotide is also present, its presence and expression may need to be confirmed. For example, if a sequence encoding a "BREAST CANCER GENE" polypeptide is inserted within a marker gene sequence, transformed cells containing sequences which encode a "BREAST CANCER GENE" polypeptide can be identified by the absence of marker gene function. Alternatively, a marker gene can be placed in tandem with a sequence encoding a "BREAST CANCER GENE" polypeptide under the control of a single promoter. Expression of the marker gene in response to induction or selection usually indicates expression of the "BREAST CANCER GENE" polynucleotide.

Alternatively, host cells which contain a "BREAST CANCER GENE" polynucleotide and which express a "BREAST CANCER GENE" polypeptide can be identified by a variety of procedures known to those of skill in the art. These procedures include, but are not limited to, DNA-DNA or DNA-RNA hybridization and protein bioassay or immunoassay techniques which include membrane, solution, or chip-based technologies for the detection and/or quantification of polynucleotide or protein. For example, the presence of a polynucleotide sequence encoding a "BREAST CANCER GENE" polypeptide can be detected by DNA-DNA or DNA-RNA hybridization or amplification using probes or fragments or fragments of polynucleotides encoding a "BREAST CANCER GENE" polypeptide. Nucleic acid amplification-based assays involve the use of oligonucleotides selected from sequences encoding a "BREAST CANCER GENE" polypeptide to detect transformants which contain a "BREAST CANCER GENE" polynucleotide.

A variety of protocols for detecting and measuring the expression of a "BREAST CANCER GENE" polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS).

A two-site, monoclonal-based immunoassay using monoclonal antibodies reactive to two non-interfering epitopes on a "BREAST CANCER GENE" polypeptide can be used, or a competitive binding assay can be employed. These and other assays are described in(27).

A wide variety of labels and conjugation techniques are known by those skilled in the art and can be used in various nucleic acid and amino acid assays. Means for producing labeled hybridization or PCR probes for detecting sequences related to polynucleotides encoding "BREAST CANCER GENE" polypeptides include oligo labeling, nick translation, end-labeling, or PCR amplification using a labeled nucleotide. Alternatively, sequences encoding a "BREAST CANCER GENE" polypeptide can be cloned into a vector for the production of an mRNA probe. Such vectors are known in the art, are commercially available, and can be used to synthesize RNA probes in vitro by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of Polypeptides*

Host cells transformed with nucleotide sequences encoding a "BREAST CANCER GENE" polypeptide can be cultured under conditions suitable for the expression and recovery of the protein from cell culture. The polypeptide produced by a transformed cell can be secreted or stored intracellular depending on the sequence and/or the vector used. As will be understood by those of skill in the art, expression vectors containing polynucleotides which encode "BREAST CANCER GENE" polypeptides can be designed to contain signal sequences which direct secretion of soluble "BREAST CANCER GENE" polypeptides through a prokaryotic or eukaryotic cell membrane or which direct the membrane insertion of membrane-bound "BREAST CANCER GENE" polypeptide.

As discussed above, other constructions can be used to join a sequence encoding a "BREAST CANCER GENE" polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the "BREAST CANCER GENE" polypeptide also can be used to facilitate purification. One such expression vector provides for expression of a fusion protein containing a "BREAST CANCER GENE" polypeptide and 6 histidine residues preceding a thioredoxin or an enterokinase cleavage site. The histidine residues facilitate purification by IMAC (immobilized metal ion affinity, while the enterokinase cleavage site provides a means for purifying the "BREAST CANCER GENE" polypeptide from the fusion protein. Vectors which contain fusion proteins are disclosed in (28).

*Chemical Synthesis*

Sequences encoding a "BREAST CANCER GENE" polypeptide can be synthesized, in whole or in part, using chemical methods well known in the art (29). Alternatively, a "BREAST CANCER GENE" polypeptide itself can be produced using chemical methods to synthesize its amino acid sequence, such as by direct peptide synthesis using solid-phase techniques (30). Protein synthesis can be performed using manual techniques or by automation. Automated synthesis can be achieved, for example, using Applied Biosystems 431A Peptide Synthesizer (Perkin Elmer). Optionally, fragments of "BREAST CANCER GENE" polypeptides can be separately synthesized and combined using chemical methods to produce a full-length molecule.

The newly synthesized peptide can be substantially purified by preparative high performance liquid chromatography (31). The composition of a synthetic "BREAST CANCER GENE" polypeptide can be confirmed by amino acid analysis or sequencing (e.g., the Edman degradation procedure). Additionally, any portion of the amino acid sequence of the "BREAST CANCER GENE" polypeptide can be altered during direct synthesis and/or combined using chemical methods with sequences from other proteins to produce a variant polypeptide or a fusion protein.

*Production of Altered Polypeptides*

As will be understood by those of skill in the art, it may be advantageous to produce "BREAST CANCER GENE" polypeptide-encoding nucleotide sequences possessing non-natural occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences disclosed herein can be engineered using methods generally known in the art to alter "BREAST CANCER GENE" polypeptide-encoding sequences for a variety of reasons, including but not limited to, alterations which modify the cloning, processing, and/or expression of the polypeptide or mRNA product. DNA shuffling by random fragmentation and PCR re-assembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*Predictive, Diagnostic and Prognostic Assays*

In clinical applications, biological samples can be screened for the presence and/or absence of the biomarkers identified herein. Such samples are for example needle biopsy cores, surgical resection samples, or body fluids like serum, thin needle nipple aspirates and urine. For example, these methods include obtaining a biopsy, which is optionally fractionated by cryostat sectioning to enrich diseases cells to about 80% of the total cell population. In certain embodiments, polynucleotides extracted from these samples may be amplified using techniques well known in the art. The expression levels of selected markers detected would be compared with statistically valid groups of diseased and healthy samples.

In one embodiment the method comprises determining whether a subject has an abnormal mRNA and/or protein level of the disclosed markers, such as by Northern blot analysis, reverse transcription-polymerase chain reaction (RT-PCR), in situ hybridization, immunoprecipitation, Western blot hybridization, or immunohistochemistry. According to the method, cells are obtained from a subject and the levels of the disclosed biomarkers, protein or mRNA level, is determined and compared to the level of these markers in a healthy subject. An abnormal level of the biomarker polypeptide or mRNA levels is likely to be indicative of malignant neoplasia such as breast cancer.

*1. DNA arrays technology*

The method may employ polynucleotide probes immobilized on a DNA chip in an organized array. Oligonucleotides can be bound to a solid support by a variety of processes, including lithography. For example a chip can hold up to 410.000 oligonucleotides (GeneChip, Affymetrix).

The method includes obtaining a biological sample which can be a biopsy of an affected person, which is optionally fractionated by cryostat sectioning to enrich diseased cells to about 80% of the total cell population and the use of body fluids such as serum or urine, serum or cell containing liquids (e.g. derived from fine needle aspirates). The DNA or RNA is then extracted, amplified, and analyzed with a DNA chip to determine the presence of absence of the marker polynucleotide sequences. In one embodiment, the polynucleotide probes are spotted onto a substrate in a two-dimensional matrix or array. samples of polynucleotides can be labeled and then hybridized to the probes. Double-stranded polynucleotides, comprising the labeled sample polynucleotides bound to probe polynucleotides, can be detected once the unbound portion of the sample is washed away.

The probe polynucleotides can be spotted on substrates including glass, nitrocellulose, etc. The probes can be bound to the substrate by either covalent bonds or by non-specific interactions, such as hydrophobic interactions. The sample polynucleotides can be labeled using radioactive labels, fluorophores, chromophores, etc. Techniques for constructing arrays and methods of using these arrays are described in EP0 799 897; WO 97/29212; WO 97/27317; EP 0 785 280; WO 97/02357; U.S. Pat. No. 5,593,839; U.S. Pat. No. 5,578,832; EP 0 728 520; U.S. Pat. No. 5,599,695; EP 0 721 016; U.S. Pat. No. 5,556,752; WO 95/22058; and U.S. Pat. No. 5,631,734. Further, arrays can be used to examine differential expression of genes and can be used to determine gene function. For example, arrays of the instant polynucleotide sequences can be used to determine if any of the polynucleotide sequences are differentially expressed between normal cells and diseased cells, for example. High expression of a particular message in a diseased sample, which is not observed in a corresponding normal sample, can indicate a breast cancer specific protein.

*2. Data analysis methods*

Comparison of the expression levels of one or more "BREAST CANCER GENES" with reference expression levels, e.g., expression levels in diseased cells of breast cancer or in normal counterpart cells, is preferably conducted using computer systems. Expression levels may be obtained in two cells and these two sets of expression levels are introduced into a computer system for comparison. Preferably, one set of expression levels is entered into a computer system for comparison with values that are already present in the computer system, or in computer-readable form that is then entered into the computer system.

Computer readable form of the gene expression profile data may be provided, or of values corresponding to the level of expression of at least one "BREAST CANCER GENE" in a diseased cell. The values can be mRNA expression levels obtained from experiments, e.g., microarray analysis. The values can also be mRNA levels normalized relative to a reference gene whose expression is constant in numerous cells under numerous conditions, e.g., GAPDH. The values in the computer may be ratios of, or differences between, normalized or non-normalized mRNA levels in different samples.

The gene expression profile data can be in the form of a table, such as an Excel table. The data can be alone, or it can be part of a larger database, e.g., comprising other expression profiles. For example, the expression profile data can be part of a public database. The computer readable form can be in a computer. Alternatively, the computer may display the gene expression profile data.

The invention may employ a method for determining the similarity between the level of expression of one or more "BREAST CANCER GENES" in a first cell, e.g., a cell of a subject, and that in a second cell, comprising obtaining the level of expression of one or more "BREAST CANCER GENES" in a first cell and entering these values into a computer comprising a database including records comprising values corresponding to levels of expression of one or more "BREAST CANCER GENES" in a second cell, and processor instructions, e.g., a user interface, capable of receiving a selection of one or more values for comparison purposes with data that is stored in the computer. The computer may further comprise a means for converting the comparison data into a diagram or chart or other type of output.

Values representing expression levels of "BREAST CANCER GENES" may be entered into a computer system, comprising one or more databases with reference expression levels obtained from more-than-one cell. For example, the computer comprises expression data of diseased and normal cells. Instructions are provided to the computer, and the computer is capable of comparing the data entered with the data in the computer to determine whether the data entered is more similar to that of a normal cell or of a diseased cell.

Alternatively the computer comprises values of expression levels in cells of subjects at different stages of breast cancer, and the computer is capable of comparing expression data entered into the computer with the data stored, and produce results indicating to which of the expression profiles in the computer, the one entered is most similar, such as to determine the stage of breast cancer in the subject.

Further, the reference expression profiles in the computer may be expression profiles from cells of breast cancer of one or more subjects, which cells are treated *in vivo* or *in vitro* with a drug used for therapy of breast cancer. Upon entering of expression data of a cell of a subject treated *in vitro* or *in vivo* with the drug, the computer is instructed to compare the data entered to the data in the computer, and to provide results indicating whether the expression data input into the computer are more similar to those of a cell of a subject that is responsive to the drug or more similar to those of a cell of a subject that is not responsive to the drug. Thus, the results indicate whether the subject is likely to respond to the treatment with the drug or unlikely to respond to it.

Further disclosed is a system that comprises a means for receiving gene expression data for one or a plurality of genes; a means for comparing the gene expression data from each of said one or plurality of genes to a common reference frame; and a means for presenting the results of the comparison. This system may further comprise a means for clustering the data.

Yet further disclosed is a computer program for analyzing gene expression data comprising (i) a computer code that receives as input gene expression data for a plurality of genes and (ii) a computer code that compares said gene expression data from each of said plurality of genes to a common reference frame.

Further disclosed a machine-readable or computer-readable medium including program instructions for performing the following steps: (i) comparing a plurality of values corresponding to expression levels of one or more genes characteristic of breast cancer in a query cell with a database including records comprising reference expression or expression profile data of one or more reference cells and an annotation of the type of cell; and (ii) indicating to which cell the query cell is most similar based on similarities of expression profiles. The reference cells can be cells from subjects at different stages of breast cancer. The reference cells can also be cells from subjects responding or not responding to a particular drug treatment and optionally incubated *in vitro* or *in vivo* with the drug.

The reference cells may also be cells from subjects responding or not responding to several different treatments, and the computer system indicates a preferred treatment for the subject.

The relative abundance of an mRNA in two biological samples can be scored as a perturbation and its magnitude determined (i.e., the abundance is different in the two sources of mRNA tested), or as not perturbed (i.e., the relative abundance is the same). In various embodiments, a difference between the two sources of RNA of at least a factor of about 25% (RNA from one source is 25% more abundant in one source than the other source), more usually about 50%, even more often by a factor of about 2 (twice as abundant), 3 (three times as abundant) or 5 (five times as abundant) is scored as a perturbation. Perturbations can be used by a computer for calculating and expression comparisons.

Preferably, in addition to identifying a perturbation as positive or negative, it is advantageous to determine the magnitude of the perturbation. This can be carried out, as noted above, by calculating the ratio of the emission of the two fluorophores used for differential labeling, or by analogous methods that will be readily apparent to those of skill in the art.

The computer readable medium may further comprise a pointer to a descriptor of a stage of breast cancer or to a treatment for breast cancer.

In operation, the means for receiving gene expression data, the means for comparing the gene expression data, the means for presenting, the means for normalizing, and the means for clustering within the context of the systems can involve a programmed computer with the respective functionalities described herein, implemented in hardware or hardware and software; a logic circuit or other component of a programmed computer that performs the operations specifically identified herein, dictated by a computer program; or a computer memory encoded -with-executable instructions representing a computer program that can cause a computer to function in the particular fashion described herein.

Those skilled in the art will understand that the systems and methods may be applied to a variety of systems, including IBM-compatible personal computers running MS-DOS or Microsoft Windows.

The computer may have internal components linked to external components. The internal components may include a processor element interconnected with a main memory. The computer system can be an Intel Pentium®-based processor of 200 MHz or greater clock rate and with 32 MB or more of main memory. The external component may comprise a mass storage, which can be one or more hard disks (which are typically packaged together with the processor and memory). Such hard disks are typically of 1 GB or greater storage capacity. Other external components include a user interface device, which can be a monitor, together with an inputting device, which can be a "mouse", or other graphic input devices, and/or a keyboard. A printing device can also be attached to the computer.

Typically, the computer system is also linked to a network link, which can be part of an Ethernet link to other local computer systems, remote computer systems, or wide area communication networks, such as the Internet. This network link allows the computer system to share data and processing tasks with other computer systems.

Loaded into memory during operation of this system are several software components, which are both standard in the art and special to the disclosed method. These software components collectively cause the computer system to function according to the method. These software components are typically stored on a mass storage. A software component represents the operating system, which is responsible for managing the computer system and its network interconnections. This operating system can be, for example, of the Microsoft Windows' family, such as Windows 95, Windows 98, or Windows NT. A software component represents common languages and functions conveniently present on this system to assist programs implementing the methods specific to this invention. Many high or low level computer languages can be used to program the analytic methods of this invention. Instructions can be interpreted during run-time or compiled. Preferred languages include C/C++, and JAVA®. Most preferably, the method is programmed in mathematical software packages which allow symbolic entry of equations and high-level specification of processing, including algorithms to be used, thereby freeing a user of the need to procedurally program individual equations or algorithms. Such packages include Matlab from Mathworks (Natick, Mass.), Mathematica from Wolfram Research (Champaign, Ill.), or S-Plus from Math Soft (Cambridge, Mass.). Accordingly, a software component represents the analytic methods as programmed in a procedural language or symbolic package. In a preferred embodiment, the computer system also contains a database comprising values representing levels of expression of one or more genes characteristic of breast cancer. The database may contain one or more expression profiles of genes characteristic of breast cancer in different cells.

In an exemplary implementation, to practice the method, a user first loads expression profile data into the computer system. These data can be directly entered by the user from a monitor and keyboard, or from other computer systems linked by a network connection, or on removable storage media such as a CD-ROM or floppy disk or through the network. Next the user causes execution of expression profile analysis software which performs the steps of comparing and, e.g., clustering co-varying genes into groups of genes.

In another exemplary implementation, expression profiles are compared using a method described in U.S. Patent No. 6,203,987. A user first loads expression profile data into the computer system. Gene set profile definitions are loaded into the memory from the storage media or from a remote computer, preferably from a dynamic gene set database system, through the network. Next the user causes execution of projection software which performs the steps of converting expression profile to projected expression profiles. The projected expression profiles are then displayed.

In yet another exemplary implementation, a user first leads a projected profile into the memory. The user then causes the loading of a reference profile into the memory. Next, the user causes the execution of comparison software which performs the steps of objectively comparing the profiles.

*3. Detection of variant polynucleotide sequence*

Disclosed are methods for determining whether a subject is at risk for developing a disease, such as a predisposition to develop malignant neoplasia, for example breast cancer, associated with an aberrant activity of any one of the polypeptides encoded by any of the polynucleotides of the SEQUENCE: 1 to 86, wherein the aberrant activity of the polypeptide is characterized by detecting the presence or absence of a genetic lesion characterized by at least one of these:

(i) an alteration affecting the integrity of a gene encoding a marker polypeptides, or

(ii) the disregulated expression of the encoding polynucleotide.

To illustrate, such genetic lesions can be detected by ascertaining the existence of at least one of these:

I. a deletion of one or more nucleotides from the polynucleotide sequence

II. an addition of one or more nucleotides to the polynucleotide sequence

III. a substitution of one or more nucleotides of the polynucleotide sequence

IV. a gross chromosomal rearrangement of the polynucleotide sequence

V. a gross alteration in the level of a messenger RNA transcript of the polynucleotide sequence

VI. aberrant modification of the polynucleotide sequence, such as of the methylation pattern of the genomic DNA

VII. the presence of a non-wild type splicing pattern of a messenger RNA transcript of the gene

VIII. a non-wild type level of the marker polypeptide

IX. allelic loss of the gene

X. inappropriate post-translational modification of the marker polypeptide

Assay techniques for detecting mutations in the encoding polynucleotide sequence are disclosed. These methods include, but are not limited to, methods involving sequence analysis, Southern blot hybridization, restriction enzyme site mapping, and methods involving detection of absence of nucleotide pairing between the polynucleotide to be analyzed and a probe.

Specific diseases or disorders, e.g., genetic diseases or disorders, are associated with specific allelic variants of polymorphic regions of certain genes, which do not necessarily encode a mutated protein. Thus, the presence of a specific allelic variant of a polymorphic region of a gene in a subject can render the subject susceptible to developing a specific disease or disorder. Polymorphic regions in genes, can be identified, by determining the nucleotide sequence of genes in populations of individuals. If a polymorphic region is identified, then the link with a specific disease can be determined by studying specific populations of individuals, e.g. individuals which developed a specific disease, such as breast cancer. A polymorphic region can be located in any region of a gene, e.g., exons, in coding or non coding regions of exons, introns, and promoter region.

In an exemplary embodiment, there is provided a polynucleotide composition comprising a polynucleotide probe including a region of nucleotide sequence which is capable of hybridizing to a sense or antisense sequence of a gene or naturally occurring mutants thereof, or 5' or 3' flanking sequences or intronic sequences naturally associated with the subject genes or naturally occurring mutants thereof. The polynucleotide of a cell is rendered accessible for hybridization, the probe is contacted with the polynucleotide of the sample, and the hybridization of the probe to the sample polynucleotide is detected. Such techniques can be used to detect lesions or allelic variants at either the genomic or mRNA level, including deletions, substitutions, etc., as well as to determine mRNA transcript levels.

A preferred detection method is allele specific hybridization using probes overlapping the mutation or polymorphic site and having about 5, 10, 20, 25, or 30 nucleotides around the mutation or polymorphic region. Several probes capable of hybridizing specifically to allelic variants may be attached to a solid phase support, e.g., a "chip". Mutation detection analysis using these chips comprising oligonucleotides, also termed "DNA probe arrays" is described e.g., in (40). In one embodiment, a chip comprises all the allelic variants of at least one polymorphic region of a gene. The solid phase support is then contacted with a test polynucleotide and hybridization to the specific probes is detected. Accordingly, the identity of numerous allelic variants of one or more genes can be identified in a simple hybridization experiment.

Detection of the lesion may comprise utilizing the probe/primer in a polymerase chain reaction (PCR) (see, e.g. U.S. Patent Nos. 4,683,195 and 4,683,202), such as anchor PCR or RACE PCR, or, alternatively, in a ligase chain reaction (LCR) (41), the latter of which can be particularly useful for detecting point mutations in the gene; (42). In a merely illustrative embodiment, the method includes the steps of (i) collecting a sample of cells from a patient, (ii) isolating polynucleotide (e.g., genomic, mRNA or both) from the cells of the sample, (iii) contacting the polynucleotide sample with one or more primers which specifically hybridize to a polynucleotide sequence under conditions such that hybridization and amplification of the polynucleotide (if present) occurs, and (iv) detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication, transcriptional amplification system, Q-Beta, or any other polynucleotide amplification method, followed by the detection of the amplified molecules using techniques well known to those of skill in the art. These detection schemes are especially useful for the detection of polynucleotide molecules if such molecules are present in very low numbers.

Mutations in, or allelic variants, of a gene from a sample cell may be identified by alterations in restriction enzyme cleavage patterns. For example, sample and control DNA is isolated, amplified (optionally), digested with one or more restriction endonucleases, and fragment length sizes are determined by gel electrophoresis. Moreover; the use of sequence specific ribozymes (see, for example, U.S. Patent No. 5;498,531) can be used to score for the presence of specific mutations by development or loss of a ribozyme cleavage site.

*4. In situ hybridization*

In one aspect, the method comprises *in situ* hybridization with a probe derived from a given marker polynucleotide, which sequence is selected from any of the polynucleotide sequences of the SEQUENCE: 1 to 86 or a sequence complementary thereto. The method comprises contacting the labeled hybridization probe with a sample of a given type of tissue from a patient potentially having malignant neoplasia and breast cancer in particular as well as normal tissue from a person with no malignant neoplasia, and determining whether the probe labels tissue of the patient to a degree significantly different (e.g., by at least a factor of two, or at least a factor of five, or at least a factor of twenty, or at least a factor of fifty) than the degree to which normal tissue is labeled. In situ hybridization may be performed either to DNA in the nucleus of said cell in tissues or to the mRNA in the cytoplasm to stain for transcriptional activity.

*Polypeotide detection*

Further disclosed is a method of determining whether a cell sample obtained from a subject possesses an abnormal amount of marker polypeptide which comprises (a) obtaining a cell sample from the subject, (b) quantitatively determining the amount of the marker polypeptide in the sample so obtained, and (c) comparing the amount of the marker polypeptide so determined with a known standard, so as to thereby determine whether the cell sample obtained from the subject possesses an abnormal amount of the marker polypeptide. Such marker polypeptides may be detected by immunohistochemical assays, dot-blot assays, ELISA and the like.

*Antibodies*

Any type of antibody known in the art can be generated to bind specifically to an epitope of a "BREAST CANCER GENE" polypeptide. An antibody as used herein includes intact immunoglobulin molecules, as well as fragments thereof, such as Fab, $F(ab)_2$, and Fv, which are capable of binding an epitope of a "BREAST CANCER GENE" polypeptide. Typically, at least 6, 8, 10, or 12 contiguous amino acids are required to form an epitope. However, epitopes which involve non-contiguous amino acids may require more, e.g., at least 15, 25, or 50 amino acids.

An antibody which specifically binds to an epitope of a "BREAST CANCER GENE" polypeptide can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassay typically involve the measurement of complex formation between an immunogen and an antibody which specifically binds to the immunogen.

Typically, an antibody which specifically binds to a "BREAST CANCER GENE" polypeptide provides a detection signal at least 5-, 10-, or 20-fold higher than a detection signal provided with other proteins when used in an immunochemical assay. Preferably, antibodies which specifically bind to "BREAST CANCER GENE" polypeptides do not detect other proteins in immunochemical assays and can immunoprecipitate a "BREAST CANCER GENE" polypeptide from solution.

"BREAST CANCER GENE" polypeptides can be used to immunize a mammal, such as a mouse, rat, rabbit, guinea pig, monkey, or human, to produce polyclonal antibodies. If desired, a "BREAST CANCER GENE" polypeptide can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (e.g., aluminum hydroxide), and surface active substances (e.g. lysolecithin, pluronic polyols, polyanions, peptides, oil emulsions, keyhole limpet hemocyanin, and dinitrophenol). Among adjuvants used in humans, BCG (bacilli Calmette-Guerin) and Corynebacterium parvum are especially useful.

Monoclonal antibodies which specifically bind to a "BREAST CANCER GENE" polypeptide can be prepared using any technique which provides for the production of antibody molecules by continuous cell lines in culture. These techniques include, but are not limited to, the well known hybridoma technique, the human B cell hybridoma technique, and the EBV hybridoma technique.

In addition, techniques developed for the production of chimeric antibodies, the splicing of mouse antibody genes to human antibody genes to obtain a molecule with appropriate antigen specificity and biological activity, can be used (46). Monoclonal and other antibodies also can be humanized to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Alternatively, humanized antibodies can be produced using recombinant methods, as described in GB2188638B. Antibodies which specifically bind to a "BREAST CANCER GENE" polypeptide can contain antigen binding sites which are either partially or fully humanized, as disclosed in U.S. Patent 5,565,332.

Alternatively, techniques described for the production of single chain antibodies can be adapted using methods known in the art to produce single chain antibodies which specifically bind to "BREAST CANCER GENE" polypeptides. Antibodies with related specificity, but of distinct idiotypic composition, can be generated by chain shuffling from random combinatorial immunoglobulin libraries (47).

Single-chain antibodies also can be constructed using a DNA amplification method, such as PCR, using hybridoma cDNA as a template (48). Single-chain antibodies can be mono- or bispecific, and can be bivalent or tetravalent. Construction of tetravalent, bispecific single-chain antibodies is taught, for example (49). Construction of bivalent, bispecific single-chain antibodies is taught in (50).

A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology.

Antibodies which specifically bind to "BREAST CANCER GENE" polypeptides also can be produced by inducing in vivo production in the lymphocyte population or by screening immunoglobulin libraries or panels of highly specific binding reagents as disclosed in the literature (51).

Other types of antibodies can be constructed and used therapeutically. For example, chimeric antibodies can be constructed as disclosed in WO 93/03151. Binding proteins which are derived from immunoglobulins and which are multivalent and multispecific, such as the antibodies described in WO 94/13804, also can be prepared.

Antibodies can be purified by methods well known in the art. For example, antibodies can be affinity purified by passage over a column to which a "BREAST CANCER GENE" polypeptide is bound. The bound antibodies can then be eluted from the column using a buffer with a high salt concentration.

Immunoassays are commonly used to quantify the levels of proteins in cell samples, and many other immunoassay techniques are known in the art. The method may include both homogeneous and heterogeneous procedures. Exemplary immunoassays which can be conducted include fluorescence polarization immunoassay (FPIA), fluorescence immunoassay (FIA), enzyme immunoassay (EIA), nephelometric inhibition immunoassay (NIA), enzyme linked immunosorbent assay (ELISA), and radioimmunoassay (RIA). An indicator moiety, or label group, can be attached to the subject antibodies and is selected so as to meet the needs of various uses of the method which are often dictated by the availability of assay equipment and compatible immunoassay procedures. General techniques to be used in performing the various immunoassays noted above are known to those of ordinary skill in the art.

Other methods to quantify the level of a particular protein, or a protein fragment, or modified protein in a particular sample are based on flow-cytometric methods. Flow cytometry allows the identification of proteins on the cell surface as well as of intracellular proteins using fluorochrome labeled, protein specific antibodies or non-labeled antibodies in combination with fluorochrome labeled secondary antibodies. General techniques to be used in performing flow cytometric assays noted above are known to those of ordinary skill in the art. A special method based on the same principles is the microsphere-based flow cytometric. Microsphere beads are labeled with precise quantities of fluorescent dye and particular antibodies. Such techniques are provided by Luminex Inc. WO 97/14028. In another embodiment the level of a particular protein or a protein fragment, or modified protein in a particular sample may be determined by 2D gel-electrophoresis and/or mass spectrometry. Determination of protein nature, sequence, molecular mass as well charge can be achieved in one detection step. Mass spectrometry can be performed with methods known to those with skills in the art as MALDI, TOF, or combinations of these.

In another embodiment, the level of the encoded product, i.e., the product encoded by any of the polynucleotide sequences of the SEQUENCE: 1 to 86 or a sequence complementary thereto, in a biological fluid (e.g., blood or urine) of a patient may be determined as a way of monitoring the level of expression of the marker polynucleotide sequence in cells of that patient. Such a method would include the steps of obtaining a sample of a biological fluid from the patient, contacting the sample (or proteins from the sample) with an antibody specific for a encoded marker polypeptide, and determining the amount of immune complex formation by the antibody, with the amount of immune complex formation being indicative of the level of the marker encoded product in the sample. This determination is particularly instructive when compared to the amount of immune complex formation by the same antibody in a control sample taken from a normal individual or in one or more samples previously or subsequently obtained from the same person.

In another embodiment, the method can be used to determine the amount of marker polypeptide present in a cell, which in turn can be correlated with progression of the disorder, e.g., plaque formation. The level of the marker polypeptide can be used predictively to evaluate whether a sample of cells contains cells which are, or are predisposed towards becoming, plaque associated cells. The observation of marker polypeptide level can be utilized in decisions regarding, e.g., the use of more stringent therapies.

As set out above, one aspect of the present invention relates to diagnostic assays for determining, in the context of cells isolated from a patient, if the level of a marker polypeptide is significantly reduced in the sample cells. The term "significantly reduced" refers to a cell phenotype wherein the cell possesses a reduced cellular amount of the marker polypeptide relative to a normal cell of similar tissue origin. For example, a cell may have less than about 50%, 25%, 10%, or 5% of the marker polypeptide that a normal control cell. In particular, the assay evaluates the level of marker polypeptide in the test cells, and, preferably, compares the measured level with marker polypeptide detected in at least one control cell, e.g., a normal cell and/or a transformed cell of known phenotype.

Of particular importance is the ability to quantify the level of marker polypeptide as determined by the number of cells associated with a normal or abnormal marker polypeptide level. The number of cells with a particular marker polypeptide phenotype may then be correlated with patient prognosis. In one embodiment, the marker polypeptide phenotype of the lesion is determined as a percentage of cells in a biopsy which are found to have abnormally high/low levels of the marker polypeptide. Such expression may be detected by immunohistochemical assays, dot-blot assays, ELISA and the like.

*Immunohistochemistry*

Where tissue samples are employed, immunohistochemical staining may be used to determine the number of cells having the marker polypeptide phenotype. For such staining, a multiblock of tissue is taken from the biopsy or other tissue sample and subjected to proteolytic hydrolysis, employing such agents as protease K or pepsin. In certain embodiments, it may be desirable to isolate a nuclear fraction from the sample cells and detect the level of the marker polypeptide in the nuclear fraction.

The tissues samples are fixed by treatment with a reagent such as formalin, glutaraldehyde, methanol, or the like. The samples are then incubated with an antibody, preferably a monoclonal antibody, with binding specificity for the marker polypeptides. This antibody may be conjugated to a Label for subsequent detection of binding. samples are incubated for a time Sufficient for formation of the immunocomplexes. Binding of the antibody is then detected by virtue of a Label conjugated to this antibody. Where the antibody is unlabelled, a second labeled antibody may be employed, e.g., which is specific for the isotype of the anti-marker polypeptide antibody. Examples of labels which may be employed include radionuclides, fluorescence, chemoluminescence, and enzymes.

Where enzymes are employed, the Substrate for the enzyme may be added to the samples to provide a colored or fluorescent product. Examples of suitable enzymes for use in conjugates include horseradish peroxidase, alkaline phosphatase, malate dehydrogenase and the like. Where not commercially available, such antibody-enzyme conjugates are readily produced by techniques known to those skilled in the art.

In one embodiment, the assay is performed as a dot blot assay. The dot blot assay finds particular application where tissue samples are employed as it allows determination of the average amount of the marker polypeptide associated with a Single cell by correlating the amount of marker polypeptide in a cell-free extract produced from a predetermined number of cells.

In yet another embodiment, a panel of antibodies is contemplated which are generated against the marker polypeptides, which polypeptides are encoded by any of the polynucleotide sequences of the SEQUENCE: 1 to 86. Such a panel of antibodies may be used as a reliable diagnostic probe for breast cancer. The assay comprises contacting a biopsy sample containing cells, e.g., macrophages, with a panel of antibodies to one or more of the encoded products to determine the presence or absence of the marker polypeptides.

The diagnostic methods may also be employed as follow-up to treatment, e.g., quantification of the level of marker polypeptides may be indicative of the effectiveness of current or previously employed therapies for malignant neoplasia and breast cancer in particular as well as the effect of these therapies upon patient prognosis.

The diagnostic assays described above can be adapted to be used as prognostic assays, as well. Such an application takes advantage of the sensitivity of the assays to events which take place at characteristic stages in the progression of plaque generation in case of malignant neoplasia. For example, a given marker gene may be up- or down-regulated at a very early stage, perhaps before the cell is developing into a foam cell, while another marker gene may be characteristically up or down regulated only at a much later stage. Such a method could involve the steps of contacting the mRNA of a test cell with a polynucleotide probe derived from a given marker polynucleotide which is expressed at different characteristic levels in breast cancer tissue cells at different stages of malignant neoplasia progression, and determining the approximate amount of hybridization of the probe to the mRNA of the cell, such amount being an indication of the level of expression of the gene in the cell, and thus an indication of the stage of disease progression of the cell; alternatively, the assay can be carried out with an antibody specific for the gene product of the given marker polynucleotide, contacted with the proteins of the test cell. A battery of such tests will disclose not only the existence of a certain neoplastic lesion, but also will allow the clinician to select the mode of treatment most appropriate for the disease, and to predict the likelihood of success of that treatment.

The methods can also be used to follow the clinical course of a given breast cancer predisposition. For example, the assay can be applied to a blood sample from a patient; following treatment of the-patient-for breast cancer, another blood sample is taken and the test repeated. Successful treatment will result in removal of demonstrate differential expression, characteristic of the breast cancer tissue cells, perhaps approaching or even surpassing normal levels.

*Polypeptide activity*

Further disclosed is a method for screening potentially therapeutic agents which modulate the activity of one or more "BREAST CANCER GENE" polypeptides, such that if the activity of the polypeptide is increased as a result of the upregulation of the "BREAST CANCER GENE" in a subject having or at risk for malignant neoplasia and breast cancer in particular, the therapeutic substance will decrease the activity of the polypeptide relative to the activity of the some polypeptide in a subject not having or not at risk for malignant neoplasia or breast cancer in particular but not treated with the therapeutic agent. Likewise, if the activity of the polypeptide as a result of the downregulation of the "BREAST CANCER GENE" is decreased in a subject having or at risk for malignant neoplasia or breast cancer in particular, the therapeutic agent will increase the activity of the polypeptide relative to the activity of the same polypeptide in a subject not having or not at risk for malignant neoplasia or breast cancer in particular, but not treated with the therapeutic agent.

The activity of the "BREAST CANCER GENE" polypeptides indicated in Table1 and 2 may be measured by any means known to those of skill in the art, and which are particular for the type of activity performed by the particular polypeptide. Examples of specific assays which may be used to measure the activity of particular polynucleotides are shown below.

a) G protein coupled receptors

The "BREAST CANCER GENE" polynucleotide may encode a G protein coupled receptor. A method of screening potential modulators (inhibitors or activators) of the G protein coupled receptor may measure changes in the activity of the receptor in the presence of a candidate modulator.

1) $G_i$ -coupled receptors

Cells (such as CHO cells or primary cells) are stably transfected with the relevant receptor and with an inducible CRE-luciferase construct. Cells are grown in 50% Dulbecco's modified Eagle medium / 50% F12 (DMEM/F12) supplemented with 10% FBS, at 37°C in a humidified atmosphere with 10% $CO_2$ and are routinely split at a ratio of 1:10 every 2 or 3 days. Test cultures are seeded into 384 - well plates at an appropriate density (e.g. 2000 cells / well in 35 µl cell culture medium) in DMEM/F12 with FBS, and are grown for 48 hours (range: ∼ 24 - 60 hours, depending on cell line). Growth medium is then exchanged against serum free medium (SFM; e.g. Ultra-CHO), containing 0,1% BSA. Test compounds dissolved in DMSO are diluted in SFM and transferred to the test cultures (maximal final concentration 10 µmolar), followed by addition of forskolin (∼ 1 µmolar, final conc.) in SFM + 0,1% BSA 10 minutes later. In case of antagonist screening both, an appropriate concentration of agonist, and forskolin are added. The plates are incubated at 37°C in 10% $CO_2$ for 3 hours. Then the supernatant is removed, cells are lysed with lysis reagent (25 mmolar phosphate-buffer, pH 7,8, containing 2 mmolar DDT, 10% glycerol and 3% Triton X100). The luciferase reaction is started by addition of substrate-buffer (e.g. luciferase assay reagent, Promega) and luminescence is immediately determined (e.g. Berthold luminometer or Hamamatzu camera system).

2) $G_s$ -coupled receptors

Cells (such as CHO cells or primary cells) are stably transfected with the relevant receptor and with an inducible CRE-luciferase construct. Cells are grown in 50% Dulbecco's modified Eagle medium / 50% F12 (DMEM/F12) supplemented with 10% FBS, at 37°C in a humidified atmosphere with 10% $CO_2$ and are routinely split at a ratio of 1:10 every 2 or 3 days. Test cultures are seeded into 384 - well plates at an appropriate density (e.g. 1000 or 2000 cells / well in

35 $\mu$l cell culture medium) in DMEM/F12 with FBS, and are grown for 48 hours (range: ~ 24 - 60 hours, depending on cell line). The assay is started by addition of test-compounds in serum free medium (SFM; e.g. Ultra-CHO) containing 0,1% BSA: Test compounds are dissolved in DMSO, diluted in SFM and transferred to the test cultures (maximal final concentration 10 $\mu$molar, DMSO conc, < 0,6 %). In case of antagonist screening an appropriate concentration of agonist is added 5 - 10 minutes later. The plates are incubated at 37°C in 10% $CO_2$ for 3 hours. Then the cells are lysed with 10 $\mu$l lysis reagent per well (25 mmolar phosphate-buffer, pH 7,8 , containing 2 mmolar DDT, 10% glycerol and 3% Triton X100) and the luciferase reaction is started by addition of 20 $\mu$l substrate-buffer per well (e.g. luciferase assay reagent, Promega). Measurement of luminescence is started immediately (e.g. Berthold luminometer or Hamamatzu camera system).

### 3) $G_q$-coupled receptors

Cells (such as CHO cells or primary cells) are stably transfected with the relevant receptor. Cells expressing functional receptor protein are grown in 50% Dulbecco's modified Eagle medium / 50% F12 (DMEM/F12) supplemented with 10% FBS, at 37°C in a humidified atmosphere with 5% $CO_2$ and are routinely split at a cell line dependent ratio every 3 or 4 days. Test cultures are seeded into 384 - well plates at an appropriate density (e.g. 2000 cells / well in 35 $\mu$l cell culture medium) in DMEM/F12 with FBS, and are grown for 48 hours (range: ~ 24 - 60 hours, depending on cell line). Growth medium is then exchanged against physiological salt solution (e.g. Tyrode solution). Test compounds dissolved in DMSO are diluted in Tyrode solution containing 0.1% BSA and transferred to the test cultures (maximal final concentration 10 $\mu$molar). After addition of the receptor specific agonist the resulting Gq-mediated intracellular calcium increase is measured using appropriate read-out systems (e.g. calcium-sensitive dyes).

### b) Ion channels

Ion channels are integral membrane proteins involved in electrical signaling, transmembrane signal transduction, and electrolyte and solute transport. By forming macromolecular pores through the membrane lipid bilayer, ion channels account for the flow of specific ion species driven by the electrochemical potential gradient for the permeating ion. At the single molecule level, individual channels undergo conformational transitions ("gating") between the 'open' (ion conducting) and 'closed' (non conducting) state. Typical single channel openings last for a few milliseconds and result in elementary transmembrane currents in the range of $10^{-9}$ - $10^{-12}$ Ampere. Channel gating is controlled by various chemical and/or biophysical parameters, such as neurotransmitters and intracellular second messengers ('ligand-gated' channels) or membrane potential ('voltage-gated' channels). Ion channels are functionally characterized by their ion selectivity, gating properties, and regulation by hormones and pharmacological agents. Because of their central role in signaling and transport processes, ion channels present ideal targets for pharmacological therapeutics in various pathophysiological settings.

The "BREAST CANCER GENE" may encode an ion channel. A method of screening potential activators or inhibitors of channels activity of the "BREAST CANCER GENE" polypeptide may screen for compounds interacting with ion channels to either inhibit or promote their activity and can be based on (1.) binding and (2.) functional assays in living cells.

1. For ligand-gated channels, e.g. ionotropic neurotransmitter/hormone receptors, assays can be designed detecting binding to the target by competition between the compound and a labeled ligand.

2. Ion channel function can be tested functionally in living cells. Target proteins are either expressed endogenously in appropriate reporter cells or are introduced recombinantly. Channel activity can be monitored by (2.1) concentration changes of the permeating ion (most prominently $Ca^{2+}$ ions), (2.2) by changes in the transmembrane electrical potential gradient, and (2.3) by measuring a cellular response (e.g. expression of a reporter gene, secretion of a neurotransmitter) triggered or modulated by the target activity.

2.1 Channel activity results in transmembrane ion fluxes. Thus activation of ionic channels can be monitored by the resulting changes in intracellular ion concentrations using luminescent or fluorescent indicators. Because of its wide dynamic range and availability of suitable indicators this applies particularly to changes in intracellular $Ca^{2+}$ ion concentration ($[Ca^{2+}]_i$). $[Ca^{2+}]_i$ can be measured, for example, by aequorin luminescence or fluorescence dye technology (e.g. using Fluo-3, Indo-1, Fura-2). Cellular assays can be designed where either the $Ca^{2+}$ flux through the target channel itself is measured directly or where modulation of the target channel affects membrane potential and thereby the activity of co-expressed voltage-gated $Ca^{2+}$ channels.

2.2 Ion channel currents result in changes of electrical membrane potential ($V_m$) which can be monitored directly using potentiometric fluorescent probes. These electrically charged indicators (e.g. the anionic oxonol dye $DiBAC_4$ (3)) redistribute between extra- and intracellular compartment in response to voltage changes. The equilibrium distribution is governed by the Nernst-equation. Thus changes in membrane potential results in concomitant changes in cellular fluorescence. Again, changes in $V_m$ might be caused directly by the activity of the target ion channel or through amplification and/or prolongation of the signal by channels co-expressed in the same cell.

2.3 Target channel activity can cause cellular $Ca^{2+}$ entry either directly or through activation of additional $Ca^{2+}$ channel (see 2.1). The resulting intracellular $Ca^{2+}$ signals regulate a variety of cellular responses, e.g. secretion or gene transcription. Therefore modulation of the target channel can be detected by monitoring secretion of a known hormone/transmitter from the target-expressing cell or through expression of a reporter gene (e.g. luciferase) controlled by an $Ca^{2+}$-responsive promoter element (e.g. cyclic AMP/ $Ca^{2+}$-responsive elements; CRE).

cb) DNA-binding proteins and transcription factors

The "BREAST CANCER GENE" may encode a DNA-binding protein or a transcription factor. The activity of such a DNA-binding protein or a transcription factor may be measured, for example, by a promoter assay which measures the ability of the DNA-binding protein or the transcription factor to initiate transcription of a test sequence linked to a particular promoter. A method of screening test compounds for its ability to modulate the activity of such a DNA-binding protein or a transcription factor may measure the changes in the expression of a test gene which is regulated by a promoter which is responsive to the transcription factor.

*Promotor assays*

A promoter assay was set up with a human hepatocellular carcinoma cell HepG2 that was stably transfected with a luciferase gene under the control of a gene of interest (e.g. thyroid hormone) regulated promoter. The vector 2xIROluc, which was used for transfection, carries a thyroid hormone responsive element (TRE) of two 12 bp inverted palindromes separated by an 8 bp spacer in front of a tk minimal promoter and the luciferase gene. Test cultures were seeded in 96 well plates in serum - free Eagle's Minimal Essential Medium supplemented with glutamine, tricine, sodium pyruvate, non - essential amino acids, insulin, selen, transferrin, and were cultivated in a humidified atmosphere at 10 % $CO_2$ at 37°C. After 48 hours of incubation serial dilutions of test compounds or reference compounds (L-T3, L-T4 e.g.) and co-stimulator if appropriate (final concentration 1 nM) were added to the cell cultures and incubation was continued for the optimal time (e.g. another 4-72 hours). The cells were then lysed by addition of buffer containing Triton X100 and luciferin and the luminescence of luciferase induced by T3 or other compounds was measured in a luminometer. For each concentration of a test compound replicates of 4 were tested. $EC_{50}$ - values for each test compound were calculated by use of the Graph Pad Prism Scientific software.

*Screening Methods*

Disclosed are assays for screening test compounds which bind to or modulate the activity of a "BREAST CANCER GENE" polypeptide or a "BREAST CANCER GENE" polynucleotide. A test compound preferably binds to a "BREAST CANCER GENE" polypeptide or polynucleotide. More preferably, a test compound decreases or increases "BREAST CANCER GENE" activity by at least about 10, preferably about 50, more preferably about 75, 90, or 100% relative to the absence of the test compound.

*Test Compounds*

Test compounds can be pharmacological agents already known in the art or can be compounds previously unknown to have any pharmacological activity. The compounds can be naturally occurring or designed in the laboratory. They can be isolated from microorganisms, animals, or plants, and can be produced recombinant, or synthesized by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the one-bead one-compound library method, and synthetic library methods using affinity chromatography selection. The biological library approach is limited to polypeptide libraries, while the other four approaches are applicable to polypeptide, non-peptide oligomer, or small molecule libraries of compounds.

Methods for the synthesis of molecular libraries are well known in the art (52). Libraries of compounds can be presented in solution (53), or on beads (54), DNA-chips (55), bacteria or spores (Ladner, U.S. Patent 5,223,409), plasmids (56), or phage (57).

*High Through put Screening*

Test compounds can be screened for the ability to bind to "BREAST CANCER GENE" polypeptides or polynucleotides or to affect "BREAST CANCER GENE" activity or "BREAST CANCER GENE" expression using high throughput screening. Using high throughput screening, many discrete compounds can be tested in parallel so that large numbers of test compounds can be quickly screened. The most widely established techniques utilize 96-well, 384-well or 1536-well microtiter plates. The wells of the microtiter plates typically require assay volumes that range from 5 to 500 $\mu$l. In addition to the plates, many instruments, materials, pipettors, robotics, plate washers, and plate readers are commercially available to fit the microwell formats.

Alternatively, free format assays, or assays that have no physical barrier between samples, can be used. For example, an assay using pigment cells (melanocytes) in a simple homogeneous assay for combinatorial peptide libraries is described by (58). The cells are placed under agarose in culture dishes, then beads that carry combinatorial compounds are placed on the surface of the agarose. The combinatorial compounds are partially released the compounds from the beads. Active compounds can be visualized as dark pigment areas because, as the compounds diffuse locally into the gel matrix, the active compounds cause the cells to change colors.

Another example of a free format assay is described in (59). Chelsky placed a simple homogenous enzyme assay for carbonic anhydrase inside an agarose gel such that the enzyme in the gel would cause a color change throughout the gel. Thereafter, beads carrying combinatorial compounds via a photolinker were placed inside the gel and the compounds were partially released by UV light. Compounds that inhibited the enzyme were observed as local zones of inhibition having less color change.

In another example, combinatorial libraries were screened for compounds that had cytotoxic effects on cancer cells growing in agar (60).

Another high throughput screening method is described in Beutel et al., U.S. Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

*Binding Assays*

For binding assays, the test compound is preferably a small molecule which binds to and occupies, for example, the ATP/GTP binding site of the enzyme or the active site of a "BREAST CANCER GENE" polypeptide, such that normal biological activity is prevented. Examples of such small molecules include, but are not limited to, small peptides or peptide-like molecules.

In binding assays, either the test compound or a "BREAST CANCER GENE" polypeptide can comprise a detectable label, such as a fluorescent, radioisotopic, chemiluminescent, or enzymatic label, such as horseradish peroxidase, alkaline phosphatase, or luciferase. Detection of a test compound which is bound to a "BREAST CANCER GENE" polypeptide can then be accomplished, for example, by direct counting of radioemmission, by scintillation counting, or by determining conversion of an appropriate substrate to a detectable product.

Alternatively, binding of a test compound to a "BREAST CANCER GENE" polypeptide can be determined without labeling either of the interactants. For example, a microphysiometer can be used to detect binding of a test compound with a "BREAST CANCER GENE" polypeptide. A microphysiometer (e.g., CytosensorJ) is an analytical instrument that measures the rate at which a cell acidifies its environment using a light-addressable potentiometric sensor (LAPS). Changes in this acidification rate can be used as an indicator of the interaction between a test compound and a "BREAST CANCER GENE" polypeptide (61).

Determining the ability of a test compound to bind to a "BREAST CANCER GENE" polypeptide also can be accomplished using a technology such as real-time Bimolecular Interaction Analysis (BIA) (62). BIA is a technology for studying biospecific interactions in real time, without labeling any of the interactants (e.g., BIAcore™). Changes in the optical phenomenon surface plasmon resonance (SPR) can be used as an indication of real-time reactions between biological molecules.

A "BREAST CANCER GENE" polypeptide can be used as a "bait protein" in a two-hybrid assay or three-hybrid assay [see, e.g., U.S. Patent 5,283,317 and Brent WO 94/10300], to identify other proteins which bind to or interact with the "BREAST CANCER GENE" polypeptide and modulate its activity.

The two-hybrid system is based on the modular nature of most transcription factors, which consist of separable DNA-binding and activation domains. Briefly, the assay utilizes two different DNA constructs. For example, in one construct, polynucleotide encoding a "BREAST CANCER GENE" polypeptide can be fused to a polynucleotide encoding the DNA binding domain of a known transcription factor (e.g., GAL4). In the other construct a DNA sequence that encodes an unidentified protein ("prey" or "sample") can be fused to a polynucleotide that codes for the activation domain of the known transcription factor. If the "bait" and the "prey" proteins are able to interact in vivo to form an protein- dependent complex, the DNA-binding and activation domains of the transcription factor are brought into close proximity. This proximity allows transcription of a reporter gene (e.g., LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with the "BREAST CANCER GENE" polypeptide.

It may be desirable to immobilize either a "BREAST CANCER GENE" polypeptide (or polynucleotide) or the test compound to facilitate separation of bound from unbound forms of one or both of the interactants, as well as to accommodate automation of the assay. Thus, either a "BREAST CANCER GENE" polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach a "BREAST CANCER GENE" polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to a "BREAST CANCER GENE" polypeptide (or polynucleotide) can be accomplished in any vessel suitable for containing the reactants. Examples of such vessels include microtiter plates, test tubes, and microcentrifuge tubes.

A "BREAST CANCER GENE" polypeptide may be a fusion protein comprising a domain that allows the "BREAST CANCER GENE" polypeptide to be bound to a solid support. For example, glutathione S-transferase fusion proteins can be adsorbed onto glutathione sepharose beads (Sigma Chemical, St. Louis, Mo.) or glutathione derivatized microtiter plates, which are then combined with the test compound or the test compound and the nonadsorbed "BREAST CANCER GENE" polypeptide; the mixture is then incubated under conditions conducive to complex formation (e.g., at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays. For example, either a "BREAST CANCER GENE" polypeptide (or polynucleotide) or a test compound can be immobilized utilizing conjugation of biotin and streptavidin. Biotinylated "BREAST CANCER GENE" polypeptides (or polynucleotides) or test compounds can be prepared from biotin NHS (N-hydroxysuccinimide) using techniques well known in the art (e.g., biotinylation kit, Pierce Chemicals, Rockford, Ill.) and immobilized in the wells of streptavidin-coated 96 well plates (Pierce Chemical). Alternatively, antibodies which specifically bind to a "BREAST CANCER GENE" polypeptide, polynucleotide, or a test compound, but which do not interfere with a desired binding site, such as the ATP/GTP binding site or the active site of the "BREAST CANCER GENE" polypeptide, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to a "BREAST CANCER GENE" polypeptide or test compound, enzyme-linked assays which rely on detecting an activity of a "BREAST CANCER GENE" polypeptide, and SDS gel electrophoresis under non-reducing conditions.

Screening for test compounds which bind to a "BREAST CANCER GENE" polypeptide or polynucleotide also can be carried out in an intact cell. Any cell which comprises a "BREAST CANCER GENE" polypeptide or polynucleotide can be used in a cell-based assay system. A "BREAST CANCER GENE" polynucleotide can be naturally occurring in the cell or can be introduced using techniques such as those described above. Binding of the test compound to a "BREAST CANCER GENE" polypeptide or polynucleotide is determined as described above.

_Modulation of Gene Expression_

Test compounds which increase or decrease "BREAST CANCER GENE" expression may be identified. A "BREAST CANCER GENE" polynucleotide is contacted with a test compound in an appropriate expression test system as described below or in a cell system, and the expression of an RNA or polypeptide product of the "BREAST CANCER GENE" polynucleotide is determined. The level of expression of appropriate mRNA or polypeptide in the presence of the test compound is compared to the level of expression of mRNA or polypeptide in the absence of the test compound. The test compound can then be identified as a modulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of "BREAST CANCER GENE" mRNA or polypeptide expression in the cells can be determined by methods well known in the art for detecting mRNA or polypeptide. Either qualitative or quantitative methods can be used. The presence of polypeptide products of a "BREAST CANCER GENE" polynucleotide can be determined, for example, using a variety of techniques known in the art, including immunochemical methods such as radioimmunoassay, Western blotting, and immunohistochemistry. Alternatively, polypeptide synthesis can be determined in vivo, in a cell culture, or in an in vitro translation system by detecting incorporation of labeled amino acids into a "BREAST CANCER GENE" polypeptide.

Such screening can be carried out either in a cell-free assay system or in an intact cell. Any cell which expresses a "BREAST CANCER GENE" polynucleotide can be used in a cell-based assay system. A "BREAST CANCER GENE" polynucleotide can be naturally occurring in the cell or can be introduced using techniques such as those described above. Either a. primary culture or an established cell line, such as CHO or human embryonic kidney 293 cells, can be used.

*Therapeutic Indications and Methods*

Therapies for treatment of breast cancer primarily relied upon effective chemotherapeutic drugs for intervention on the cell proliferation, cell growth or angiogenesis. The advent of genomics-driven molecular target identification has opened up the possibility of identifying new breast cancer-specific targets for therapeutic intervention that will provide safer, more effective treatments for malignant neoplasia patients and breast cancer patients in particular. Thus, newly discovered breast cancer-associated genes and their products can be used as tools to develop innovative therapies. The identification of the Her2/neu receptor kinase presents exciting new opportunities for treatment of a certain subset of tumor patients as described before. Genes playing important roles in any of the physiological processes outlined above can be characterized as breast cancer targets. Genes or gene fragments identified through genomics can readily be expressed in one or more heterologous expression systems to produce functional recombinant proteins. These proteins are characterized in vitro for their biochemical properties and then used as tools in high-throughput molecular screening programs to identify chemical modulators of their biochemical activities. Modulators of target gene expression or protein activity can be identified in this manner and subsequently tested in cellular and in vivo disease models for therapeutic activity. Optimization of lead compounds with iterative testing in biological models and detailed pharmacokinetic and toxicological analyses form the basis for drug development and subsequent testing in humans.

A reagent which affects human "BREAST CANCER GENE" activity can be administered to a human cell, either in vitro or in vivo, to reduce or increase human "BREAST CANCER GENE" activity. The reagent preferably binds to an expression product of a human "BREAST CANCER GENE". If the expression product is a protein, the reagent is preferably an antibody. For treatment of human cells ex vivo, an antibody can be added to a preparation of stem cells which have been removed from the body. The cells can then be replaced in the same or another human body, with or without clonal propagation, as is known in the art.

The reagent may be delivered using a liposome. Preferably, the liposome is stable in the animal into which it has been administered for at least about 30 minutes, more preferably for at least about 1 hour, and even more preferably for at least about 24 hours. A liposome comprises a lipid composition that is capable of targeting a reagent, particularly a polynucleotide, to a particular site in an animal, such as a human. Preferably, the lipid composition of the liposome is capable of targeting to a specific organ of an animal, such as the lung, liver, spleen, heart brain, lymph nodes, and skin.

A liposome may comprise a lipid composition that is capable of fusing with the plasma membrane of the targeted cell to deliver its contents to the cell. Preferably, the transfection efficiency of a liposome is about 0.5 $\mu$g of DNA per 16 nmol of liposome delivered to about $10^6$ cells, more preferably about 1.0 $\mu$g of DNA per 16 nmol of liposome delivered to about $10^6$ cells, and even more preferably about 2.0 $\mu$g of DNA per 16 nmol of liposome delivered to about $10^6$ cells. Preferably, a liposome is between about 100 and 500 nm, more preferably between about 150 and 450 nm, and even more preferably between about 200 and 400 nm in diameter.

Suitable liposomes include those liposomes usually used in, for example, gene delivery methods known to those of skill in the art. More preferred liposomes include liposomes having a polycationic lipid composition and/or liposomes having a cholesterol backbone conjugated to polyethylene glycol. Optionally, a liposome comprises a compound capable of targeting the liposome to a particular cell type, such as a cell-specific ligand exposed on the outer surface of the liposome.

Complexing a liposome with a reagent such as an antisense oligonucleotide or ribozyme can be achieved using methods which are standard in the art (see, for example, U.S. Patent 5,705,151). Preferably, from about 0.1 $\mu$g to about 10 $\mu$g of polynucleotide is combined with about 8 nmol of liposomes, more preferably from about 0.5 $\mu$g to about 5 $\mu$g of polynucleotides are combined with about 8 nmol liposomes, and even more preferably about 1.0 $\mu$g of polynucleotides is combined with about 8 nmol liposomes.

Antibodies may be delivered to specific tissues in vivo using receptor-mediated targeted delivery. Receptor-mediated DNA delivery techniques are taught in, for example (63).

*Determination of a Therapeutically Effective Dose*

The determination of a therapeutically effective dose is well within the capability of those skilled in the art. A therapeutically effective dose refers to that amount of active ingredient which increases or decreases human "BREAST CANCER GENE" activity relative to the human "BREAST CANCER GENE" activity which occurs in the absence of the therapeutically effective dose.

For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, e.g., $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$.

Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

If the reagent is a single-chain antibody, polynucleotides encoding the antibody can be constructed and introduced into a cell either ex vivo or in vivo using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, a gene gun, and DEAE- or calcium phosphate-mediated transfection.

Effective in vivo dosages of an antibody are in the range of about 5 μg to about 50 μg/kg, about 50 μg to about 5 mg/kg, about 100 μg to about 500 μg/kg of patient body weight, and about 200 to about 250 μg/kg of patient body weight. For administration of polynucleotides encoding single-chain antibodies, effective in vivo dosages are in the range of about 100 ng to about 200 ng, 500 ng to about 50 mg, about 1 μg to about 2 mg, about 5 μg to about 500 μg, and about 20 μg to about 100 μg of DNA.

If the expression product is mRNA, the reagent is preferably an antisense oligonucleotide or a ribozyme. Polynucleotides which express antisense oligonucleotides or ribozymes can be introduced into cells by a variety of methods, as described above.

Preferably, a reagent reduces expression of a "BREAST CANCER GENE" gene or the activity of a "BREAST CANCER GENE" polypeptide by at least about 10, preferably about 50, more preferably about 75, 90, or 100% relative to the absence of the reagent. The effectiveness of the mechanism chosen to decrease the level of expression of a "BREAST CANCER GENE" gene or the activity of a "BREAST CANCER GENE" polypeptide can be assessed using methods well known in the art, such as hybridization of nucleotide probes to "BREAST CANCER GENE"-specific mRNA, quantitative RT-PCR, immunologic detection of a "BREAST CANCER GENE" polypeptide, or measurement of "BREAST CANCER GENE" activity.

Any of the therapeutic methods described above can be applied to any subject in need of such therapy, including, for example, birds and mammals such as dogs, cats, cows, pigs, sheep, goats, horses, rabbits, monkeys, and most preferably, humans.

The above disclosure generally describes the present invention. A more complete understanding can be obtained by reference to the following specific examples which are provided for purposes of illustration only and are not intended to limit the scope of the invention

*Pharmaceutical Compositions*

Disclosed are pharmaceutical compositions which can be administered to a patient to achieve a therapeutic effect. Pharmaceutical compositions may comprise, for example, a "BREAST CANCER GENE" polypeptide, "BREAST CANCER GENE" polynucleotide, ribozymes or antisense oligonucleotides, antibodies which specifically bind to a "BREAST CANCER GENE" polypeptide, or mimetics, agonists, antagonists, or inhibitors of a "BREAST CANCER GENE" polypeptide activity. The compositions can be administered alone or in combination with at least one other agent, such as stabilizing compound, which can be administered in any sterile, biocompatible pharmaceutical carrier, including, but not limited to, saline, buffered saline, dextrose, and water. The compositions can be administered to a patient alone, or in combination with other agents, drugs or hormones.

In addition to the active ingredients, these pharmaceutical compositions can contain suitable pharmaceutically acceptable carriers comprising excipients and auxiliaries which facilitate processing of the active compounds into preparations which can be used pharmaceutically. Pharmaceutical compositions can be administered by any number of routes including, but not limited to, oral, intravenous, intramuscular, intraarterial, intramedullary, intrathecal, intraventricular, transdermal, subcutaneous, intraperitoneal, intranasal, parenteral, topical, sublingual, or rectal means. Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethylcellulose, or sodium carboxymethylcellulose; gums including arabic and tragacanth; and proteins such as gelatin and collagen. If desired, disintegrating or solubilizing agents can be added, such as the cross-linked polyvinyl pyrrolidone, agar, alginic acid, or a salt thereof, such as sodium alginate.

Dragee cores can be used in conjunction with suitable coatings, such as concentrated sugar solutions, which also can contain gum arabic, talc, polyvinylpyrrolidone, carbopol gel, polyethylene glycol, and/or titanium dioxide, lacquer solutions, and suitable organic solvents or solvent mixtures. Dyestuffs or pigments can be added to the tablets or dragee coatings for product identification or to characterize the quantity of active compound, i.e., dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Pharmaceutical formulations suitable for parenteral administration can be formulated in aqueous solutions, preferably in physiologically compatible buffers such as Hanks' solution, Ringer's solution, or physiologically buffered saline. Aqueous injection suspensions can contain substances which increase the viscosity of the suspension, such as sodium carboxymethyl cellulose, sorbitol, or dextran. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers also can be used for delivery. Optionally, the suspension also can contain suitable stabilizers or agents which increase the solubility of the compounds to allow for the preparation of highly concentrated solutions. For topical or nasal administration, penetrants appropriate to the particular barrier to be permeated are used in the formulation. Such penetrants are generally known in the art.

The pharmaceutical compositions can be manufactured in a manner that is known in the art, e.g., by means of conventional mixing, dissolving, granulating, dragee making, levigating, emulsifying, encapsulating, entrapping, or lyophilizing processes. The pharmaceutical composition can be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation can be a lyophilized powder which can contain any or all of the following: 150 mM histidine, 0.1%2% sucrose, and 27% mannitol, at a pH range of 4.5 to 5.5, that is combined with buffer prior to use.

Further details on techniques for formulation and administration can be found in the latest edition of REMINGTON'S PHARMACEUTICAL SCIENCES (64). After pharmaceutical compositions have been prepared, they can be placed in an appropriate container and labeled for treatment of an indicated condition. Such labeling would include amount, frequency, and method of administration.

One strategy for identifying genes that are involved in breast cancer is to detect genes that are expressed differentially under conditions associated with the disease versus non-disease or in the context of therapy response conditions. The sub-sections below describe a number of experimental systems which can be used to detect such differentially expressed genes. In general, these experimental systems include at least one experimental condition in which subjects or samples are treated in a manner associated with breast cancer, in addition to at least one experimental control condition lacking such disease associated treatment or does not respond to such treatment. Differentially expressed genes are detected, as described below, by comparing the pattern of gene expression between the experimental and control conditions.

Once a particular gene has been identified through the use of one such experiment, its expression pattern may be further characterized by studying its expression in a different experiment and the findings may be validated by an independent technique. Such use of multiple experiments may be useful in distinguishing the roles and relative importance of particular genes in breast cancer and the treatment thereof. A combined approach, comparing gene expression pattern in cells derived from breast cancer patients to those of *in vitro* cell culture models can give substantial hints on the pathways involved in development and/or progression of breast cancer. It can also elucidate the role of such genes in the development of resistance or insensitivity to certain therapeutic agents (e.g. chemotherapeutic drugs).

Among the experiments which may be utilized for the identification of differentially expressed genes involved in malignant neoplasia and breast cancer in particular, are experiments designed to analyze those genes which are involved in signal transduction. Such experiments may serve to identify genes involved in the proliferation of cells.

Below are methods described for the identification of genes which are involved in breast cancer. Such represent genes which are differentially expressed in breast cancer conditions relative to their expression in normal, or non-breast cancer conditions or upon experimental manipulation based on clinical observations. Such differentially expressed genes represent "target" and/or "marker" genes. Methods for the further characterization of such differentially expressed genes, and for their identification as target and/or marker genes, are presented below.

Alternatively, a differentially expressed gene may have its expression modulated, i.e., quantitatively increased or decreased, in normal versus breast cancer states, or under control versus experimental conditions. The degree to which expression differs in normal versus breast cancer or control versus experimental states need only be large enough to be visualized via standard characterization techniques, such as, for example, the differential display technique described below. Other such standard characterization techniques by which expression differences may be visualized include but are not limited to quantitative RT-PCR and Northern analyses, which are well known to those of skill in the art.

In Addition to the experiments described above the following describes algorithms and statistical analyses which can be utilized for data evaluation and for the classification as well as response prediction for a so far not classified biological sample in the context of control samples. Predictive algorithms and equations described below have already shown their power to subdivide individual cancers.

## EXAMPLE 1

*Withdrawal of clinical specimens and isolation of nucleic acids*

Patients with primary breast cancer with neoadjuvant PST treatment comprising the anti-cancer drug classes of anthracyclines, taxol (and derivatives thereof) and cyclophosphamide (TEC, ECT, TAC or ET) were selected for analysis. The neoadjuvant chemotherapy consisted of epirubicin 50 mg $m^2$ day 1 in a short i.v. infusion, and cyclophosphamide 500 mg $m^2$ day 1 or taxane 75 mg $m^2$ day 1 short i.v. infusion. The study requirements were that participants have an untreated primary breast cancer disease that was amenable to serial core biopsies and that was going to be treated with TEC, ECT, TAC or ET chemotherapy as the first therapeutic intervention prior to surgery. Serial core biopsies of the primary tumor were performed prior to treatment and 24 hours post the initiation of the first course of the neoadjuvant chemotherapy. The biopsies were obtained from a locally anesthetized region using Bard® MAGNUM™ Biopsy Instrument (C. R. Bard, Inc., Covington, U. S.) with Bard® Magnum biopsy needles (BIP GmbH, Tuerkenfeld, Germany). The serial biopsies were taken from a distinct area of the tumor using a 90° angle and an additional skin entry site. This approach was taken to ensure a high percentage of tumor material within the sample. All biopsy samples were snap-frozen in liquid nitrogen and stored at -80°C until further processing. Hematoxilin/eosin-stained sections from tumor specimens were examined to assess the relative amounts of tumor cells, benign epithelium, stroma, and lymphocytes. Standard clinical parameters, such as ER, PgR, ki-67, p53, cerbB2/HER2neu, have been routinely defined using bio- and/or immunohistochemical methods established in our laboratory .

Total RNA from tissue specimens was extracted by the guanidine-isothiocyanate method followed by phenol/chloroform extraction and DNase treatment using the recommended protocol for the Atlas™ Pure Total RNA Labeling System (BD Biosciences Clontech, Heidelberg, Germany). Total RNA was quantified using UV spectrophotometry (Photometer ECOM 6122, Eppendorf AG, Hamburg, Germany) and the quality and integrity was confirmed by electrophoresis of 0.5 μg isolated RNA on 1% formamide agarose gels. Additionally, RNA specimens were analyzed by microcapillary electrophoresis on LabChips using the Agilent 2100 bioanalyzer (Agilent Technologies GmbH, Boeblingen, Germany) following the manufacturer's instructions.

## EXAMPLE 2

*Expression profiling utilizing quantitative kinetic RT-PCR*

For a detailed analysis of gene expression by quantitative PCR methods, one will utilize primers flanking the genomic region of interest and a fluorescent labeled probe hybridizing in-between. Using the PRISM 7700 Sequence Detection System of PE Applied Biosystems (Perkin Elmer, Foster City, CA, USA) with the technique of a fluorogenic probe, consisting of an oligonucleotide labeled with both a fluorescent reporter dye and a quencher dye, one can perform such a expression measurement. Amplification of the probe-specific product causes-cleavage of the probe; generating an increase in reporter fluorescence. Primers and probes were selected using the Primer Express software and localized mostly in the 3' region of the coding sequence or in the 3' untranslated region. Primer design and selection of an appropriate target region is well known to those with skills in the art. Predefined primer and probes for the genes listed in Table 1 can also be obtained from suppliers e.g. PE Applied Biosystems. All primer pairs were checked for specificity by conventional PCR reactions and gel electrophoresis. To standardize the amount of sample RNA, GAPDH was selected as a reference, since it was not differentially regulated in the samples analyzed. To perform such an expression analysis of genes within a biological samples the respective primer/probes are prepared by mixing 25 $\mu$l of the 100 $\mu$M stock solution "Upper Primer", 25 $\mu$l of the 100 $\mu$M stock solution "Lower Primer" with 12,5 $\mu$l of the 100 $\mu$M stock solution TaqMan-probe (FAM/Tamra) and adjusted to 500 $\mu$l with aqua dest (Primer/probe-mix). For each reaction 1,25 $\mu$l cDNA of the patient samples were mixed with 8,75 $\mu$l nuclease-free water and added to one well of a 96 Well-Optical Reaction Plate (Applied Biosystems Part No. 4306737). 1,5 $\mu$l of the Primer/Probe-mix described above, 12,5$\mu$l Taq Man Universal-PCR-mix (2x) (Applied Biosystems Part No. 4318157) and 1 $\mu$l Water are then added. The 96 well plates are closed with 8 Caps/Strips (Applied Biosystems Part Number 4323032) and centrifuged for 3 minutes. Measurements of the PCR reaction are done according to the instructions of the manufacturer with a TaqMan 7700 from Applied Biosystems (No. 20114) under appropriate conditions (2 min. 50°C, 10 min. 95°C, 0.15mm. 95°C, 1 min. 60°C;. 40 cycles). Prior to the measurement of so far unclassified biological samples control experiments will e.g. cell lines, healthy control samples, samples of defined therapy response could be used for standardization of the experimental conditions.

TaqMan validation experiments were performed showing that the efficiencies of the target and the control amplifications are approximately equal which is a prerequisite for the relative quantification of gene expression by the comparative $\Delta\Delta$CT method, known to those with skills in the art. Wherefore the Software SDS 2.0 from Applied Biosystems can be used according to the respective instructions. CT-values are then further analyzed with appropriate software (Microsoft Excel™) of statistical software packages (SAS).

As well as the technology described above, provided by Perkin Elmer, one may use other technique implementations like Lightcycler TM from Roche Inc. or iCycler from Stratagene Inc. capable of real time detection of an RT-PCR reaction.

## EXAMPLE 3

*Expression profiling utilizing DNA microarrays*

Expression profiling can bee carried out using the Affymetrix Array Technology. By hybridization of mRNA to such a DNA-array or DNA-Chip, it is possible to identify the expression value of each transcripts due to signal intensity at certain position of the array. Usually these DNA-arrays are produced by spotting of cDNA, oligonucleotides or subcloned DNA fragments. In case of Affymetrix technology app. 400.000 individual oligonucleotide sequences were synthesized on the surface of a silicon wafer at distinct positions. The minimal length of oligomers is 12 nucleotides, preferable 25 nucleotides or full length of the questioned transcript. Expression profiling may also be carried out by hybridization to nylon or nitro-cellulose membrane bound DNA or oligonucleotides. Detection of signals derived from hybridization may be obtained by either colorimetric, fluorescent, electrochemical, electronic, optic or by radioactive readout. Detailed description of array construction have been mentioned above and in other patents cited. To determine the quantitative and qualitative changes in the gene expression of certain breast cancer specimens, RNA from tumor tissue extracted prior to any chemotherapy has to be compared among each other individually and/or to RNA extracted from benign tissue (e.g. epithelial breast tissue, or micro dissected ductal tissue) on the basis of expression profiles for the whole transcriptome. With minor modifications, the sample preparation protocol followed the Affymetrix GeneChip Expression Analysis Manual (Santa Clara, CA). Total RNA extraction and isolation from tumor or benign tissues, biopsies, cell isolates or cell containing body fluids can be performed by using TRIzol (Life Technologies, Rockville, MD) and Oligotex mRNA Midi kit (Qiagen, Hilden, Germany), and an ethanol precipitation step should be carried out to bring the concentration to 1 mg/ml. Using 5-10 mg of mRNA to create double stranded cDNA by the SuperScript system (Life Technologies). First strand cDNA synthesis was primed with a T7-(dT24) oligonucleotide. The cDNA can be extracted with phenol/chloroform and precipitated with ethanol to a final concentration of 1mg /ml. From the generated cDNA, cRNA can be synthesized using Enzo's (Enzo Diagnostics Inc., Farmingdale, NY) in vitro Transcription Kit. Within the same step the cRNA can be labeled with biotin nucleotides Bio-11-CTP and Bio-16-UTP (Enzo Diagnostics Inc., Farmingdale, NY). After labeling and cleanup (Qiagen, Hilden (Germany) the cRNA then should be fragmented in an appropriated fragmentation buffer (e.g., 40 mM Tris-Acetate, pH 8.1, 100 mM KOAc, 30 mM MgOAc, for 35 minutes at 94 °C). As per the Affymetrix protocol, fragmented cRNA should be hybridized on the HG_U133 arrays (as used herein), comprising app. 40.000 probed transcripts each, for 24 hours at 60 rpm in a 45°C hybridization oven. After Hybridization step the chip surfaces have to be washed and stained with streptavidin phycoerythrin (SAPE; Molecular Probes, Eugene, OR) in Affymetrix fluidics stations. To amplify staining, a second labeling step can be introduced, which is recommended but not compulsive. Here one should add SAPE solution twice with an antistreptavidin biotinylated antibody. Hybridization to the probe arrays may be detected by fluorometric scanning (Hewlett Packard Gene Array Scanner; Hewlett Packard Corporation, Palo Alto, CA).

After hybridization and scanning, the microarray images can be analyzed for quality control, looking for major chip defects or abnormalities in hybridization signal. Therefor either Affymetrix GeneChip MAS 5.0 Software or other microarray image analysis software can be utilized. Primary data analysis should be carried out by software provided by the manufacturer. In case of the genes analyses the primary data have been analyzed by further bioinformatic tools and additional filter criteria as described in example 4.

## EXAMPLE 4

*Data analysis from expression profiling experiments*

According to Affymetrix measurement technique (Affymetrix GeneChip Expression Analysis Manual, Santa Clara, CA) a single gene expression measurement on one chip yields the average difference value and the absolute call. Each chip contains 16-20 oligonucleotide probe pairs per gene or cDNA clone. These probe pairs include perfectly matched sets and mismatched sets, both of which are necessary for the calculation of the average difference, or expression value, a measure of the intensity difference for each probe pair, calculated by subtracting the intensity of the mismatch from the intensity of the perfect match. This takes into consideration variability in hybridization among probe pairs and other hybridization artifacts that could affect the fluorescence intensities. The average difference is a numeric value supposed to represent the expression value of that gene. The absolute call can take the values 'A' (absent), 'M' (marginal), or 'P' (present) and denotes the quality of a single hybridization. We used both the quantitative information given by the average difference and the qualitative information given by the absolute call to identify the genes which are differentially expressed in biological samples from individuals with breast cancer versus biological samples from the normal population. With other algorithms than the Affymetrix one we have obtained different numerical values representing the same expression values and expression differences upon comparison.

The differential expression E in one of the breast cancer groups compared to the normal population is calculated as follows. Given n average difference values d1, d2, ..., dn in the breast cancer population and m average difference values c1, c2, ..., cm in the population of normal individuals, it is computed by the equation:

$$E \equiv \exp\left(\frac{1}{m}\sum\nolimits_{i=1}^{m}\ln(c_i) - \frac{1}{n}\sum\nolimits_{i=1}^{n}\ln(d_i)\right) \text{ (equation 1)}$$

A gene is called up-regulated in breast cancer in tissues responding or non-responding to chemotherapy, if E >= average change factor given in Table 3 and if the number of absolute calls equal to 'P' in the breast cancer population is greater than n/2. The average fold change factors in Table 3 are given for those tumor population non responding to a given PST chemotherapy (NC), those that do respond to a given PST chemotherapy (CR) or those tissues without any pathological signs of a tumor (NB). Fold changes greater than 1 refers to an increase in gene expression in the first named tissue sample compared to the second. This regulation factors are based on the median expression values and may differ individually, here the combined profiles of all 86 genes listed in Table 1 in a cluster analysis or a principle component analysis (PCA) will indicate the classification group for such sample. By a PCA one will identify the major components (Eigengenes or Eigenvectors) which do discriminate the samples analyzed.

According to the above, a gene is called down-regulated in one tumor class versus another or normal breast tissue if E<= minimal change factor given in Table 3 and if the number of absolute calls equal to 'P' in the breast cancer population is greater than n/2. Values smaller than 1 describe an decreased expression of the given gene.

The average fold change factors given in Table 3 indicate also the relative up- and down-regulation (arrows) and indicating by (X) those gene indicative of tumor presence (NB_tissue row). The final list of differentially regulated genes consists of all up-regulated and all down-regulated genes in biological samples from individuals with breast cancer versus biological samples from the normal population or of an individual response pattern. Those genes on this list which are interesting for a diagnostic or pharmaceutical application were finally validated by quantitative real time RT-PCR (see Example 2). If a good correlation between the expression values/behavior of a transcript could be observed with both techniques, such a gene is listed in Table 1.

*Table 1:86 Genes differentially expressed and capable of predicting therapeutic success.*

| SEQUENCE | GENE SYMBOL | GENE DESCRIPTION | REF. SEQUENCES | LOCUS LINK ID | UNIGENE ID | OMIM |
|---|---|---|---|---|---|---|
| 1 | PPARBP | RB18A protein thyroid hormone receptor-associated protein complex component TRAP220 | NM_004774 | 5469 | 15589 | 604311 |
| 2 | ERBB2 | tyrosine kinase-type receptor (HER2) v-erb-b2 avian erythroblastic leukemia viral oncogene homolog 2 (neuroglioblastoma derived oncogene homolog) | NM_004448 | 2064 | 323910 | 164870 |
| 3 | DEEPEST | coiled-coil related protein DEEPEST mitotic spindle coiled-coil related protein associated with mitotic spindle apparatus coiled-coil related protein | NM_006461 | 10615 | 16244 | - |
| 4 | SIX1 | SIX1 protein sine oculis (Drosophila) homolog) | homeobox NM_005982 | 6495 | 54416 | 601205 |
| 5 | PPP1R10 | protein phosphatase 1 regulatory subunit 10 | NM_002714 | 5514 | 106019 | 603771 |
| 6 | MYBL2 | B-myb gene v-myb avian myeloblastosis viral oncogene homolog-like 2 B-myb gene; myb homologue B-myb protein | NM_002466 | 4605 | 179718 | 601415 |
| 7 | NEK2 | NIMA (never in mitosis gene a)-related kinase 2 | NM_002497 | 4751 | 153704 | 604043 |
| 8 | TOP2A | DNA topoisomerase II alpha (170kD) | NM_001067 | 7153 | 156346 | 126430 |
| 9 | MAC30 | MAC30 hypothetical protein | - | 27346 | 199695 | - |
| 10 | FXR2 | fragile X mental retardation syndrome related protein (FXR2) fragile X | NM_004860 | 9513 | 52788 | 605339 |
| 11 | TTK | HUMTTK kinase (TTK) TTK protein kinase | NM_003318 | 7272 | 169840 | 604092 |
| 12 | CD24 | CD24 antigen (small cell lung carcinoma cluster 4 antigen) accessory proteins BAP31/BAP29 | NM_013230 | 934 | 286124 | 600074 |
| 13 | PAPA-1 | PAPA-1ds ESTs | NM_031288 | 83444 | 118282 | - |

(continued)

| SEQUENCE | GENE SYMBOL | GENE DESCRIPTION | REF. SEQUENCES | LOCUS LINK ID | UNIGENE ID | OMIM |
|---|---|---|---|---|---|---|
| 14 | KNSL2 | kinesin-related protein Similar to kinesin family member C1 ATP-binding; MHC region; motor domains of a plus end-directed microtuble motor protein kinesin-related protein | NM_002263 | 3833 | 20830 | 603763 |
| 15 | CDC20 | CDC20 (cell division cycle 20 S. cerevisiae homolog) | NM_001255 | 991 | 82906 | 603618 |
| 16 | RHO7 | GTP-binding protein Rho7 | NM_005440 | 8153 | 99034 | 601555 |
| 17 | CA8 | carbonic anhydrase VIII | NM_004056 | 767 | 250502 | 114815 |
| 18 | FOXM1 | hepatocyte nuclear factor-3 fork head homolog 11A | NM_021953 | 2305 | 239 | 602341 |
| 19 | CDC2 | cell division cycle 2 G 1 to S and G2 to M | NM_001786 | 983 | 184572 | 116940 |
| 20 | HNRPL | novel heterogeneous nuclear RNP protein L protein | NM_001533 | 3191 | 2730 | 603083 |
| 21 | E2-EPF | ubiquitin carrier protein (E2-EPF) | NM_014501 | 27338 | 174070 | - |
| 22 | UBE2C | cyclin-selective ubiquitin carrier protein ubiquitin carrier protein E2-C | NM_007019 | 11065 | 93002 | 605574 |
| 23 | STK6 | serine/threonine kinase 6 | NM_003158 | 6790 | 48915 | 602687 |
| 24 | SUPT6H | putative chromatin structure regulator (SUPT6H) suppressor of Ty (S.cerevisiae) 6 | NM_003170 | 6830 | 12303 | 601333 |
| 25 | TRAF4 | MLN62 TNF receptor-associated factor 4 MLN 62 gene cystein rich domain associated to RING and TRAF protein | NM_004295 | 9618 | 8375 | 602464 |
| 26 | CCNB2 | cyclin B2 | NM_004701 | 9133 | 194698 | 602755 |
| 27 | ASPM | hypothetical protein FLJ10517 hypothetical protein FLJ10549 | NM_018123 | 259266 | 279797 | 605481 |
| 28 | RAP1GA1 | GTPase activating protein (rap1GAP) RAP1 GTPase activating protein 1 | NM_002885 | 5909 | 75151 | 600278 |
| 29 | GMPS | guanosine 5-monophosphate synthase guanine monophosphate | NM_003875 | 8833 | 5398 | 600358 |
| 30 | PHKA1 | phosphorylase kinase alpha 1 (muscle) | NM_002637 | 5255 | 2393 | 311870 |

(continued)

| SEQUENCE | GENE SYMBOL | GENE DESCRIPTION | REF. SEQUENCES | LOCUS LINK ID | UNIGENE ID | OMIM |
|---|---|---|---|---|---|---|
| 31 | CKS2 | ckshs2 CDC28 protein kinase 2 | NM_001827 | 1164 | 83758 | 116901 |
| 32 | SMARCA4 | SWISNF related matrix associated actin dependent regulator of chromatin subfamily a member 4 | NM_003072 | 6597 | 78202 | 603254 |
| 33 | HDAC2 | transcriptional regulator homolog RPD3 histone deacetylase 2 | NM_001527 | 3066 | 3352 | 605164 |
| 34 | SMARCD2 | SWISNF related matrix associated actin dependent regulator of chromatin subfamily d member 2 | NM_003077 | 6603 | 250581 | 601736 |
| 35 | SHAPY | DKFZp762K231_rl Similar to RIKEN 5830420C20 gene clone IMAGE: 3633379 ds Homo sapiens cDNA: FLJ22508 fis clone HRC11772 EST | NM_138793 | 124583 | 8859 | - |
| 36 | PRC1 | protein regulator of cytokinesis 1 | NM_003981 | 9055 | 5101 | 603484 |
| 37 | FTSJ3 | hypothetical protein FLJ20062 EST | NM_017647 | 117246 | 257486 | - |
| 38 | EPHB3 | HEK2 protein tyrosine kinase receptor EphB3 (EPHB3) | NM_004443 | 2049 | 2913 | 601839 |
| 39 | RFC4 | HUMACT1A replication factor C 37-kDa subunit replication factor C (activator 1) | NM_002916 | 5984 | 35120 | 102577 |
| 40 | TIF1 | transcriptional intermediary factor 1 | NM_003852 | 8805 | 183858 | 603406 |
| 41 | TCF33 | (HeLa) helix-loop-helix protein HE47 (E2A) transcription factor 3 (E2A immunoglobulin enhancer binding factors E12E47) | NM_003200 | 6929 | 101047 | 147141 |
| 42 | CA9 | MaTu MN p54 58N protein carbonic anhydrase IX | NM_001216 | 768 | 63287 | 603179 |
| 43 | FLOT2 | surface antigen flotillin 2 | NM_004475 | 2319 | 184488 | 131560 |
| 44 | AP2B1 | beta adaptin adaptor-related protein complex 2 beta 1 subunit beta | NM_001282 | 163 | 74626 | 601025 |
| 45 | HAN11 | FLJ22355 fis clone HRC06344 | NM_005828 | 10238 | 288283 | 605973 |

(continued)

| SEQUENCE | GENE SYMBOL | GENE DESCRIPTION | REF. SEQUENCES | LOCUS LINK ID | UNIGENE ID | OMIM |
|---|---|---|---|---|---|---|
| 46 | SMC4L1 | chromosome-associated polypeptide C EST SMC4 structural maintenance of chromosomes 4-like 1 (yeast) | NM_005496 | 10051 | 50758 | 605575 |
| 47 | PCTK1 | PCTAIRE-1serine threonine protein kinase | NM_006201 | 5127 | 171834 | 311550 |
| 48 | EMD | EDMD gene emerin (Emery-Dreifuss muscular dystrophy) | NM_000117 | 2010 | 2985 | 300384 |
| 49 | PLXNC1 | plexin C1 | NM_005761 | 10154 | 286229 | 604259 |
| 50 | SAA2 | serum amyloid A2 alpha (SAA2) | NM_000331 | 6288 | 18162 | 104750 |
| 51 | FBLN2 | fibulin-2 | NM_001998 | 2199 | 198862 | 135821 |
| 52 | T1A-2 | lung type-I cell membrane-associated glycoprotein | NM_006474 | 10630 | 135150 | - |
| 53 | RECK | ST15 reversion-inducing-cysteine-rich protein with kazal motifs suppression of tumorigenicity 15 (reversion-inducing-cysteine-rich protein with kazal motifs) | NM_021111 | 8434 | 29640 | 605227 |
| 54 | PCDH7 | PCDH7 (BH-Pcdh)a BH-protocadherin (brain-heart) | NM_002589 | 5099 | 34073 | 602988 |
| 55 | CAV1 | caveolin 1 caveolae protein 22kD | NM_001753 | 857 | 74034 | 601047 |
| 56 | CYP2A6 | HUMCYIIA4A cytochrome P450IIA4 (CYP2A4) cytochrome P450 subfamily IIA | NM_000762 | 1549 | 183584 | 122720 |
| 57 | FCER1A | high affinity IgE receptor alpha-subunit (FcERI) Fc fragment of IgE high affinity I receptor for alpha polypeptide clone | NM_002001 | 2205 | 897 | 147140 |
| 58 | PDGFRL | PDGF receptor beta-like tumor suppressor (PRLTS) PDGF receptor beta-like tumor suppressor; | NM_006207 | 5157 | 170040 | 604584 |
| 59 | IGF1 | IGF-I insulin-like growth factor I insulin-like growth factor 1 (somatomedin C) | NM_000618 | 3479 | 85112 | 147440 |
| 60 | MAOA | monoamine oxidase A (MAOA) | NM_000240 | 4128 | 183109 | 309850 |

(continued)

| SEQUENCE | GENE SYMBOL | GENE DESCRIPTION | REF. SEQUENCES | LOCUS LINK ID | UNIGENE ID | OMIM |
|---|---|---|---|---|---|---|
| 61 | HPGD | hydroxyprostaglandin dehydrogenase (PDGH) d hydroxyprostaglandin dehydrogenase 15-(NAD) NAD+-dependent 15-hydroxyprostaglandin dehydrogenase | NM_000860 | 3248 | 77348 | 601688 |
| 62 | FBLN1 | fibulin-1D gene for Fibulin 1 | TNM_001996 | 2192 | 79732 | 135820 |
| 63 | OMD | osteomodulinosteomodulin | NM_005014 | 4958 | 94070 | - |
| 64 | CTGF | connective tissue growth factor | NM_001901 | 1490 | 75511 | 121009 |
| 65 | CSPG2 | proteoglycan RG-M(V3)d chondroitin sulfate proteoglycan 2 (versican) | NM_004385 | 1462 | 81800 | 118661 |
| 66 | CX3CR1 | G protein-coupled receptor V28 protein-coupled receptor V28 d chemokine (C-X3-C) receptor 1 | GNM_001337 | 1524 | 78913 | 601470 |
| 67 | CHN1 | n-chimaerin 1 | NM_001822 | 1123 | 169965 | 118423 |
| 68 | NPD009 | 29b8 NPD009 d NPD009 protein EST | NM_020686 | 57416 | 283675 | - |
| 69 | LMOD1 | a 64 Kd autoaritigen expressed in thyroid and extra-ocular muscle leiomodin 1 (smooth muscle) 64 Kd | NM_012134 | 25802 | 79386 | 602715 |
| 70 | SPON1 | spondin 1 (f-spondin) extracellular matrix protein KIAA0762 protein | NM_006108 | 10418 | 5378 | 604989 |
| 71 | C1S | complement component C1r complement subcomponent C1s alpha- and beta-chainsd complement component 1 s subcomponent | NM_001734 | 716 | 169756 | 120580 |
| 72 | EFEMP1 | extracellular protein (S1-5) EGF-containing fibulin-like extracellular matrix protein 1 | NM_004105 | 2202 | 76224 | 601548 |
| 73 | FOXO1A | forkhead protein (FKHR) forkhead box O1A (rhabdomyosarcoma) | NM_002015 | 2308 | 170133 | 136533 |
| 74 | DCN | chondroitin dermatan sulfate proteoglycan (PG40) core protein decorinds alternatively splice decorin matrix protein; | NM_001920 | 1634 | 76152 | 125255 |

(continued)

| SEQUENCE | GENE SYMBOL | GENE DESCRIPTION | REF. SEQUENCES | LOCUS LINK ID | UNIGENE ID | OMIM |
|---|---|---|---|---|---|---|
| 75 | GAS1 | HUMGAS1A gas1 gene growth arrest-specific 1 | NM_002048 | 2619 | 65029 | 139185 |
| 76 | SDF1 | intercrine-alpha (hIRH) stromal cell-derived factor 1 | NM_000609 | 6387 | 237356 | 600835 |
| 77 | CES1 | carboxylesterase acyl coenzyme A: | NM_001266 | 1066 | 76688 | 114835 |
| 78 | FHL1 | LIM protein SLIMMER four and a half LIM domains 1 skeletal and cardiac muscle | NM_001449 | 2273 | 239069 | 300163 |
| 79 | FBN1 | fibrillin 1 (Marfan syndrome) | NM_000138 | 2200 | 750 | 139797 |
| 80 | ASS | argininosuccinate synthetase | NM_000050 | 445 | 160786 | 603470 |
| 81 | RGS2 | helix-loop-helix basic phosphoprotein (G0S8) regulator of G-protein signalling 2 24kD basic protein; basic-helix-loop-helix protein; | NM_002923 | 5997 | 78944 | 600861 |
| 82 | AKR1C3 | KIAA0119 gene aldo-keto reductase family 1 member C3 (3-alpha hydroxysteroid dehydrogenase type II) | NM_003739 | 8644 | 78183 | 603966 |
| 83 | CPA3 | mast cell carboxypeptidase A CPA) gene mast cell carboxypeptidase A3 precursor | (MC-NM_ 001870 | 1359 | 646 | 114851 |
| 84 | LILRB2 | monocyte macrophage lg-related receptor MIR-10 (MIR cl-10) leukocyte immunoglobulin-like receptor subfamily B | NM_005874 | 10288 | 22405 | 604815 |
| 85 | CYP2B7 | cytochrome P450-IIB (hIIB3) ds (phenobarbital-inducible), | NR_001278 | 1556 | 330780 | - |
| 86 | CYP2B6 | HUMCYP2BB cytochrome P450-IIB (hIIB1) cytochrome P450 subfamily IIB (phenobarbital-inducible | NM_000767 | 1555 | 1360 | 605059 |

*Table 2: Putative function of Proteins encoded by 86 genes differentially expressed and capable of predicting therapeutic success.*

| SEQU ENCE | Gene_ Symbol | putative protein function | cellular localization |
|---|---|---|---|
| 1 | PPARBP | Protein with similarity to nuclear receptor-interacting proteins; binds and coactivates the nuclear receptors PPARalpha (PPARA), RARalpha (RARA), RXR, TRbetal, and VDR | unknown |
| 2 | ERBB2 | Tyrosine kinase receptor that has similarity to the EGF receptor, a critical component of IL-6 signaling through the MAP kinase pathway, overexpression may be associated with prostate, ovary and breast cancer | membrane |
| 3 | DEEPEST | Protein of unknown function | unknown |
| 4 | SIX1 | Member of the homeodomain family of DNA binding proteins that regulate gene expression and participate in control of cell differentiation, implicated in control of muscle development | intracellular |
| 5 | PPP1R10 | Widely expressed protein with glycine-rich domain, has strong similarity to rat Rn.6755, which is a putative regulatory subunit of protein phosphatase 1 | intracellular |
| 6 | MYBL2 | Transcription factor of the oncoprotein myb family, may have a role in cell cycle progression | intracellular |
| 7 | NEK2 | mitosis(experimental evidence) control of mitosis (experimental evidence) cell cycle control (experimental evidence) | intracellular |
| 8 | TOP2A | DNA topoisomerase (predicted/computed) DNA topoisomerase (ATP-hydrolyzing)(experimental evidence) DNA binding (experimental evidence) | intracellular |
| 9 | MAC30 | Protein of unknown function | membrane |

(continued)

| SEQU ENCE | Gene_ Symbol | putative protein function | cellular localization |
|---|---|---|---|
| 10 | FXR2 | RNA-binding protein that has similarity to FMR1, associates with the 60S ribosomal subunit and forms homomers or heteromers with FMR1 and FXR1 | intracellular |
| 11 | TTK | Dual specificity serine/ threonine and tyrosine kinase that is upregulated in tumor cells and rapidly proliferating cell lines; S. cerevisiae homolog Mpslp plays a role in the mitotic spindle assembly checkpoint pathway | intracellular |
| 12 | CD24 | humoral defense mechanism plasma membrane Differentiation Anti-pathogen response Integral membrane | membrane |
| 13 | PAPA-1 | Protein of unknown function | unknown |
| 14 | KNSL2 | Protein of unknown function | intracellular |
| 15 | CDC20 | Protein that is essential for cell division, has seven WD repeats that are probably involved in protein interactions, may function in the formation of the mitotic spindle and have a role in the mitotic spindle checkpoint | intracellular |
| 16 | RHO7 | Member of the rho subfamily of ras-related GTP binding proteins | intracellular |
| 17 | CA8 | - | intracellular |
| 18 | FOXM1 | Cell-cycle regulated HNF-3/fork head transcriptional regulator, normally expressed in embryonic mesenchymal and epithelial cells but also expressed in adult cells in response to proliferative signals or oxidative stress | intracellular |
| 19 | CDC2 | Cyclin-dependent protein kinase that interacts with B-type cyclins and regulates entry into mitosis | intracellular |

(continued)

| SEQU ENCE | Gene_ Symbol | putative protein function | cellular localization |
|---|---|---|---|
| 20 | HNRPL | Component of heterogeneous nuclear ribonucleoprotein complexes, contains two RRM (RNA recognition motif) RNA binding domains, interacts with the giant loops of lampbrush chromosomes in the newt | intracellular |
| 21 | E2-EPF | Keratinocyte ubiquitin carrier protein, which is required for ubiquitin-protein conjugation, links ubiquitin with a thiol ester linkage in a ubiquitin activating enzyme-dependent manner, may be associated with endemic pemphigus foliaceus (EPF) | intracellular |
| 22 | UBE2C | Subunit of a complex that exhibits cyclin-selective ubiquitin ligase activity, degrades cyclin A and B and plays a role in cell cycle regulation | intracellular |
| 23 | STK6 | Centrosome-associated serine/threonine kinase, causes malignant transformation when overexpressed; corresponding gene is amplified and overexpressed in tumor cell lines | intracellular |
| 24 | SUPT6H | Putative modulator of chromatin structure, has a highly acidic amino terminus, a degenerate SH2 domain, and a predicted leucine zipper, has similarity to S. cerevisiae Spt6p | intracellular |
| 25 | TRAF4 | Member of a family of proteins that interact with the cytoplasmic domain of TNF receptors, expressed in breast carcinoma epithelial cells | intracellular |
| 26 | CCNB2 | Cyclin B2, a member of the cyclin family of CDK kinase regulatory subunits | intracellular |

(continued)

| SEQU ENCE | Gene_ Symbol | putative protein function | cellular localization |
|---|---|---|---|
| 27 | ASPM | - | intracellular |
| 28 | RAP1GA1 | GTPase activating protein for rap1, a member of the ras family of GTP binding proteins | intracellular |
| 29 | GMPS | Guanine monophosphate synthestase, functions in de novo synthesis of guanine nucleotides, highly expressed in proliferating transformed cells, but expression is down-regulated by treatment with phorbol ester | intracellular |
| 30 | PHKA1 | Alpha M regulatory subunit of phosphorylase kinase, which phosphorylates and thereby activates glycogen phoshorylase, muscle isoform; mutations in the gene are associated with muscle glycogenosis, a glycogen storage disease | secreted |
| 31 | CKS2 | Protein that binds and regulates CDK-cyclin complexes; similar to S. pombe p13suc1 | unknown |
| 32 | SMARCA4 | Member of SWI/SNF related family of proteins that are implicated in regulation of transcription via their effects on chromatin structure, activates transcription in cooperation with nuclear receptors | intracellular |
| 33 | HDAC2 | Histone deacetylase, has roles in transcriptional regulation through its effect on chromatin structure | intracellular |
| 34 | SMARCD2 | transcription co-activator transcription regulation from Pol II promoter chromatin modelling nucleoplasm Pol II transcription Nuclear | secreted |
| 35 | SHAPY | - | unknown |

(continued)

| SEQU ENCE | Gene_ Symbol | putative protein function | cellular localization |
|---|---|---|---|
| 36 | PRC1 | Protein that associates with the mitotic spindle and is required for cytokinesis but not nuclear division, a probable substrate for phosphorylation by cyclin-dependent protein kinases | unknown |
| 37 | FTSJ3 | NM 017647 (analysis) hypothetical protein FLJ20062 | intracellular |
| 38 | EPHB3 | Eph-related receptor tyrosine kinase | membrane |
| 39 | RFC4 | Subunit of replication factor C (activator 1), an activator of DNA polymerases | intracellular |
| 40 | TIF1 | Mediator of the ligand-dependent activation function (AF-2) of the nuclear estrogen receptor, also functions in the ligand-dependent activation function (AF-2) of other nuclear receptors | intracellular |
| 41 | TCF3 | Helix-loop-helix transcription factor involved in the regulation of immunoglobulin gene expression; chromosomal rearrangements which alter the corresponding gene are associated with acute leukemias | intracellular |
| 42 | CA9 | Carbonic anhydrase (carbonate hydro-lyase), converts carbon dioxide to carbonic acid, may function in cellular transport and metabolic processes, associated with tumors | membrane |
| 43 | FLOT2 | Epidermal surface antigen (flotillin), plays a role in intercellular epidermal adhesion and in epidermal structure and maintenance, expressed in skin epidermal cells | intracellular |

**EP 1 904 645 B1**

(continued)

| SEQU ENCE | Gene_ Symbol | putative protein function | cellular localization |
|---|---|---|---|
| 44 | AP2B1 | Beta adaptin, a plasma membrane adaptor complex AP2 subunit, and clathrin coated vesicle component, which function in intracellular protein transport, has very strong similarity to rat Rn.37383, which interacts with clathrin and initiates coat assembly | intracellular |
| 45 | HAN11 | Protein of unknown function | intracellular |
| 46 | SMC4L1 | Chromosome-associated polypeptide-C, member of the structural maintenance of chromosomes (SMC) family and subunit of hCAP-C/hCAP-E complex, involved in the condensation of mitotic chromosomes | - |
| 47 | PCTK1 | Serine/threonine protein kinase that is related to cyclin-dependent protein kinases | intracellular |
| 48 | EMD | Emerin, a serine-rich nuclear membrane protein with similarity to secretory pathway proteins and expressed in skeletal, cardiac, and smooth muscles; reduced expression causes Emery-Dreifuss muscular dystrophy | membrane |
| 49 | PLXNC1 | cell adhesion(predicted/computed) | membrane |
| 50 | SAA2 | Member of the serum amyloid A protein family of small, differentially-expressed high density apolipoproteins produced by the liver, expressed at high levels during chronic infection, inflammation, or trauma, potentially resulting in amyloidosis | secreted |
| 51 | FBLN2 | Fibulin-2, an extracellular matrix protein with EGF-like repeats and similarity to anaphylatoxins | secreted |

50

(continued)

| SEQU ENCE | Gene_ Symbol | putative protein function | cellular localization |
|---|---|---|---|
| 52 | T1A-2 | integral plasma membrane protein Apical plasma membrane | membrane |
| 53 | RECK | Protein with epidermal growth factor-like and serine-protease inhibitor-like domains, regulates matrix metalloproteinase-9 and inhibits tumor invasion, possibly functions in linking oncogenic signals to metastasis and tumor invasion | secreted |
| 54 | PCDH7 | BH-protocadherin, a cadherin-related protein expressed predominantly in the brain and the heart | membrane |
| 55 | CAV1 | tumor suppressor : structural protein | membrane |
| 56 | CYP2A6 | Member of the cytochrome P450 CYP2A family, does not have coumarin hydroxylase activity | secreted |
| 57 | FCER1A | Alpha subunit of the high affinity IgE receptor, a member of the Ig family that is involved in triggering allergic responses | membrane |
| 58 | PDGFRL | Protein with similarity to the extracellular ligand-binding domain of platelet-derived growth factor receptor beta | secreted |
| 59 | IGF1 | Insulin-like growth factor (somatomedin C), activates cell proliferation and differentiation, regulates skeletal muscle development, induces calcineurin-mediated signaling pathways through the activation of GATA2 and NFATC1, has strong similarity to murin | - |

(continued)

| SEQU ENCE | Gene_ Symbol | putative protein function | cellular localization |
|---|---|---|---|
| 60 | MAOA | Monoamine oxidase A, an enzyme involved in the metabolism of various biogenic amine hormones, requires covalently bound FAD as a cofactor; mutation of the corresponding gene causes slight mental retardation and increases in impulsive aggression | intracellular |
| 61 | HPGD | 15-hydroxyprostaglandin dehydrogenase (NAD+-dependent 15-hydroxyprostaglandin dehydrogenase), inactivates many prostaglandins by oxidation of their C-15 residues | secreted |
| 62 | FBLN1 | Fibulin, secreted glycoprotein that has EGF-like repeats and similarity to the complement component anaphylatoxins C3a, C4a, and C5a, incorporated into the extracellular matrix and also found in the plasma | secreted |
| 63 | OMD | Protein containing a leucine rich repeat N-terminal cysteine rich domain and leucine rich repeats, has moderate similarity to keratocan (murine Kera), which is a keratan sulfate proteoglycan that has a role in development of corneal transparency | secreted |
| 64 | CTGF | Connective tissue growth factor, binds IGF and may have a role in regulating normal and neoplastic cell growth | secreted |

(continued)

| SEQU ENCE | Gene_ Symbol | putative protein function | cellular localization |
|---|---|---|---|
| 65 | CSPG2 | Versican, a proteoglycan that contains hyaluronic acid and glycosaminoglycan-binding domains, epidermal growth factor (EGF)-like repeats, a lectin-like sequence and a complement regulatory protein-like domain, may control cell growth and differentiation | secreted |
| 66 | CX3CR1 | CX3C chemokine receptor, a G protein-coupled receptor that binds the CX3C chemokine fractalkine and signals through a pertussis toxin sensitive G-protein, mediates leukocyte migration and adhesion | membrane |
| 67 | CHN1 | Alpha 1 chimerin (chimaerin), a GTPase activating protein for rac (a member of the ras family of GTP binding proteins), has divergent SH2 domain at N-terminus but shares C-terminal GTPase activating domain of alpha 1 chimerin | intracellular |
| 68 | NPD009 | Proteasome | unknown |
| 69 | LMOD1 | Protein that is expressed in the thyroid and extraocular muscle, an autoantigen in several disorders including Hashimoto's thyroiditis, Graves' disease, and idiopathic myxedema | intracellular |
| 70 | SPON1 | Protein with very strong similarity to rat Rn.7546 (F-spondin), which is a member of the thrombospondin family of cell adhesion molecules that may have a role in the growth and guidance of axons | secreted |

(continued)

| SEQU ENCE | Gene_ Symbol | putative protein function | cellular localization |
|---|---|---|---|
| 71 | C1S | Complement component C1s, a serine protease with a cysteine-rich segment; deficiency is associated with a lupus erythematosus-like syndrome | secreted |
| 72 | EFEMP1 | EGF-containing fibulin-like extracellular matrix protein, associated with the development of Malattia Leventinese and Doyne honeycomb retinal dystrophy | secreted |
| 73 | FOXO1A | Member of the HNF-3/fork head family of transcriptional regulators; fusion of the activation domain to the PAX3 DNA-binding domain is associated with solid tumor alveolar rhabdomyosarcoma | intracellular |
| 74 | DCN | Decorin, a small proteoglycan that binds to collagen fibrils growth factors; member of the specialized collagens and small leucine-rich proteoglycan family (SLRP_family); deficiency is associated with a lethal form of Marfan syndrome | secreted |
| 75 | GAS1 | Protein that when overexpressed is able to block cell proliferation in carcinoma cell lines, has strong similarity to murine Gas1, which is a component of a negative circuit that controls growth suppression | secreted |
| 76 | SDF1 | Stromal cell-derived factor 1, a lymphocyte chemoattractant that signals through the receptor CXCR4, inhibits infection by lymphocyte-tropic HIV-1 strains | secreted |

(continued)

| SEQU ENCE | Gene_ Symbol | putative protein function | cellular localization |
|---|---|---|---|
| 77 | CES1 | Monocyte/macrophage serine esterase, hydrolyzes aromatic and aliphatic esters, expressed only in the monocyte/macrophage lineage and may have a role in detoxification in the lung | secreted |
| 78 | FHL1 | Protein with four LIM domains and an additional zinc finger, expressed in skeletal muscle and heart | intracellular |
| 79 | FBN1 | Fibrillin-1, a structural protein of connective tissue microfibrils; mutation of the corresponding gene causes Ectopia lentis, Marfan syndrome and Shprintzen-Goldberg syndrome | secreted |
| 80 | ASS | Argininosuccinate synthetase, catalyzes the ATP-dependent ligation of citrulline to aspartate to form argininosuccinate in the urea cycle; mutations in the corresponding gene cause citrullinemia | unknown |
| 81 | RGS2 | Regulator of G-protein signaling 2, one of a family of proteins that negatively regulates G protein-coupled receptor signaling by binding to G-protein alpha subunits and stimulating their intrinsic GTPase activity, has a basic helix-loop-helix motif | intracellular |
| 82 | AKR1C3 | 3 alpha-hydroxysteroid dehydrogenase, oxidizes xenobiotic alicyclic alcohols and 3alpha- or 17beta-hydroxy-5beta-androstanes, activated on exposure to all-trans-retinoic acid, may function in control of cell growth and differentiation | intracellular |
| 83 | CPA3 | Protein of unknown function | secreted |

(continued)

| SEQU ENCE | Gene_ Symbol | putative protein function | cellular localization |
|---|---|---|---|
| 84 | LILRB2 | Member of the immunoglobulin superfamily that contains immunoreceptor tyrosine-based inhibitory motifs (ITIMs), plays a role in regulation of immune responses and is expressed in lymphoid and myeloid cells | membrane |
| 85 | CYP2B7 | Member of the cytochrome P450 IIB subfamily, de-ethylates 7-ethoxy-coumarin | secreted |
| 86 | CYP2B6 | Member of the cytochrome P450 IIB subfamily, de-ethylates 7-ethoxy-coumarin | secreted |

*Data Filtering:*

Raw data were acquired using Microsuite 5.0 software of Affymetrix and normalized following a standard practice of scaling the average of all gene signal intensities to a common arbitrary value. 59 Genes corresponding to Affymetrix controls (housekeeping genes, etc.) were removed from the analysis. The only exception has been done for the genes for GAPDH and Beta-actin, which expression levels were used for the normalization purposes. One hundred genes, which expression levels are routinely used in order to normalized between HG-U133A and HG-U133B GeneChips, were also removed from the analysis. Genes with potentially high levels of noise (81 probe sets), which is observed for genes with low absolute expression values (genes, which expression levels did not achieve 30 RLU (TGT=100) through all experiments), were removed from the data set. The remaining genes were preprocessed to eliminate the genes (3196 probe sets) whose signal intensities were not significantly different from their background levels and thus labeled as "Absent" by Affymetrix MicroSuite 5.0 in all experiments. We eliminated genes that were not present in at least 10% of samples (3841 probe sets). Data for remaining 15,006 probe sets were subsequently analyzed by statistical methods.

*Statistical Analysis:*

In order to optimize prediction of non responding tumor samples one may use this class from the training cohort and run multiple statistical tests, suitable for group comparison including nonparametric Wilcoxon rank sum test, two-sample independent Students' t-test, Welch test, and Wilcoxon test (see Table 4). As listed in Table 4 one can identify such genes with a differential expression in the response group (favorable outcome) vs. the non response group (non favorable outcome) and a significance level (p-value) below 0.05. Hereby we identified 86 significantly differentially regulated genes displayed in Table 1.

Additionally one may apply correction for multiple testing errors such as Benjamini-Hochberg and may apply tests for False Discovery Detection such as permutations with Bootstrap or Jack-knife algorithms.

*Table 3: Relative expression of 86 genes in breast cancers responding to PST chemotherapy compared to non responding tumors. (and as compared to normal healthy tissue)*

| | | Expression | | | Rel FC | | | NB_Tissue | Regulation |
|---|---|---|---|---|---|---|---|---|---|
| SEQUENCE | Gene Symbol | Median NB | Median NC | Median CR | NB vs NC | NB vs CR | CR vs NC | NB_Tissue | Regulation |
| 1 | PPARBP | 2.47 | 2.83 | 57.55 | 0.873 | 0.043 | 20.336 | X | ↑ |
| 2 | ERBB2 | 200.72 | 241.315 | 2170.13 | 0.832 | 0.092 | 8.992 | X | ↑ |
| 3 | DEEPEST | 25.495 | 31.465 | 169.46 | 0.810 | 0.150 | 5.386 | X | ↑ |
| 4 | SIX1 | 5.42 | 15.82 | 60.46 | 0.343 | 0.090 | 3.822 | X | ↑ |
| 5 | PPP1R10 | 3.845 | 13.155 | 48.28 | 0.292 | 0.080 | 3.670 | X | ↑ |
| 6 | MYBL2 | 9.195 | 17.32 | 62.25 | 0.531 | 0.148 | 3.594 | X | ↑ |
| 7 | NEK2 | 10.84 | 29.13 | 99.15 | 0.372 | 0.109 | 3.404 | X | ↑ |
| 8 | TOP2A | 5.435 | 29.845 | 99.91 | 0.182 | 0.054 | 3.348 | X | ↑ |
| 9 | MAC30 | 53.71 | 133.82 | 443.91 | 0.401 | 0.121 | 3.317 | X | ↑ |
| 10 | FXR2 | 40.835 | 14.63 | 47.41 | 2.791 | 0.861 | 3.241 | X | ↑ |
| 11 | TTK | 11.65 | 17.02 | 54.1 | 0.684 | 0.215 | 3.179 | X | ↑ |
| 12 | CD24 | 485.075 | 353.985 | 1087.35 | 1.370 | 0.446 | 3.072 | X | ↑ |
| 13 | PAPA-1 | 15.285 | 13.765 | 40.93 | 1.110 | 0.373 | 2.973 | X | ↑ |
| 14 | KNSL2 | 23.195 | 26.27 | 76.87 | 0.883 | 0.302 | 2.926 | X | ↑ |
| 15 | CDC20 | 16.645 | 46:375 | 131.86 | 0.143 | 0.050 | 2.843 | X | ↑ |
| 16 | RHO7 | 32.37 | 10.06 | 28.51 | 3.218 | 1.135 | 2.834 | X | ↑ |
| 17 | CA8 | 30.85 | 15.09 | 42.73 | 2.044 | 0.722 | 2.832 | X | ↑ |
| 18 | FOXM1 | 5.155 | 30.185 | 83.07 | 0.171 | 0.062 | 2.752 | X | ↑ |
| 19 | CDC2 | 17.61 | 55.72 | 150.72 | 0.316 | 0.117 | 2.705 | X | ↑ |
| 20 | HNRPL | 64.195 | 25.46 | 64.91 | 2.521 | 0.989 | 2.549 | X | ↑ |
| 21 | E2-EPF | 76.185 | 127.005 | 321.4 | 0.600 | 0.237 | 2.531 | X | ↑ |
| 22 | UBE2C | 78.225 | 166.85 | 385.78 | 0.469 | 0.203 | 2.312 | X | ↑ |
| 23 | STK6 | 16.635 | 43.18 | 99.78 | 0.385 | 0.167 | 2.311 | X | ↑ |

| | | Expression | | | Rel FC | | | NB_Tissue | Regulation |
|---|---|---|---|---|---|---|---|---|---|
| SEQUENCE | Gene Symbol | Median NB | Median NC | Median CR | NB vs NC | NB vs CR | CR vs NC | NB_Tissue | Regulation |
| 24 | SUPT6H | 82.765 | 67.135 | 154.01 | 1.233 | 0.537 | 2.294 | | ↑ |
| 25 | TRAF4 | 60.095 | 58.07 | 132.76 | 1.035 | 0.453 | 2.286 | X | ↑ |
| 26 | CCNB2 | 23.555 | 68.995 | 144.29 | 0.341 | 0.163 | 2.091 | X | ↑ |
| 27 | ASPM | 1.56 | 32.755 | 67.19 | 0.048 | 0.023 | 2.051 | X | ↑ |
| 28 | RAP1GA1 | 70.45 | 92.38 | 188.86 | 0.763 | 0.373 | 2.044 | X | ↑ |
| 29 | GMPS | 80.96 | 64.11 | 127.89 | 1.263 | 0.633 | 1.995 | | ↑ |
| 30 | PHKA1 | 36.115 | 39.12 | 72.38 | 0.923 | 0.499 | 1.850 | | ↑ |
| 31 | CKS2 | 52.375 | 107.37 | 194.36 | 0.488 | 0.269 | 1.810 | X | ↑ |
| 32 | SMARCA4 | 70.87 | 89.225 | 160.95 | 0.794 | 0.440 | 1.804 | X | ↑ |
| 33 | HDAC2 | 184.8 | 115.07 | 207.09 | 1.606 | 0.892 | 1.800 | | ↑ |
| 34 | SMARCD2 | 143.995 | 105.82 | 185.03 | 1.361 | 0.778 | 1.749 | | ↑ |
| 35 | SHAPY | 85.355 | 194.925 | 336.02 | 0.438 | 0.254 | 1.724 | X | ↑ |
| 36 | PRC1 | 33.84 | 79.315 | 134.75 | 0.427 | 0.251 | 1.699 | X | ↑ |
| 37 | FTSJ3 | 146.42 | 120.57 | 193.49 | 1.214 | 0.757 | 1.605 | | ↑ |
| 38 | EPHB3 | 110.27 | 82.885 | 132.7 | 1.330 | 0.831 | 1.601 | | ↑ |
| 39 | RFC4 | 86.5 | 124.905 | 196.86 | 0.693 | 0.439 | 1.576 | X | ↑ |
| 40 | TIF1 | 59.67 | 52.605 | 82.64 | 1.134 | 0.722 | 1.571 | | ↑ |
| 41 | TCF3 | 27.5 | 27.345 | 42.81 | 1.006 | 0.642 | 1.566 | | ↑ |
| 42 | CA9 | 36.635 | 24.375 | 37.97 | 1.503 | 0.965 | 1.558 | | ↑ |
| 43 | FLOT2 | 238.61 | 182.915 | 275.34 | 1.304 | 0.867 | 1.505 | | ↑ |
| 44 | AP2B1 | 121.445 | 150.37 | 224.71 | 0.808 | 0.540 | 1.494 | | ↑ |
| 45 | HAN11 | 47.26 | 91.99 | 135.84 | 0.514 | 0.348 | 1.477 | X | ↑ |
| 46 | SMC4L1 | 105.01 | 155.125 | 222.03 | 0.677 | 0.473 | 1.431 | X | ↑ |
| 47 | PCTK1 | 77.52 | 103.48 | 136.26 | 0.749 | 0.569 | 1.317 | | ↑ |

EP 1 904 645 B1

| SEQUENCE | Gene Symbol | Expression | | | Rel FC | | | NB_Tissue | Regulation |
|---|---|---|---|---|---|---|---|---|---|
| | | Median NB | Median NC | Median CR | NB vs NC | NB vs CR | CR vs NC | NB_Tissue | Regulation |
| 48 | EMD | 87.905 | 66.505 | 80.71 | 1.322 | 1.089 | 1.214 | | ↑ |
| 49 | PLXNC1 | 2.695 | 42.185 | 22.06 | 0.064 | 0.122 | 0.523 | X | ↓ |
| 50 | SAA2 | 2294.93 | 74.045 | 36.6 | 30.994 | 62.703 | 0.494 | X | ↓ |
| 51 | FBLN2 | 210.76 | 126.09 | 58.62 | 1.672 | 3.595 | 0.465 | X | ↓ |
| 52 | T1A-2 | 41.18 | 60.07 | 27.85 | 0.686 | 1.479 | 0.464 | | ↓ |
| 53 | RECK | 99.48 | 55.135 | 25.26 | 1.804 | 3.938 | 0.458 | X | ↓ |
| 54 | PCDH7 | 16.355 | 41.795 | 18.97 | 0.391 | 0.862 | 0.454 | X | ↓ |
| 55 | CAV1 | 616.615 | 164.525 | 74.4 | 3.748 | 8.288 | 0.452 | X | ↓ |
| 56 | CYP2A6 | 35.58 | 47.7 | 20.99 | 0.746 | 1.695 | 0.440 | | ↓ |
| 57 | FCER1A | 35.6 | 30.37 | 13.12 | 1.172 | 2.713 | 0.432 | X | ↓ |
| 58 | PDGFRL | 183.765 | 169.22 | 71.17 | 1.086 | 2.582 | 0.421 | X | ↓ |
| 59 | IGF1 | 314.015 | 129.165 | 53.76 | 2.431 | 5.841 | 0.416 | X | ↓ |
| 60 | MAOA | 78.535 | 24.5 | 9.85 | 3.206 | 7.973 | 0.402 | X | ↓ |
| 61 | HPGD | 26.305 | 36.025 | 14.06 | 0.730 | 1.871 | 0.390 | | ↓ |
| 62 | FBLN1 | 278.595 | 320.875 | 125.05 | 0.868 | 2.228 | 0.390 | X | ↓ |
| 63 | OMD | 39.955 | 51.735 | 19.82 | 0.772 | 2.016 | 0.383 | X | ↓ |
| 64 | CTGF | 665.065 | 635.255 | 240.97 | 1.047 | 2.760 | 0.379 | X | ↓ |
| 65 | CSPG2 | 85.655 | 537.605 | 198.81 | 0.159 | 0.431 | 0.370 | X | ↓ |
| 66 | CX3CR1 | 100.3 | 110.235 | 39.72 | 0.910 | 2.525 | 0.360 | X | ↓ |
| 67 | CHN1 | 79.82 | 100.87 | 35.83 | 0.791 | 2.228 | 0.355 | X | ↓ |
| 68 | NPD009 | 33.58 | 59.04 | 20.48 | 0.569 | 1.640 | 0.347 | | ↓ |
| 69 | LMOD1 | 239.6 | 51.22 | 17.46 | 4.678 | 13.723 | 0.341 | X | ↓ |
| 70 | SPON1 | 43.15 | 48.27 | 16.33 | 0.894 | 2.642 | 0.338 | X | ↓ |
| 71 | C1S | 1210.75 | 1397.74 | 466.93 | 0.866 | 2.593 | 0.334 | X | ↓ |

| | | Expression | | | Rel FC | | | NB_Tissue | Regulation |
|---|---|---|---|---|---|---|---|---|---|
| SEQUENCE | Gene Symbol | Median NB | Median NC | Median CR | NB vs NC | NB vs CR | CR vs NC | NB_Tissue | Regulation |
| 72 | EFEMP1 | 1102.68 | 366.95 | 120.72 | 3.005 | 9.134 | 0.329 | X | ↓ |
| 73 | FOXOIA | 109.245 | 44.41 | 14.45 | 2.460 | 7.560 | 0.325 | X | ↓ |
| 74 | DCN | 2496.75 | 2932.74 | 944.88 | 0.851 | 2.642 | 0.322 | X | ↓ |
| 75 | GAS1 | 363.3 | 327.84 | 104.29 | 1.108 | 3.484 | 0.318 | X | ↓ |
| 76 | SDF1 | 1120.65 | 534.335 | 166.47 | 2.097 | 6.732 | 0.312 | X | ↓ |
| 77 | CES1 | 236.36 | 86.265 | 26.51 | 2.740 | 8.916 | 0.307 | X | ↓ |
| 78 | FHL1 | 2443.59 | 373.49 | 106.78 | 6.543 | 22.884 | 0.286 | X | ↓ |
| 79 | FBN1 | 106.995 | 248.345 | 66.57 | 0.431 | 1.607 | 0.268 | X | ↓ |
| 80 | ASS | 439.71 | 314.305 | 82.28 | 1.399 | 5.344 | 0.262 | X | ↓ |
| 81 | RGS2 | 600.04 | 291.23 | 70.14 | 2.060 | 8.555 | 0.241 | X | ↓ |
| 82 | AKR1C3 | 356.51 | 135.3 | 30.23 | 2.635 | 11.793 | 0.223 | X | ↓ |
| 83 | CPA3 | 88.93 | 217.38 | 47.18 | 0.409 | 1.885 | 0.217 | X | ↓ |
| 84 | LILRB2 | 22.11 | 60.51 | 12.63 | 0.365 | 1.751 | 0.209 | X | ↓ |
| 85 | CYP2B7 | 26.71 | 91.955 | 11.44 | 0.290 | 2.335 | 0.124 | X | ↓ |
| 86 | CYP2B6 | 43.925 | 191.745 | 20.62 | 0.229 | 2.130 | 0.108 | X | ↓ |

*Table 4: p-values for statistical significance for 86 genes predicting therapeutic success as calculated by comparing NC vs CR response subgroup.*

| SEQUENCE | Gene Symbol | T-Test | Welch | Wilcoxon |
|---|---|---|---|---|
| 1 | PPARBP | 0.002099 | 0.002858 | 0.001688 |
| 2 | ERBB2 | 0.067319 | 0.011030 | 0.026800 |
| 3 | DEEPEST | 0.000005 | 0.000036 | 0.000028 |
| 4 | SIX1 | 0.027920 | 0.031910 | 0.037420 |
| 5 | PPP1R10 | 0.015870 | 0.016240 | 0.015610 |
| 6 | MYBL2 | 0.022130 | 0.024170 | 0.004489 |
| 7 | NEK2 | 0.001185 | 0.001134 | 0.000546 |
| 8 | TOP2A | 0.000998 | 0.002423 | 0.001688 |
| 9 | MAC30 | 0.000221 | 0.000969 | 0.000546 |
| 10 | FXR2 | 0.029350 | 0.032710 | 0.012890 |
| 11 | TTK | 0.000055 | 0.000103 | 0.000288 |
| 12 | CD24 | 0.026980 | 0.028120 | 0.000737 |
| 13 | PAPA-1 | 0.021470 | 0.021150 | 0.012890 |
| 14 | KNSL2 | 0.000781 | 0.000749 | 0.001688 |
| 15 | CDC20 | 0.000267 | 0.000839 | 0.000206 |
| 16 | RHO7 | 0.027040 | 0.026000 | 0.026800 |
| 17 | CA8 | 0.017130 | 0.016580 | 0.012890 |
| 18 | FOXM1 | 0.000298 | 0.000356 | 0.000206 |
| 19 | CDC2 | 0.000226 | 0.000236 | 0.000288 |
| 20 | HNRPL | 0.002701 | 0.003065 | 0.005622 |
| 21 | E2-EPF | 0.001443 | 0.002702 | 0.002799 |
| 22 | UBE2C | 0.000119 | 0.000222 | 0.000206 |
| 23 | STK6 | 0.000374 | 0.000386 | 0.000981 |
| 24 | SUPT6H | 0.000652 | 0.002392 | 0.000099 |
| 25 | TRAF4 | 0.001124 | 0.001821 | 0.003560 |
| 26 | CCNB2 | 0.001363 | 0.001302 | 0.002185 |
| 27 | ASPM | 0.001261 | 0.001260 | 0.000099 |
| 28 | RAP1GA1 | 0.009601 | 0.011740 | 0.015610 |
| 29 | GMPS | 0.001262 | 0.001544 | 0.001688 |
| 30 | PHKA1 | 0.002545 | 0.002561 | 0.010570 |
| 31 | CKS2 | 0.000338 | 0.000418 | 0.000546 |
| 32 | SMARCA4 | 0.000303 | 0.001004 | 0.000018 |
| 33 | HDAC2 | 0.001521 | 0.002556 | 0.002799 |
| 34 | SMARCD2 | 0.006644 | 0.009873 | 0.005622 |
| 35 | SHAPY | 0.011660 | 0.011590 | 0.002185 |
| 36 | PRC1 | 0.001095 | 0.001252 | 0.004489 |
| 37 | FTSJ3 | 0.004229 | 0.005917 | 0.008625 |

(continued)

| SEQUENCE | Gene Symbol | T-Test | Welch | Wilcoxon |
|---|---|---|---|---|
| 38 | EPHB3 | 0.023940 | 0.024860 | 0.031740 |
| 39 | RFC4 | 0.028300 | 0.040890 | 0.059500 |
| 40 | TIF1 | 0.006209 | 0.009757 | 0.004489 |
| 41 | TCF3 | 0.027030 | 0.029700 | 0.022500 |
| 42 | CA9 | 0.025060 | 0.027230 | 0.026800 |
| 43 | FLOT2 | 0.002197 | 0.006717 | 0.000206 |
| 44 | AP2B1 | 0.014940 | 0.025410 | 0.018790 |
| 45 | HAN11 | 0.000702 | 0.001251 | 0.000737 |
| 46 | SMC4L1 | 0.021550 | 0.026800 | 0.004489 |
| 47 | PCTK1 | 0.018760 | 0.022960 | 0.031740 |
| 48 | EMD | 0.023840 | 0.024710 | 0.006985 |
| 49 | PLXNC1 | 0.013660 | 0.020700 | 0.026800 |
| 50 | SAA2 | 0.023580 | 0.022360 | 0.026800 |
| 51 | FBLN2 | 0.021350 | 0.031150 | 0.022500 |
| 52 | T1A-2 | 0.006338 | 0.010330 | 0.008625 |
| 53 | RECK | 0.022880 | 0.031990 | 0.022500 |
| 54 | PCDH7 | 0.001627 | 0.003678 | 0.001294 |
| 55 | CAV1 | 0.001712 | 0.002197 | 0.006985 |
| 56 | CYP2A6 | 0.031040 | 0.029550 | 0.043880 |
| 57 | FCER1A | 0.000007 | 0.000007 | 0.000044 |
| 58 | PDGFRL | 0.007491 | 0.010130 | 0.008625 |
| 59 | IGF1 | 0.010410 | 0.011270 | 0.003560 |
| 60 | MAOA | 0.054450 | 0.064820 | 0.037420 |
| 61 | HPGD | 0.002408 | 0.002415 | 0.001688 |
| 62 | FBLN1 | 0.019240 | 0.025660 | 0.018790 |
| 63 | OMD | 0.010110 | 0.017120 | 0.018790 |
| 64 | CTGF | 0.002058 | 0.003984 | 0.001294 |
| 65 | CSPG2 | 0.005272 | 0.006866 | 0.010570 |
| 66 | CX3CR1 | 0.002171 | 0.002135 | 0.001294 |
| 67 | CHN1 | 0.000429 | 0.001388 | 0.000099 |
| 68 | NPD009 | 0.032350 | 0.036310 | 0.037420 |
| 69 | LMOD1 | 0.005416 | 0.008793 | 0.026800 |
| 70 | SPON1 | 0.004290 | 0.007524 | 0.004489 |
| 71 | C1S | 0.010030 | 0.012300 | 0.026800 |
| 72 | EFEMP1 | 0.001778 | 0.002663 | 0.008625 |
| 73 | FOXOIA | 0.000612 | 0.001302 | 0.000737 |
| 74 | DCN | 0.002375 | 0.004071 | 0.005622 |
| 75 | GAS1 | 0.003313 | 0.004409 | 0.003560 |

(continued)

| SEQUENCE | Gene Symbol | T-Test | Welch | Wilcoxon |
|---|---|---|---|---|
| 76 | SDF1 | 0.016730 | 0.016220 | 0.022500 |
| 77 | CES1 | 0.000976 | 0.001041 | 0.001688 |
| 78 | FHL1 | 0.006180 | 0.005824 | 0.012890 |
| 79 | FBN1 | 0.006534 | 0.010060 | 0.022500 |
| 80 | ASS | 0.015770 | 0.016810 | 0.015610 |
| 81 | RGS2 | 0.001756 | 0.002738 | 0.006985 |
| 82 | AKR1C3 | 0.004953 | 0.005520 | 0.004489 |
| 83 | CPA3 | 0.006099 | 0.007827 | 0.004489 |
| 84 | LILRB2 | 0.014850 | 0.017290 | 0.022500 |
| 85 | CYP2B7 | 0.034260 | 0.038050 | 0.043880 |
| 86 | CYP2B6 | 0.026640 | 0.027220 | 0.018790 |

## EXAMPLE 5

*Statistical relevance of 86 genes differentially expressed in breast cancers responding to PST chemotherapy compared to non responding tumors.*

*Prediction of tumor classes based on expression profiles*

While as those algorithms described in Example 4 can be implemented in a certain kernel to classify samples according to their specific gene expression into two classes another approach can be taken to predict class membership by implementation of a k-NN classification. The method of k-Nearest Neighbors (k-NN), proposed by T. M. Cover and P. E. Hart, an important approach to nonparametric classification, is quite easy and efficient. Partly because of its perfect mathematical theory, NN method develops into several variations. As we know, if we have infinitely many sample points, then the density estimates converge to the actual density function. The classifier becomes the Bayesian classifier if the large-scale sample is provided. But in practice, given a small sample, the Bayesian classifier usually fails in the estimation of the Bayes error especially in a high-dimensional space, which is called the disaster of dimension. Therefore, the method of k-NN has a great pity that the sample space must be large enough.

In k-nearest-neighbor classification, the training data set is used to classify each member of a "target" data set. The structure of the data is that there is a classification (categorical) variable of interest (e.g. "responding breast tumors" (CR) or "non-responding breast tumors" (NC)), and a number of additional predictor variables (gene expression values). Generally speaking, the algorithm is as follows:

1. For each sample in the data set to be classified, locate the k nearest neighbors of the training data set. A Euclidean distance measure or a correlation analysis can be used to calculate how close each member of the training set is to the target sample that is being examined.

2. Examine the k nearest neighbors - which classification do most of them belong to?

3. Assign this category to the sample being examined.

4. Repeat this procedure steps 1 to 3 for the remaining samples in the target set.

Of course the computing time goes up as k goes up, but the advantage is that higher values of k provide smoothing that reduces vulnerability to noise in the training data. In practical applications, typically, k is in units or tens rather than in hundreds or thousands. In this disclosure we have used ak=3.

The "nearest neighbors" are determined if given the considered the vector and the distance measurement. Given a training set of expression values for a certain number of samples T = {(x1, y1), (x2, y2), ···, (xm, ym)}, to determine the class of the input vector x.

The most special case is the k-NN method, while k= 1, which just searches the one nearest neighbor:

j = argmin //x - xi// then, (x, yj) is the solution.

For estimation on the error rate of this classification the following considerations could be made:

A training set T = {(x1, y1), (x2, y2), ⋯, (xm, ym)} is called (k, d%)-stable if the error rate of k-NN method is d%, where d% is the empirical error rate from independent experiments. If the clustering of data are quite distinct (the class distance is the crucial standard of classification), then the k must be small. The key idea is we prefer the least k in the case that d% is bigger the threshold value.

The k-NN method gathers the nearest k neighbors and let them vote - the class of most neighbors wins. Theoretically, the more neighbors we consider, the smaller error rate it takes place. The general case is a little more complex. But by imagination, it is true to be the more
k the lower upper bound asymptotic to PBayes(e) if N is fixed.

One can use such algorithm to classify and cross validate a given cohort of samples based on the genes presented in Table 1. Most preferably the classification shall be performed based on the expression levels of the genes presented in Table 1 but may also combined with clinicopathological data as fare a they are measured in a continuos manner (e.g. immunohistochemistry data, scoring date such as TNM status or biochemical properties of such tumor tissue.

With k = 3 and > 100 iteration one can get classifications as depicted below for a cross-validation experiment with the two classes "responding breast tumors" (favorable response) or "non-responding breast tumors" (poor response). Affinities ranging from -1 to 1 for a given class (see Table 5a and 5b).

Table 5a

| Experiment | normal breast | poor response | favorable response | classification |
|---|---|---|---|---|
| normal mammary tissue_1 | 1 | -0.5 | -0.5 | true |
| normal mammary tissue_2 | 1 | -0.5 | -0.5 | true |
| normal mammary tissue_3 | 1 | -0.5 | -0.5 | true |
| normal mammary tissue_4 | 1 | -0.5 | -0.5 | true |
| sample_1 | -0.5 | 1 | -0.5 | true |
| sample_2 | -0.3636 | 0.8636 | -0.5 | true |
| sample_3 | 1 | -0.5 | -0.5 | false |
| sample_4 | -0.5 | 1 | -0.5 | true |
| sample_5 | -0.5 | 1 | -0.5 | true |
| sample_6 | -0.5 | 1 | -0.5 | true |
| sample_7 | -0.5 | 1 | -0.5 | true |
| sample_8 | -0.5 | 1 | -0.5 | true |
| sample_9 | -0.5 | 1 | -0.5 | true |
| sample_10 | 0.1316 | 0.3684 | -0.5 | true |
| sample_11 | -0.5 | 0.8125 | -0.3125 | false |
| sample_12 | -0.1053 | 0.6053 | -0.5 | false |
| sample_13 | -0.5 | -0.5 | 1 | true |
| sample_14 | -0.5 | -0.4423 | 0.9423 | true |
| sample_15 | -0.5 | -0.5 | 1 | true |
| sample_16 | -0.5 | 0.2222 | 0.2778 | true |
| sample_17 | -0.5 | -0.5 | 1 | true |
| sample_18 | -0.5 | -0.5 | 1 | true |
| sample_19 | -0.5 | 0.8929 | -0.3929 | true |

(continued)

| Experiment | normal breast | poor response | favorable response | classification |
|---|---|---|---|---|
| sample_20 | -0.5 | -0.5 | 1 | true |
| sample_21 | -0.5 | -0.5 | 1 | true |
| sample_22 | -0.5 | -0.5 | 1 | true |
| sample_23 | -0.5 | -0.5 | 1 | true |

Table 5b

| | True normal | True poor response | True favorable response | Correct [%] |
|---|---|---|---|---|
| Predicted normal | 544 | 47 | 0 | 92.05 |
| Predicted poor response | 1 | 246 | 40 | 85.71 |
| Predicted favorable response | 0 | 4 | 262 | 98.5 |
| Correct [%] | 99.82 | 82.83 | 86.75 | - |

The misclassification of some samples or not classifiable samples may be due to low tumor amount in specimen.

The process of model generation and cross-validation of predictive gene sets may follow the path outlined in Figure 1, wherein a given cohort of samples is subdivided into two sets a so called training and a test set. Based on such training set genes can be picked and a preliminary model can be evaluated, further such model can be validated with the sample taken from the test set cohort. These two independent classifications of samples will lead to a final model (e.g. KNN algorithm and matrix) which can be further applied to new independent tumor samples.

## EXAMPLE 6

*Analysis of differential gene expression patterns using support vector machines and class prediction*

Support vector machines (SVM) are well suited for two-class or multi-class pattern recognition (66 ; 67).

For the two-class classification problem, (e.g. tumor tissue vs. non tumor tissue, or therapy response vs. non response) assume that we have a set of samples, i.e., a series of input vectors

$$\vec{x_i} \in \mathbf{R}^d \ (i = 1, 2, ..., m)$$

with corresponding labels

$$y_i \in \{+1, -1\} \ (i = 1, 2, ..., m).$$

Here, +1 and -1 indicate the two classes. To classify gene expression patterns of marker genes from Table 1 for describing the current tumor status or probable response to a therapeutic agent, the input vector dimension is equal to the number of different oligonucleotide types present on the oligonucleotide array or a subset hereof, and each input vector unit stands for the hybridization value of one specific oligonucleotide type.

The goal is to construct a binary classifier or derive a decision function from the available samples which has a small probability of misclassifying a future sample.

An SVM implements the following idea: it maps the input vectors

$$\vec{x_i} \in \mathbf{R}^d$$

into a high-dimensional feature space

$$\Phi(\vec{x}) \in H$$

and constructs an Optimal Separating Hyperplane (OSH), which maximizes the margin, the distance between the hyperplane and the nearest data points of each class in the space *H*. By choosing OSH from among the many that can separate the positive from the negative examples in the feature space, SVMs are avoiding the risk of overfitting.

Different mappings construct different SVMs. The mapping

$$\Phi : \mathbf{R}^d \mapsto H$$

is performed by a kernel function

$$K(\vec{x_i}, \vec{x_j})$$

which defines an inner product in the space *H*.

The decision function implemented by SVM can be written as (Burges, 1998 (77):

$$f(\vec{x}) = \mathrm{sgn}\left( \sum_{i=1}^{m} y_i \alpha_i \cdot K(\vec{x}, \vec{x_i}) + b \right) \quad \text{(equation 2)}$$

where the coefficients $\alpha_i$ are obtained by solving the following convex Quadratic Programming (QP) problem:

Maximize

$$\sum_{i=1}^{m} \alpha_i - \frac{1}{2} \sum_{i=1}^{m} \sum_{j=1}^{m} \alpha_i \alpha_j \cdot y_i y_j \cdot K(\vec{x_i}, \vec{x_j})$$

subject to

$$0 \le \alpha_i \le C \quad \text{(equation 3)}$$

and

$$\sum_{i=1}^{m} \alpha_i y_i = 0$$

The regularity parameter C (equation 3) controls the trade off between margin and misclassification error. The $\mathbf{x}_j$ are called Support Vectors only if the corresponding $\alpha_j > 0$.

Two of the kernel functions used in the current example:

$$K\left(\overrightarrow{\mathbf{x}_i}, \overrightarrow{\mathbf{x}_j}\right) = \left(\overrightarrow{\mathbf{x}_i} \cdot \overrightarrow{\mathbf{x}_j} + 1\right)^d \qquad \text{(equation 4)}$$

$$K\left(\overrightarrow{\mathbf{x}_i}, \overrightarrow{\mathbf{x}_j}\right) = e^{\left(-r\|\overrightarrow{\mathbf{x}_i} - \overrightarrow{\mathbf{x}_j}\|^2\right)} \qquad \text{(equation 5)}$$

where the first one (equation 4) is called the polynomial kernel function of degree $d$ which will eventually revert to the linear function when $d = 1$, the latter (equation 5) is called the Radial Basic Function (RBF) kernel.

For a given data set, only the kernel function and the regularity parameter C must be selected to specify one SVM. An SVM has many attractive features. For instance, the solution of the QP problem is globally optimized while with neural networks the gradient based training algorithms only guarantee finding a local minima. In addition, SVM can handle large feature spaces, can effectively avoid overfitting (see above) by controlling the margin, can automatically identify a small subset made up of informative points, i.e., the Support Vectors, etc.

The classification of biological sample and thereby the identification of an neoplastic lesion as well as the response of such lesion to therapeutic agents based on gene expression data is a multi-class classification problem. The class number $k$ is equal to the number tumor subclasses (e.g. histological features, TNM stage, grade, hormonal status) and is equal to response subgroup to a certain therapeutic agent (e.g. pathologically confirmed complete remission, good remission, partial remission, or no remission, as well as progressive disease) which shall be predicted, i.e., which are present in the training data set. Due to the limited number of different classes in the present sample set, we decided to handle the multi-class classification by reducing the multi-classification to a series of binary classifications. For a $k$-class classification, $k$ SVMs are constructed. The ith SVM will be trained with all of the samples in the ith class with positive labels and all other samples with negative labels. Finally an unknown sample is classified into the class that corresponds to the SVM with the highest output value. This method is used to construct a prediction/classification system for gene expression patterns of differentially expressed marker genes as given in Table 1.

Each data point generated by a microarray hybridization experiment or by real time RT-PCR (cf. example 2 and 3) corresponds to and is determined by the number of mRNA copies present in the analyzed sample, i.e., from an experiment with $n$ oligonucleotide types on a polynucleotide array, a series of $n$ expression-level values is obtained. These $n$ values are typically stored in a metrics file which is the result of the analysis of a "cel file" by the Affymetrix® Microarray Suite or software described above. The data from a series of $m$ metrics files (representing $m$ expression analyses) are taken to build an expression matrix, in which each of the $m$ rows consists of an $n$-element expression vector for a single experiment. In order to normalize the expression values of the $m$ experiments, we define $x_{i,j}$ to be the sum of the logarithms of the expression level $a_{i,j}$ for gene $j$ (whose mRNA hybridizes with the oligonucleotide type $j$ present on the microarray, or gives a valid $\Delta\Delta C_T$ intesity), normalized so that the expression vector $\mathbf{x}_i$ has the Euclidean length 1:

$$x_{j,i} = \frac{\ln(a_{i,j})}{\sqrt{\sum_{k=1}^{n} \ln(a_{i,k})^2}} \qquad \text{(equation 6)}$$

Initial analyses are carried out using a set of 20000-element expression vectors for 27 experiments as described in example 2 to 4. Using the knowledge that the 27 experiments represent three different response classes and two different tumor states as well as the information of tumor and non-tumor tissue, we trained the SVMs described above with the training set to recognize those response classes and disease states. The test set was used to assess the prediction accuracy. Here we have preformed cross-validations utilizing the "leave one out" method and for more stringent testing a four to five fold validation (leave 25% out) with n iterations ( n> 10.000).

In such cross-validations and classification experiments the predictive power of a subset of marker genes chosen from Table 6a and b has been tested with affinity levels as follows:

Table 6a

| Experiment | normal breast & adjacent | poor response | favorable response | classification |
|---|---|---|---|---|
| normal mammary tissue 1 | 1 | -0.5 | -0.5 | true |
| normal mammary tissue 2 | 1 | -0.5 | -0.5 | true |
| normal mammary tissue 3 | 1 | -0.5 | -0.5 | true |
| normal mammary tissue 4 | 1 | -0.5 | -0.5 | true |
| sample 1 | -0.5 | 1 | -0.5 | true |
| sample 2 | -0.5 | 1 | -0.5 | true |
| sample 3 | 0.8966 | -0.3966 | -0.5 | false |
| sample 4 | -0.5 | 1 | -0.5 | true |
| sample 5 | -0.5 | 1 | -0.5 | true |
| sample 6 | -0.5 | 1 | -0.5 | true |
| sample 7 | -0.5 | 1 | -0.5 | true |
| sample 8 | -0.5 | 1 | -0.5 | true |
| sample 9 | -0.5 | 1 | -0.5 | true |
| sample 10 | -0.5 | 1 | -0.5 | true |
| sample 11 | -0.5 | 1 | -0.5 | true |
| sample 12 | -0.5 | 1 | -0.5 | true |
| sample 13 | -0.5 | -0.5 | 1 | true |
| sample 14 | -0.5 | -0.5 | 1 | true |
| sample 15 | -0.5 | -0.5 | 1 | true |
| sample 16 | -0.5 | -0.4464 | 0.9464 | true |
| sample 17 | -0.5 | -0.5 | 1 | true |
| sample 18 | -0.5 | -0.5 | 1 | true |
| sample 19 | -0.5 | -0.5 | 1 | true |
| sample 20 | -0.5 | -0.5 | 1 | true |
| sample 21 | -0.5 | -0.5 | 1 | true |
| sample 22 | -0.5 | -0.5 | 1 | true |
| sample 23 | -0.5 | -0.5 | 1 | true |

Table 6b

| | True normal | True poor response | favorable response | True Correct [%] |
|---|---|---|---|---|
| Predicted normal | 563 | 27 | 0 | 95.42 |
| Predicted poor response | 4 | 310 | 1 | 98.41 |
| Predicted favorable response | 0 | 0 | 272 | 100 |
| Correct [%] | 99.29 | 91.99 | 99.63 | - |

## EXAMPLE 7

In order to get the most accurate prediction for response to chemotherapy based on the expression levels of genes listed in Table I. One can implement a step wise classification model (e.g. decision tree) identifying first those individuals (tumor tissues) with the highest affinity (e.g. by k-NN classification) to the class of responding tumors (good prognosis group; CR). If an so far unclassified tumor sample did not belong to this class on may perform a second classification step for this sample using the expression levels of the genes from Table 1 and some of the established clinicopahtological parameters such as hormone receptor status, age, TNM classification and risk criteria as established at the St. Gallen consensus conference or the NHI consensus conferences. Nevertheless a classification by the genes listed in Table 1 is sufficient to identify all patients at high risk for recurrence and/or distant metastasis.

**REFERENCES**

*Patents cited*

| | |
|---|---|
| U.S. Pat. No. 4,843,155 | Chomczynski, P.4,683,202 |
| U.S. Pat. No. 5,262,31 | Liang, P., and Pardee, A. B., 19935,593,839 |
| U.S. Pat. No. 4,683,202 | Mullis, K. B., 19875,578,832 |
| U.S. Pat. No. 5,593,8395,556,752 | |
| U.S. Pat. No. 5,578,8325,631,734 | |
| U.S. Pat. No. 5,556,7525,599,695 | |
| U.S. Pat. No. 5,631,7344,683,195 | |
| U.S. Pat. No. 5,599,695 | |
| U.S. Pat. No. 4,683,195 | |
| U.S. Pat. No. 5,498,531 | |
| U.S. Pat. No. 5,714,331 | |
| U.S. Pat. No. 5,641,673 | Haseloff et al., |
| U.S. Pat. No. 5,223,409 | Lander, E., |
| U.S. Pat. No. 5,976,813 | Beutel et al. |
| U.S. Pat. No. 5,283,317 | |
| U.S. Pat No. 6,203,987 | |

WO 97/29212
WO 97/27317
WO 95/22058
WO 99/12826
WO 97/02357
WO 94/13804
WO 94/10300
WO 97/14028
EP 0 785 280
EP 0 799 897
EP 0 728 520
EP 0 721 016
EP 0 321 201
GB2188638B

*Other references cited*

(1) WHO. International Classification of Diseases, 10th edition (ICD-10). WHO
(2) Sabin, L.H., Wittekind, C. (eds): TNM Classification of Malignant Tumors. Wiley, New York, 1997
(3) Sorlie et al., Proc Natl Acad Sci U S A. 2001 Sep 11;98(19):10869-74 (3);
(4) van 't Veer et al., Nature. 2002 Jan 31;415(6871):530-6. (4).

(5) Perez, E.A.: Current Managment of Metastatic Breast Cancer. Semin. Oncol., 1999; 26 (Suppl.12): 1-10

(6) Sambrook et al., MOLECULAR CLONING: A LABORATORY MANUAL, 2d ed., 1989

(7) Tedder, T. F. et al., Proc. Natl. Acad. Sci. U.S.A. 85:208-212, 1988

(8) Hedrick, S. M. et al., Nature 308:149-153, 1984

(9) Sarkar, PCR Methods Applic. 2, 318-322, 1993

(10) Triglia et al., Nucleic Acids Res. 16, 81-86, 1988

(11) Lagerstrom et al., PCR Methods Applic. 1, 111-119, 1991

(12) Cohen, et al., Nature 366: 698-701, 1993

(13) Bonner et al., J. Mol. Biol. 81, 123 1973

(14) Bolton and McCarthy, Proc. Natl. Acad. Sci. U.S.A. 48, 1390 1962

(15) Brown, Meth. Mol. Biol. 20, 18, 1994

(16) Gee et al., in Huber & Carr, MOLECULAR AND IMMUNOLOGIC APPROACHES, Publishing Co., Mt. Kisco, N.Y., 1994

(17) Agrawal et al., Trends Biotechnol. 10, 152-158, 1992

(18) Couture & Stinchcomb, Trends Genet. 12, 510-515, 1996

(19) Haseloff et al. Nature 334, 585-591, 1988

(20) Altschul et al., Bull. Math. Bio. 48:603, 1986,

(21) Henikoff & Henikoff, Proc. Natl. Acad. Sci. USA 89:10915, 1992

(22) Pearson & Lipman, Proc. Nat'l Acad. Sci. USA 85:2444, 1988

(23) Needleman & Wunsch, J. Mol. Biol.48:444, 1970

(24) Sellers, SIAM J. Appl. Math.Xno:787, 1974

(25) Logan & Shenk, Proc. Natl. Acad. Sci. 81, 3655-3659, 1984

(26) Freshney R.I., ed., ANIMAL CELL CULTURE , 1986

(27) Hampton et al., SEROLOGICAL METHODS: A LABORATORY MANUAL, APS Press, St. Paul, Minn., 1990

(28) Kroll et al., DNA Cell Biol. 12, 441-453, 1993

(29) Caruthers et al., Nucl. Acids Res. Symp. Ser. 215-223, 1980

(30) Roberge et al., Science Xno9, 202-204, 1995

(31) Creighton, PROTEINS: STRUCTURES AND MOLECULAR PRINCIPLES, WH and Co., New York, N.Y., 1983

(40) Cronin et al., Human Mutation 7:244, 1996

(41) Landegran et al., Science 241:1077-1080, 1988

(42) Abravaya et al., Nuc Acid Res 23:675-682, 1995

(43) Guatelli, J.C. et al., Proc. Natl. Acad. Sci. USA 87:1874-1878, 1990

(44) Kwoh, D.Y. et al., Proc. Natl. Acad. Sci. USA 86:1173 -1177, 1989

(45) Lizardi, P.M. et al., Bio/Technology 6:1197, 1988

(46) Morrison et al., Proc. Natl. Acad. Sci. 81, 6851-6855, 1984

(47) Burton, Proc. Natl. Acad. Sci. 88, 11120-11123, 1991

(48) Thirion et al., Eur. J. Cancer Prev. 5, 507-11, 1996

(49) Coloma & Morrison, Nat. Biotechnol. 15, 159-63, 1997

(50) Mallender & Voss, J. Biol. Chem. Xno9, 199-206, 1994

(51) Orlandi et al., Proc. Natl. Acad. Sci. 86, 3833-3837, 1989

(52) Gallop et al., J. Med. Chem. 37, 1233, 1994

(53) Houghten, BioTechniques 13, 412-421, 1992

(54) Lam, Nature 354, 8284, 1991

(55) Fodor, Nature 364, 555-556, 1993

(56) Cull et al., Proc. Natl. Acad. Sci. U.S.A. 89, 1865-1869, 1992

(57) Scott & Smith, Science 249, 386-390, 1990

(58) Jayawickreme et al., Proc. Natl. Acad. Sci. U.S.A. 19, 1614-1618, 1994

(59) Chelsky, Strategies for Screening Combinatorial Libraries 1995

(60) Salmon et al., Molecular Diversity 2, 57-63, 1996

(61) McConnell et al., Science 257, 1906-1912, 1992

(62) Szabo et al., Curr. Opin. Struct. Biol. 5, 699-705, 1995

(63) Findeis et al. Trends in Biotechnol. 11, 202-205, 1993

(64) REMINGTON'S PHARMACEUTICAL SCIENCES Maack Publishing Co., Easton, Pa.

(65) Hille, Excitable Membranes, Sunderland, MA, Sinauer Associates, Inc.

(66) Vapnik, The Nature of Statistical Learning Theory, 1995, Springer, New York

(67) Burges, Data Mining and Knowledge Discovery, 2(2):955-974, 1998

(68) Perou et al., Nature, 406:747-752. 2000.

(69) Sorlie et al., Proc Natl Acad Sci U S A, 100:8418-8423,

**EP 1 904 645 B1**

(70) Pusztai et al., Clin Cancer Res., 9:2406-2415, 2003.
(71) Ahr et al., J. Pathol., 195:312-320, 2001.
(72) Martin et al., Cancer Res., 60:2232-2238, 2000.
(73) van de Rijn et al., Am J Pathol., 161:1991-1996, 2002.
(74) Huang et al., Lancet, 361:1590-1596, 2003.
(75) West et al., Proc Natl Acad Sci U S A, 98:11462-11467, 2001
(76) Van de Vijver et al., N Engl J Med. 347:1999-2009, 2002.
(77) Sotiriou et al.,Breast Cancer Res., 4:R3, Epub 2002 Mar 20.
(78) Chang et al., Lancet, 362:362-369, 2003.
(79) Korn et al., Br J Cancer, 86:1093-1096, 2002.
(80) Fisher et al., J Clin Oncol., 15:2483-2493, 1997.
(81) Fisher et al., J Clin Oncol., 16:2672-2685, 1998.
(82) Makris et al., Ann Oncol., 9:1179-1184, 1998.
(83) Chang et al., Cancer, 89:2145-2152, 2000.
(84) Quackenbush, Nature, 2:418-427, 2001.
(85) Eisen et al., Proc. Natl. Acad. Sci. USA, 95:14863-14868, 1998.

**Claims**

1. A method for prediction of therapeutic success of a primary systemic chemotherapy in a subject having breast cancer, comprising

(i) determining the pattern of expression levels of at least 1, 2, 3, 5, 10, 15, 20, 30, or 86 marker genes, comprised in a group of marker genes consisting of SEQUENCE: 1 to 86 of Table 1 in biological samples from said subject, said marker gene or combination of marker genes comprising PPARBP,
(ii) comparing the pattern of expression levels determined in (i) with one or several reference pattern(s) of expression levels, and
(iii) classifying the state of said breast cancer in said subject in comparison to step (ii) and predicting of therapeutic success of a primary systemic chemotherapy.

2. The method of claim 1, wherein said method comprises multiple determinations of a pattern of expression levels, at different points in time, thereby allowing to monitor the development of said breast cancer in said subject.

3. The method of claim 1, wherein said method additional comprises an estimation of the likelihood of success of a given mode of treatment for said breast cancer in said subject.

**Patentansprüche**

1. Ein Verfahren zur Prädikation des Therapieerfolgs einer primär systemischen Chemotherapie bei einem an Brustkrebs erkrankten Subjekt, wobei das Verfahren Folgendes umfasst:

(i) die Bestimmung des Musters der Expressionsprofile von mindestens 1, 2, 3, 5, 10, 15, 20, 30 oder 86 Markergenen aus einer Gruppe von Markergenen, bestehend aus der SEQUENZ: 1 bis 86 von Tabelle 1 in biologischen Proben von dem besagten Subjekt, wobei das besagte Markergen oder die Kombination von Markergenen PPARBP enthält,
(ii) den Vergleich des in (i) bestimmten Musters der Expressionsprofile mit einem oder mehreren Referenzmuster(n) von Expressionsprofilen und
(iii) die Klassifizierung des Stadiums des besagten Brustkrebses bei dem besagten Subjekt im Vergleich zu Schritt (ii) und Prädikation des Therapieerfolgs einer primär systemischen Chemotherapie.

2. Das Verfahren gemäß Anspruch 1, wobei das besagte Verfahren Mehrfachbestimmungen eines Musters von Expressionsprofilen zu unterschiedlichen Zeitpunkten umfasst, was die Überwachung der Entwicklung des besagten Brustkrebses bei dem besagten Subjekt erlaubt.

3. Das Verfahren gemäß Anspruch 1, wobei das besagte Verfahren zusätzlich eine Abschätzung der Wahrscheinlichkeit des Erfolgs einer bestimmten Art der Behandlung für den besagten Brustkrebs bei dem besagten Subjekt umfasst.

**Revendications**

1. Procédé de prévision du succès thérapeutique d'une chimiothérapie systémique primaire chez un sujet ayant un cancer du sein, dans lequel

   (i) on détermine le diagramme de niveaux d'expression d'au moins 1, 2, 3, 5, 10, 15, 20, 30 ou 86 gènes de marqueur compris dans un groupe de gènes de marqueur consistant en séquence : 1 à 86 du tableau 1 dans des échantillons biologiques provenant du sujet, le gène du marqueur ou la combinaison de gènes de marqueur comprenant PPARBP,
   (ii) on compare le diagramme de niveaux d'expression des déterminé dans ( i ) à un ou à plusieurs diagrammes de témoin de niveaux d'expression et,
   (iii) on classe l'état du cancer du sein chez le sujet en comparaison au stade ( ii ) et on prédit le succès thérapeutique d'une chimiothérapie systémique primaire.

2. Procédé suivant la revendication 1, dans lequel le procédé comprend des déterminations multiples d'un schémas de niveaux d'expression à des instants différents dans le temps, en permettant ainsi de contrôler la progression du cancer du sein chez le sujet.

3. Procédé suivant la revendication 1, dans lequel le procédé comprend supplémentairement une estimation de la probabilité de succès d'un mode donné de traitement du cancer du sein chez le sujet.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5556752 A
- WO 9714028 A
- US 4843155 A
- US 5262311 A
- US 4683202 A
- US 5714331 A
- WO 9912826 A
- US 5641673 A
- EP 0321201 A
- EP 0799897 A
- WO 9729212 A
- WO 9727317 A
- EP 0785280 A
- WO 9702357 A
- US 5593839 A
- US 5578832 A
- EP 0728520 A
- US 5599695 A
- EP 0721016 A

- WO 9522058 A
- US 5631734 A
- US 6203987 B
- US 4683195 A
- GB 2188638 B
- US 5565332 A
- WO 9303151 A
- WO 9413804 A
- US 5223409 A
- US 5976813 A
- US 5283317 A
- WO 9410300 A
- US 5705151 A
- US 526231 A
- US 55938395556752 A
- US 55788325631734 A
- US PATNO055567525599695 A
- US PATNO56317344683195 A
- US 5498531 A

**Non-patent literature cited in the description**

- **Sorlie et al.** *Proc Natl Acad Sci U S A.,* 11 September 2001, vol. 98 (19), 10869-74
- **van 't Veer et al.** *Nature,* 31 January 2002, vol. 415 (6871), 530-6
- **Perez, E.A.** *Current Managment of Metastatic Breast Cancer. Semin. Oncol.,* 1999, vol. 26, 1-10
- **Sambrook et al.** *MOLECULAR CLONING: A LABORATORY MANUAL,* 1989
- **Tedder, T. F. et al.** *Proc. Natl. Acad. Sci. U.S.A.,* 1988, vol. 85, 208-212
- **Hedrick, S. M. et al.** *Nature,* 1984, vol. 308, 149-153
- **Sarkar.** *PCR Methods Applic,* 1993, vol. 2, 318-322
- **Triglia et al.** *Nucleic Acids Res.,* 1988, vol. 16, 81-86
- **Lagerstrom et al.** *PCR Methods Applic,* 1991, vol. 1, 111-119
- **Cohen et al.** *Nature,* 1993, vol. 366, 698-701
- **Bonner et al.** *J. Mol. Biol.,* 1973, vol. 81, 123
- **Bolton ; McCarthy.** *Proc. Natl. Acad. Sci. U.S.A.,* 1962, vol. 48, 1390
- **Brown.** *Meth. Mol. Biol,* 1994, vol. 20, 18
- **Gee et al.** MOLECULAR AND IMMUNOLOGIC APPROACHES. Publishing Co, 1994

- **Agrawal et al.** *Trends Biotechnol,* 1992, vol. 10, 152-158
- **Couture ; Stinchcomb.** *Trends Genet,* 1996, vol. 12, 510-515
- **Haseloff et al.** *Nature,* 1988, vol. 334, 585-591
- **Altschul et al.** *Bull. Math. Bio,* 1986, vol. 48, 603
- **Henikoff ; Henikoff.** *Proc. Natl. Acad. Sci. USA,* 1992, vol. 89, 10915
- **Pearson ; Lipman.** *Proc. Nat'l Acad. Sci. USA,* 1988, vol. 85, 2444
- **Needleman ; Wunsch.** *J. Mol. Biol.,* 1970, vol. 48, 444
- **Sellers, SIAM.** *J. Appl. Math.Xno,* 1974, 787
- **Logan ; Shenk.** *Proc. Natl. Acad. Sci.,* 1984, vol. 81, 3655-3659
- ANIMAL CELL CULTURE. 1986
- **Hampton et al.** SEROLOGICAL METHODS: A LABORATORY MANUAL. APS Press, 1990
- **Kroll et al.** *DNA Cell Biol,* 1993, vol. 12, 441-453
- **Caruthers et al.** *Nucl. Acids Res. Symp. Ser.,* 1980, 215-223
- **Roberge et al.** *Science,* 1995, 202-204

- **Creighton.** PROTEINS: STRUCTURES AND MOLECULAR PRINCIPLES. WH and Co, 1983
- **Cronin et al.** *Human Mutation,* 1996, vol. 7, 244
- **Landegran et al.** *Science,* 1988, vol. 241, 1077-1080
- **Abravaya et al.** *Nuc Acid Res,* 1995, vol. 23, 675-682
- **Guatelli, J.C et al.** *Proc. Natl. Acad. Sci. USA,* 1990, vol. 87, 1874-1878
- **Kwoh, D.Y. et al.** *Proc. Natl. Acad. Sci. USA,* 1989, vol. 86, 1173-1177
- **Lizardi, P.M et al.** *Bio/Technology,* 1988, vol. 6, 1197
- **Morrison et al.** *Proc. Natl. Acad. Sci.,* 1984, vol. 81, 6851-6855
- **Burton.** *Proc. Natl. Acad. Sci.,* 1991, vol. 88, 11120-11123
- **Thirion et al.** *Eur. J. Cancer Prev,* 1996, vol. 5, 507-11
- **Coloma ; Morrison.** *Nat. Biotechnol.,* 1997, vol. 15, 159-63
- **Mallender ; Voss.** *J. Biol. Chem.,* 1994, 199-206
- **Orlandi et al.** *Proc. Natl. Acad. Sci.,* 1989, vol. 86, 3833-3837
- **Gallop et al.** *J. Med. Chem.,* 1994, vol. 37, 1233
- **Houghten.** *BioTechniques,* 1992, vol. 13, 412-421
- **Lam.** *Nature,* 1991, vol. 354, 8284
- **Fodor.** *Nature,* 1993, vol. 364, 555-556
- **Cull et al.** *Proc. Natl. Acad. Sci. U.S.A.,* 1992, vol. 89, 1865-1869
- **Scott ; Smith.** *Science,* 1990, vol. 249, 386-390
- **Jayawickreme et al.** *Proc. Natl. Acad. Sci. U.S.A.,* 1994, vol. 19, 1614-1618
- **Chelsky.** *Strategies for Screening Combinatorial Libraries,* 1995
- **Salmon et al.** *Molecular Diversity,* 1996, vol. 2, 57-63
- **McConnell et al.** *Science,* 1992, vol. 257, 1906-1912
- **Szabo et al.** *Curr. Opin. Struct. Biol.,* 1995, vol. 5, 699-705
- **Findeis et al.** *Trends in Biotechnol,* 1993, vol. 11, 202-205
- REMINGTON'S PHARMACEUTICAL SCIENCES. Maack Publishing Co,
- **Hille.** Excitable Membranes. Sinauer Associates, Inc
- **Vapnik.** *The Nature of Statistical Learning Theory,* 1995
- **Burges.** *Data Mining and Knowledge Discovery,* 1998, vol. 2 (2), 955-974
- **Perou et al.** *Nature,* 2000, vol. 406, 747-752
- **Sorlie et al.** *Proc Natl Acad Sci U S A,* vol. 100, 8418-8423
- **Pusztai et al.** *Clin Cancer Res.,* 2003, vol. 9, 2406-2415
- **Ahr et al.** *J. Pathol.,* 2001, vol. 195, 312-320
- **Martin et al.** *Cancer Res.,* 2000, vol. 60, 2232-2238
- **van de Rijn et al.** *Am J Pathol.,* 2002, vol. 161, 1991-1996
- **Huang et al.** *Lancet,* 2003, vol. 361, 1590-1596
- **West et al.** *Proc Natl Acad Sci U S A,* 2001, vol. 98, 11462-11467
- **Van de Vijver et al.** *N Engl J Med,* 2002, vol. 347, 1999-2009
- **Sotiriou et al.** *Breast Cancer Res.,* 2002, vol. 4
- **Chang et al.** *Lancet,* 2003, vol. 362, 362-369
- **Korn et al.** *Br J Cancer,* 2002, vol. 86, 1093-1096
- **Fisher et al.** *J Clin Oncol.,* 1997, vol. 15, 2483-2493
- **Fisher et al.** *J Clin Oncol.,* 1998, vol. 16, 2672-2685
- **Makris et al.** *Ann Oncol.,* 1998, vol. 9, 1179-1184
- **Chang et al.** *Cancer,* 2000, vol. 89, 2145-2152
- **Quackenbush.** *Nature,* 2001, vol. 2, 418-427
- **Eisen et al.** *Proc. Natl. Acad. Sci. USA,* 1998, vol. 95, 14863-14868